(19) Europäisches Patentamt
European Patent Office
Office européen des brevets

(11) EP 0 944 623 B1

# (12) EUROPÄISCHE PATENTSCHRIFT

(45) Veröffentlichungstag und Bekanntmachung des Hinweises auf die Patenterteilung:
**01.09.2004 Patentblatt 2004/36**

(21) Anmeldenummer: **97954346.9**

(22) Anmeldetag: **01.12.1997**

(51) Int Cl.[7]: **C07D 417/04**, A01N 43/56, C07D 413/10, C07D 417/10, C07D 413/04

(86) Internationale Anmeldenummer:
**PCT/EP1997/006715**

(87) Internationale Veröffentlichungsnummer:
**WO 1998/027090 (25.06.1998 Gazette 1998/25)**

## (54) SUBSTITUIERTE PYRAZOL-3-YLBENZAZOLE

SUBSTITUTED PYRAZOLE-3-YL BENZAZOLES

PYRAZOL-3-YLBENZAZOLES SUBSTITUES

(84) Benannte Vertragsstaaten:
**AT BE CH DE DK ES FI FR GB GR IE IT LI NL PT SE**
Benannte Erstreckungsstaaten:
**RO SI**

(30) Priorität: **16.12.1996 DE 19652240**

(43) Veröffentlichungstag der Anmeldung:
**29.09.1999 Patentblatt 1999/39**

(73) Patentinhaber: **BASF AKTIENGESELLSCHAFT**
**67056 Ludwigshafen (DE)**

(72) Erfinder:
- **ZAGAR, Cyrill**
  **D-67061 Ludwigshafen (DE)**
- **HAMPRECHT, Gerhard**
  **D-69469 Weinheim (DE)**
- **MENGES, Markus**
  **D-68161 Mannheim (DE)**
- **MENKE, Olaf**
  **D-67317 Altleiningen (DE)**
- **SCHÄFER, Peter**
  **D-67308 Ottersheim (DE)**
- **WESTPHALEN, Karl-Otto**
  **D-67346 Speyer (DE)**
- **MISSLITZ, Ulf**
  **D-67433 Neustadt (DE)**
- **WALTER, Helmut**
  **D-67283 Obrigheim (DE)**

(56) Entgegenhaltungen:
**EP-A- 0 155 523**
**EP-A- 0 235 567**
**WO-A-92/02509**
**WO-A-92/06962**
**WO-A-96/01254**
**WO-A-96/15115**
**WO-A-96/15116**
**DE-A- 3 705 933**
**DE-A- 3 705 935**
**DE-A- 4 241 658**
**US-A- 4 376 122**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue substituierte Pyrazol-3-ylbenzazole der Formel I

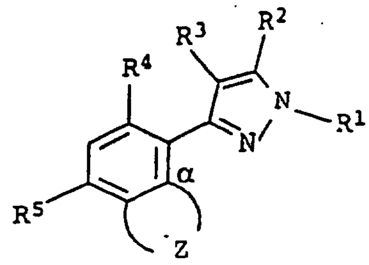

I,

in der die Variablen folgende Bedeutungen haben:

$R^1$ Wasserstoff, $C_1$-$C_4$-Alkyl oder $C_1$-$C_4$-Halogenalkyl;

$R^2$ $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkyl-sulfonyl oder $C_1$-$C_4$-Halogenalkylsulfonyl;

$R^3$ Wasserstoff, Halogen, Cyano, Nitro, $C_1$-$C_4$-Alkyl oder $C_1$-$C_4$-Halogenalkyl;

$R^4$ Wasserstoff oder Halogen;

$R^5$ Halogen, Cyano oder Trifluormethyl

Z eine Gruppe -N=C($XR^6$)-O- oder -N=C($XR^6$)-S-, die über den Stickstoff, Sauerstoff oder Schwefel an $\alpha$ gebunden sein kann;

X eine chemische Bindung, Sauerstoff, Schwefel, -S(O)-, -$SO_2$-, -NH- oder -N($R^7$)-;

$R^6$, $R^7$ unabhängig voneinander $C_1$-$C_6$-Alkyl, Cyano-$C_1$-$C_4$-alkyl, Hydroxy-$C_1$-$C_4$-alkyl, $C_3$-$C_6$-Alkenyl, Cyano-$C_3$-$C_6$-alkenyl, $C_3$-$C_6$-Halogen-alkenyl, $C_3$-$C_6$-Alkinyl, Cyano-$C_3$-$C_6$-alkinyl, $C_3$-$C_6$-Halogen-alkinyl, $C_1$-$C_4$-Alkoxy-$C_1$-$C_4$-alkyl, $C_1$-$C_4$-Halogenalkoxy-$C_1$-$C_4$-alkyl, $C_3$-$C_4$-Alkenyloxy-$C_1$-$C_4$-alkyl, $C_3$-$C_4$-Alkinyloxy-$C_1$-$C_4$-alkyl, $C_3$-$C_8$-Cycloalkyloxy-$C_1$-$C_4$-alkyl, Amino-$C_1$-$C_4$-alkyl, $C_1$-$C_4$-Alkylamino-$C_1$-$C_4$-alkyl, Di($C_1$-$C_4$-alkyl)amino-$C_1$-$C_4$-alkyl, $C_1$-$C_4$-Alkylthio-$C_1$-$C_4$-alkyl, $C_1$-$C_4$-Halogenalkyl-chio-$C_1$-$C_4$-alkyl, $C_3$-$C_4$-Alkenylthio-$C_1$-$C_4$-alkyl, $C_3$-$C_4$-Alkinyl-thio-$C_1$-$C_4$-alkyl, $C_1$-$C_4$-Alkylsulfinyl-$C_1$-$C_4$-alkyl, $C_1$-$C_4$-Falogenalkylsulfinyl-$C_1$-$G_4$-alkyl, $C_3$-$C_4$-Alkenylsulfinyl-$C_1$-$C_4$-alkyl, $C_3$-$C_4$-Alkinsulfinyl-$C_1$-$C_4$-alkyl, $C_1$-$C_4$-Alkylsulfonyl-$C_1$-$C_4$-alkyl, $C_1$-$C_4$-Halogenalkylsulfonyl-$C_1$-$C_4$-alkyl, $C_3$-$C_4$-Alkenylsulfonyl-$C_1$-$C_4$-alkyl, $C_3$-$C_4$-Alkinylsulfonyl-$C_1$-$C_4$-alkyl, Hydroxycarbonyl-$C_1$-$C_4$-alkyl, ($C_1$-$C_4$-Alkoxy)carbonyl-$C_1$-$C_4$-alkyl, das eine Cyano- oder ($C_1$-$C_4$-Alkoxy)carbonylgruppe tragen kann,
($C_1$-$C_4$-Alkylthio)carbonyl-$C_1$-$C_4$-alkyl, Aminocarbonyl-$C_1$-$C_4$-alkyl, $C_1$-$C_4$-Alkylaminocarbonyl-$C_1$-$C_4$-alkyl, Di($C_1$-$C_4$-alkyl)-aminocarbonyl-$C_1$-$C_4$-alkyl, Di($C_1$-$C_4$-alkyl)phosphonyl-$C_1$-$C_4$-alkyl, ($C_1$-$C_4$-Alkoxy)imino-$C_1$-$C_4$-alkyl, ($C_3$-$C_4$-Alkenyloxy)-imino-$C_1$-$C_4$-alkyl,
$C_3$-$C_8$-Cycloalkyl, $C_3$-$C_8$-Cycloalkyl-$C_1$-$C_4$-alkyl, Phenyl, Phenyl-$C_1$-$C_4$-alkyl, 3- bis 7-gliecriges Heterocyclyl oder Heterocyclyl-$C_1$-$C_4$-alkyl, wobei jeder Cycloalkyl- und jeder Heterocyclyl-Ring ein Carbonyl- oder Thiocarbonyl-Ringglied enthalten kann,
und wobei jeder Cycloalkyl-, Phenyl- und Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, $C_1$-$C_4$-Alkyl,
$C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfonyl, $C_1$-$C_4$-Halogenalkylsulfonyl, ($C_1$-$C_4$-Alkoxy)carbonyl, ($C_1$-$C_4$-Alkyl)carbonyl, ($C_1$-$C_4$-Halogenalkyl)carbonyl, ($C_1$-$C_4$-Alkyl)carbonyloxy, ($C_1$-$C_4$-Halogenalkyl)carbonyloxy und Di($C_1$-$C_4$-alkyl)amino;
sofern X eine chemische Bindung, Sauerstoff, Schwefel, -NH- oder -N($R^7$)- ist, kann $R^6$ auch ($C_1$-$C_4$-Alkyl)carbonyl, ($C_1$-$C_4$-Halogenalkyl)carbonyl, ($C_1$-$C_4$-Alkoxy)carbonyl, $C_1$-$C_4$-Alkylsulfonyl oder $C_1$-$C_4$-Halogenalkylsulfonyl bedeuten:

sofern X eine chemische Bindung ist, kann $R^6$ außerdem Wasserstoff, Cyano, Mercapto, Amino, Halogen, $-CH_2-CH(Halogen)-R^8$, $-CH=CH-R^8$ oder $-CH=C(Halogen)-R^8$ bedeuten, wobei $R^8$ für Hydroxycarbonyl, ($C_1$-$C_4$-Alkoxy)carbonyl, ($C_1$-$C_4$-Alkylthio)carbonyl, Aminocarbonyl, $C_1$-$C_4$-Alkylaminocarbonyl, Di($C_1$-$C_4$-alkyl)aminocarbonyl oder Di($C_1$-$C_4$-alkyl)phosphonyl steht,

oder $R^6$ und $R^7$ stehen zusammen für eine 1,3-Propylen-, Tetramethylen-, Pentamethylen- oder Ethylenoxyethylen-Kette, die jeweils unsubstituiert sein oder ein bis vier $C_1$-$C_4$-Alkylgruppen oder ein oder zwei ($C_1$-$C_4$-Alkoxy)carbonylgruppen tragen kann, sowie die landwirtschaftlich brauchbaren Salze dieser Verbindungen I.

Außerdem betrifft die Erfindung

- die Verwendung der Verbindungen I als Herbizide und/oder zur Desikkation/Defoliation von Pflanzen,
- herbizide Mittel und Mittel zur Desikkation und/oder Defoliation von Pflanzen, welche die Verbindungen I als wirksame Substanzen enthalten,
- Verfahren zur Herstellung der Verbindungen I und von herbiziden Mittel und Mitteln zur Desikkation/Defoliation von Pflanzen unter Verwendung der Verbindungen I, sowie
- Verfahren zur Bekämpfung von unerwünschtem Pflanzenwuchs und zur Desikkation/Defoliation von Pflanzen mit den Verbindungen I.

Bestimmte Arylpyrazole, bei denen als Arylrest außer Phenyl prinzipiell auch verschiedene Benzheterocyclyl-Reste in Betracht kommen, werden bereits in den Patentanmeldungen WO 92/02509, WO 92/06962, WO 86/01255, WO 96/15115, WO 96/15116 und WO 96/40643 als Herbizide beschrieben.

Des weiteren fallen unter die allgemeine Formel der WO 96/01254 bei geeigneter Wahl der Substituenten - u.a. Verbindungen der Formel II

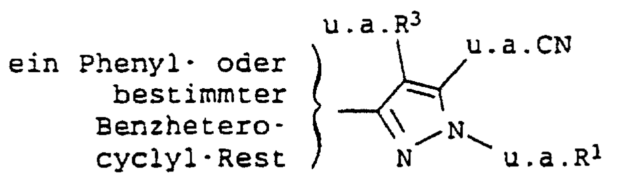

II,

denen ebenfalls eine herbizide Wirkung zugeschrieben wird.

Aufgabe der vorliegenden Erfindung war es, neue herbizid wirksame Pyrazol-Verbindungen bereitzustellen, mit denen sich unerwünschte Pflanzen besser als mit den bekannten gezielt bekämpfen lassen. Die Aufgabe erstreckte sich auch auf die Bereitstellung neuer desikkant/defoliant wirksamer Verbindungen.

Demgemäß wurden die vorliegenden substituierten Pyrazol-3-ylbenzazole der Formel I gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Desikkation/Defoliation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die bei der Definition der Substituenten $R^1$ bis $R^3$ und $R^5$ bis $R^8$ oder als Reste an Cycloalkyl-, Phenyl- oder heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Cyanoalkyl-, Oxyalkyl-, Aminoalkyl-, Oxycarbonylalkyl-, Aminocarbonylalkyl-, Phosphonylalkyl-, Oxyaminoalkyl-, Phenylalkyl-, Heterocyclylalkyl-, Alkenyl-, Halogenalkenyl-, Cyanoalkenyl-, Alkinyl-, Halogenalkinyl- und Cyanoalkinyl-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:

- $C_1$-$C_4$-Alkyl für: $CH_3$, $C_2H_5$, n-Propyl, $CH(CH_3)_2$, n-Butyl, 1-Methylpropyl, 2-Methylpropyl oder $C(CH_3)_3$;

- $C_1$-$C_4$-Halogenalkyl für: einen $C_1$-$C_4$-Alkylrest wie vorstehend genannt, der partiell oder vollständig durch Fluor, Chlor, Brom und/oder Iod substituiert ist, also z.B. $CH_2F$, $CHF_2$, $CF_3$, $CH_2Cl$, $CH(Cl)_2$, $C(Cl)_3$, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, $C_2F_5$, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, $CH_2$-$C_2F_5$, $CF_2$-$C_2F_5$, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;

- $C_1$-$C_6$-Alkyl für: $C_1$-$C_4$-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für $CH_3$, $C_2H_5$, $CH_2$-$C_2H_5$, $CH(CH_3)_2$, n-Butyl, $C(CH_3)_3$, n-Pentyl oder n-Hexyl;

- $C_1$-$C_6$-Halogenalkyl für: einen $C_1$-$C_6$-Alkylrest wie vorstehend genannt, der partiell oder vollständig durch Fluor, Chlor, Brom und/oder Iod substituiert ist, also z.B. einen der unter $C_1$-$C_4$-Halogenalkyl genannten Reste oder für 5-Fluor-1-pentyl, S-Chlor-1-pentyl, 5-Brom-1-pentyl, 5-Iod-1-pentyl, 5,5,5-Trichlor-1-penyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl;

- Cyano-$C_1$-$C_4$-alkyl für: $CH_2CN$, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-($CH_2CN$)eth-1-yl, 1-($CH_2CN$)-1-($CH_3$)-eth-1-yl oder 1-($CH_2CN$)prop-1-yl;

- Hydroxy-$C_1$-$C_4$-alkyl für: $CH_2OH$, 1-Hydroxyethyl, 2-Hydroxyethyl, 1-Hydroxyprop-1-yl, 2-Hydroxyprop-1-yl, 3-Hydroxyprop-1-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 3-Hydroxybut-2-yl, 4-Hydroxybut-2-yl, 1-($CH_2OH$)eth-1-yl, 1-($CH_2OH$)-1-($CH_3$)-eth-1-yl oder 1-($CH_2OH$)prop-1-yl;

- Amino-$C_1$-$C_4$-alkyl für: $CH_2NH_2$, 1-Aminoethyl, 2-Aminoethyl, 1-Aminoprop-1-yl, 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 4-Aminobut-2-yl, 1-($C_2NH_2$)eth-1-yl, 1-($CH_2NH_2$)-1-($CH_3$)-eth-1-yl oder 1-($CH_2NH_2$)prop-1-yl;

- Hydroxycarbonyl-$C_1$-$C_4$-alkyl für: $CH_2COOH$, 1-(COOH)ethyl, 2-(COOH)ethyl, 1-(COOH)prop-1-yl, 2-(COOH)prop-1-yl, 3-(COOH)-prop-1-yl, 1-(COOH)but-1-yl, 2-(COOH)but-1-yl, 3-(COOH)but-1-yl, 4-(COOH)but-1-yl, 1-(COOH)but-2-yl, 2-(COOH)but-2-yl, 3-(COOH)but-2-yl, 4-(COOH)but-2-yl, 1-($CH_2COOH$)eth-1-yl, 1-($CH_2COOH$)-1-($CH_3$)-eth-1-yl oder 1-($CH_2COOH$)prop-1-yl;

- Aminocarbonyl-$C_1$-$C_4$-alkyl für: $CH_2CONH_2$, 1-($CONH_2$)ethyl, 2-($CONH_2$)ethyl, 1-($CONH_2$)prop-1-yl, 2-($CONH_2$)prop-1-yl, 3-($CONH_2$)prop-1-yl, 1-($CONH_2$)but-1-yl, 2-($CONH_2$)but-1-yl, 3-($CONH_2$)but-1-yl, 4-($CONH_2$)but-1-yl, 1-($CONH_2$)but-2-yl, 2-($CONH_2$)but-2-yl, 3-($CONH_2$)but-2-yl, 4-($CONH_2$)but-2-yl, 1-($CH_2CONH_2$)eth-1-yl, 1-($CH_2CONH_2$)-1-($CH_3$)-eth-1-yl oder 1-($CH_2CONH_2$)prop-1-yl;

- Phenyl-$C_1$-$C_4$-alkyl für: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Benzyl)-eth-1-yl, 1-(Benzyl)-1-(methyl)-eth-1-yl oder 1-(Benzyl)-prop-1-yl, vorzugsweise für Benzyl oder 2-Phenylethyl;

- Heterocyclyl-$C_1$-$C_4$-alkyl für: Heterocyclylmethyl, 1-Heterocyclyl-ethyl, 2-Heterocyclyl-ethyl, 1-Heterocyclyl-prop-1-yl, 2-Heterocyclyl-prop-1-yl, 3-Heterocyclyl-prop-1-yl, 1-Heterocyclyl-but-1-yl, 2-Heterocyclyl-but-1-yl, 3-Heterocyclylbut-1-yl, 4-Heterocyclyl-but-1-yl, 1-Heterocyclyl-but-2-yl, 2-Heterocyclyl-but-2-yl, 3-Heterocyclyl-but-2-yl, 3-Heterocyclyl-but-2-yl, 4-Heterocyclyl-but-2-yl, 1-(Heterocyclylmethyl)-eth-1-yl, 1-(Heterocyclylmethyl)-1-(methyl)-eth-1-yl oder 1-(Heterocyclylmethyl)-prop-1-yl, vorzugsweise Heterocyclylmethyl oder 2-Heterocyclyl-ethyl;

- $C_1$-$C_4$-Alkoxy für: $OCH_3$, $OC_2H_5$, $OCH_2$-$C_2H_5$, $OCH(CH_3)_2$, n-Butoxy, $OCH(CH_3)$-$C_2H_5$, $OCH_2$-$CH(CH_3)_2$ oder $C(CH_3)_3$, vorzugsweise für $OCH_3$, $OC_2H_5$ oder $OCH(CH_3)_2$;

- $C_1$-$C_4$-Halogenalkoxy für: einen $C_1$-$C_4$-Alkoxyrest wie vorstehend genannt, der partiell oder vollständig durch Fluor, Chlor, Brom und/oder Iod substituiert ist, also z.B. $OCH_2F$, $OCHF_2$, $OCF_3$, $OCH_2Cl$, $OCH(Cl)_2$, $OC(Cl)_3$, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, $OC_2F_5$, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, $OCH_2$-$C_2F_5$, $OCF_2$-$C_2F_5$, 1-($CH_2F$)-2-fluorethoxy, 1-($CH_2Cl$)-2-chlorethoxy, 1-($CH_2Br$)-2-bromethoxy, 4-Fluorbutoxy, 4-Chlorbutoxy, 4-Brombutoxy oder Nonafluorbutoxy, vorzugsweise für $OCHF_2$, $OCF_3$, Dichlorfluormethoxy, Chlordifluormethoxy oder 2,2,2-Trifluorethoxy;

- $C_1$-$C_4$-Alkylthio für: $SCH_3$, $SC_2H_5$, $SCH_2$-$C_2H_5$, $SCH(CH_3)_2$, n-Butylthio, 1-Methylpropylthio, $SCH_2$-$CH(CH_3)_2$ oder $SC(CH_3)_3$, vorzugsweise für $SCH_3$ oder $SC_2H_5$;

- $C_1$-$C_4$-Halogenalkylthio für: einen $C_1$-$C_9$-Alkylthiorest wie vorstehend genannt, der partiell oder vollständig durch Fluor, Chlor, Brom und/oder Iod substituiert ist, also z.B. $SCH_2F$, $SCHF_2$, $SCF_3$, $SCH_2Cl$, $SCH(Cl)_2$, $SC(Cl)_3$, Chlorfluormethylthio, Dichlorfluormethylthio, Chlordifluormethylthio, 2-Fluorethylthio, 2-Chlorethylthio, 2-Bromethylthio, 2-Iodethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, $SC_2F_5$, 2-Fluorpropylthio, 3-Fluorpropylthio, 2,2-Difluorpropylthio, 2,3-Difluorpropylthio, 2-Chlorpropylthio, 3-Chlorpropylthio, 2,3-Dichlorpropylthio, 2-Brompropylthio, 3-Brompropylthio, 3,3,3-Trifluorpropylthio, 3,3,3-Trichlorpropylthio, $SCH_2$-$C_2F_5$, $SCF_2$-$C_2F_5$, 1-($CH_2F$)-2-fluorethylthio, 1-($CH_2Cl$)-2-chlorethylthio, 1-($CH_2Br$)-2-bromethylthio, 4-Fluorbutylthio, 4-Chlorbutylthio, 4-Brombutylthio oder $SCF_2$-$CF_2$-$C_2F_5$, vorzugsweise für $SCHF_2$, $SCF_3$, Dichlorfluormethylthio, Chlordifluormethylthio oder 2,2,2-Trifluorethylthio;

- $C_1$-$C_4$-Alkoxy-$C_1$-$C_4$-alkyl für: durch $C_1$-$C_4$-Alkoxy - wie vorstehend genannt - substituiertes $C_1$-$C_4$-Alkyl, also z.B. für $CH_2$-$OCH_3$, $CH_2$-$OC_2H_5$, n-Propoxymethyl, $CH_2$-$OCH(CH_3)_2$, n-Butoxymethyl, (1-Methylpropoxy)methyl, (2-Methylpropoxy)methyl, $CH_2$-$OC(CH_3)_3$, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)-propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy) butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxylbutyl, 3-(Methoxy)butyl, 3-(Ethoxy)butyl, 3-(n-Propoxy)butyl, 3-(1-Methylethoxy)butyl, 3-(n-Butoxy) butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise für $CH_2$-$OCH_3$, $CH_2$-$OC_2H_5$, 2-($OCH_3$) ethyl oder 2-($OC_2H_5$)ethyl;

- $C_1$-$C_4$-Halogenalkoxy-$C_1$-$C_4$-alkyl für: durch $C_1$-$C_4$-Halogenalkoxy wie vorstehend genannt substituiertes $C_1$-$C_4$-Alkyl, also z.B. für 2-($OCHF_2$)ethyl, 2-($OCF_3$)ethyl oder 2-($OC_2F_5$)ethyl;

- $C_1$-$C_4$-Alkylthio-$C_1$-$C_4$-alkyl für: durch $C_1$-$C_4$-Alkylthio - wie vorstehend genannt - substituiertes $C_1$-$C_4$-Alkyl, also z.B. für $CH_2$-$SCH_3$, $CH_2$-$SC_2H_5$, n-Propylthiomethyl, $CH_2$-$SCH(CH_3)_2$, n-Butylthiomethyl, (1-Methylpropylthio)methyl, (2-Methylpropylthio)methyl, $CH_2$-$SC(CH_3)_3$, 2-(Methylthio)ethyl, 2-(Ethylthio)-ethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 2-(Ethylthio)propyl, 2-(n-Propylthio)propyl, 2-(1-Methylethylthio)propyl, 2-(n-Butylthio)propyl, 2-(1-Methylpropylthio)propyl, 2-(2-Methylpropylthio)propyl, 2-(1,1-Dimethylethylthio) propyl, 3-(Methylthio)propyl, 3-(Ethylthio)propyl, 3-(n-Propylthio)propyl, 3-(1-Methylethylthio)propyl, 3-(n-Butylthio)propyl, 3-(1-Methylpropylthio)-propyl, 3-(2-Methylpropylthio)propyl, 3-(1,1-Dimethylethylthio)propyl, 2-(Methylthio)butyl, 2-(Ethylthio)butyl, 2-(n-Propylthio)butyl, 2-(1-Methylethylthio)butyl, 2-(n-Butylthio)butyl, 2-(1-Methylpropylthio)butyl, 2-(2-Methylpropylthio)butyl, 2-(1,1-Dimethylethylthio)butyl, 3-(Methylthio)butyl, 3-(Ethylthio)butyl, 3-(n-Propylthio)butyl, 3-(1-Methylethylthio)butyl, 3-(n-Butylthio)butyl, 3-(1-Methylpropylthio)butyl, 3-(2-Methylpropylthio)butyl, 3-(1,1-Dimethylethylthio)butyl, 4-(Methylthio)butyl, 4-(Ethylthio)butyl, 4-(n-Propylthio)butyl, 4-(1-Methylethylthio)butyl, 4-(n-Butylthio)-butyl, 4-(1-Methylpropylthio)butyl, 4-(2-Methylpropylthio)butyl oder 4-(1,1-Dimethylethylthio)butyl, vorzugsweise $CH_2$-$SCH_3$, $CH_2$-$SC_2H_5$, 2-($SCH_3$)ethyl oder 2-($SC_2H_5$)ethyl;

- $C_1$-$C_4$-Halogenalkylthio-$C_1$-$C_4$-alkyl für: durch $C_1$-$C_4$-Halogenalkylthio wie vorstehend genannt substituiertes

$C_1$-$C_4$-Alkyl, also z.B. für 2-($SCHF_2$)ethyl, 2-($SCF_3$)ethyl oder 2-($SC_2F_5$)ethyl;

- ($C_1$-$C_4$-Alkoxy)imino-$C_1$-$C_4$-alkyl für: durch ($C_1$-$C_4$-Alkoxy)imino wie =N-$CH_3$, =N-$C_2H_5$, =$NCH_2$-$C_2H_5$, =N-CH($CH_3$)$_2$, =$NCH_2$-$CH_2$-$C_2H_5$, =NCH($CH_3$)-$C_2H_5$, =$NCH_2$-CH($CH_3$)$_2$ oder =N-C($CH_3$)$_3$ substituiertes $C_1$-$C_4$-Alkyl, also z.B. für CH=N-$CH_3$, CH=N-$C_2H_5$, $CH_2$-CH=N-$CH_3$ oder $CH_2$-CH=N-$C_2H_5$;

- ($C_1$-$C_4$-Alkyl)carbonyl für: CO-$CH_3$, CO-$C_2H_5$, CO-$CH_2$-$C_2H_5$, CO-CH($CH_3$)$_2$, n-Butylcarbonyl, CO-CH($CH_3$)-$C_2H_5$, CO-$CH_2$-CH($CH_3$)$_2$ oder CO-C($CH_3$)$_3$, vorzugsweise für CO-$CH_3$ oder CO-$C_2H_5$;

- ($C_1$-$C_4$-Halogenalkyl)carbonyl für: einen ($C_1$-$C_4$-Alkyl)carbonylrest - wie vorstehend genannt - der partiell oder vollständig durch Fluor, Chlor, Brom und/oder Iod substituiert ist, also z.B. CO-$CH_2F$, CO-$CHF_2$, CO-$CF_3$, CO-$CH_2Cl$, CO-CH(Cl)$_2$, CO-C(Cl)$_3$, Chlorfluormethylcarbonyl, Dichlorfluormethylcarbonyl, Chlordifluormethylcarbonyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, CO-$C_2F_5$, 2-Fluorpropylcarbonyl, 3-Fluorpropylcarbonyl, 2,2-Difluorpropylcarbonyl, 2,3-Diiluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, CO-$CF_2$-$C_2F_5$, CO-$CF_2$-$C_2F_5$, 1-($CH_2F$)-2-fluorethylcarbonyl, 1-($CH_2Cl$)-2-chlorethylcarbonyl, 1-($CH_2Br$)-2-bromethylcarbonyl, 4-Fluorbutylcarbonyl, 4-Chlorbutylcarbonyl, 4-Brombutylcarbonyl oder Nonafluorbutylcarbonyl, vorzugsweise für CO-$CF_3$ CO-$CH_2Cl$ oder 2,2,2-Trifluorethylcarbonyl;

- ($C_1$-$C_4$-Alkyl)carbonyloxy für: O-CO-$CH_3$, O-CO-$C_2H_5$, O-CO-$CH_2$-$C_2H_5$, O-CO-CH($CH_3$)$_2$, O-CO-$CH_2$-$CH_2$-$C_2H_5$, O-CO-CH($CH_3$)-$C_2H_5$, O-CO-$CH_2$-CH($CH_3$)$_2$ oder O-CO-C($CH_3$)$_3$, vorzugsweise für O-CO-$CH_3$ oder O-CO-$C_2H_5$;

- ($C_1$-$C_4$-Halogenalkyl)carbonyloxy für: einen ($C_1$-$C_4$-Alkyl)carbonylrest - wie vorstehend genannt - der partiell oder vollständig durch Fluor, Chlor, Brom und/oder Iod substituiert ist, also z.B. O-CO-$CH_2F$, O-CO-$CHF_2$, O-CO-$CF_3$, O-CO-$CH_2Cl$, O-CO-CH(Cl)$_2$, O-CO-C(Cl)$_3$, Chlorfluormethylcarbonyloxy, Dichlorfluormethylcarbonyloxy, Chlordifluormethylcarbonyloxy, 2-Fluorethylcarbonyloxy, 2-Chlorethylcarbonyloxy, 2-Bromethyl-carbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, O-CO-$C_2F_5$, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlor-propylcarbonyloxy, O-CO-$CH_2$-$C_2F_5$, O-CO-$CF_2$-$C_2F_5$, 1-($CH_2F$)-2-fluorethylcarbonyloxy, 1-($CH_2Cl$)-2-chlorethylcarbonyloxy, 1-($CH_2Br$)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, vorzugsweise für O-CO-$CF_3$, O-CO-$CH_2Cl$ oder 2,2,2-Trifluorethylcarbonyloxy;

- ($C_1$-$C_4$-Alkoxy)carbonyl für: CO-$OCH_3$, CO-$OC_2H_5$, n-Propoxycarbonyl, CO-OCH($CH_3$)$_2$, n-Butoxycarbonyl, CO-OCH($CH_3$)-$C_2H_5$, CO-$OCH_2$-CH($CH_3$)2 oder CO-OC($CH_3$)$_3$, vorzugsweise für CO-$OCH_3$ oder CO-$OC_2H_5$;

- ($C_1$-$C_4$-Alkoxy)carbonyl-$C_1$-$C_4$-alkyl für: durch ($C_1$-$C_4$-Alkoxy)-carbonyl - wie vorstehend genannt - substituiertes $C_1$-$C_4$-Alkyl, also z.B. für $CH_2$-CO-$OCH_3$, $CH_2$-CO-$OC_2H_5$, n-Propoxycarbonylmethyl, $CH_2$-CO-OCH($CH_3$)$_2$, n-Butoxycarbonylmethyl, $CH_2$-CO-OCH($CH_3$)-$C_2H_5$, $CH_2$-CO-$OCH_2$-CH($CH_3$)$_2$, $CH_2$-CO-OC($CH_3$)$_3$, 1-(Methoxycarbonyl)ethyl, 1-(Ethoxycarbonyl)ethyl, 1-(n-Propoxycarbonyl)ethyl, 1-(1-Methylethoxycarbonyl)ethyl, 1-(n-Butoxycarbonyl)ethyl, 2-(Methoxycarbonyl)ethyl, 2-(Ethoxycarbonyl)ethyl, 2-(n-Propoxycarbonyl)ethyl, 2-(1-Methylethoxycarbonyl)ethyl, 2-(n-Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(n-Propoxycarbonyl)propyl, 2-(1-Methylethoxycarbonyl)propyl, 2-(n-Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)-propyl, 3-(Ethoxycarbonyl)propyl, 3-(n-Propoxycarbonyl)propyl, 3-(1-Methylethoxycarbonyl)propyl, 3-(n-Butoxycarbonyl) propyl, 3-(1-Methylpropoxycarbonyl)propyl, 3-(2-Methylpropoxycarbonyl)propyl, 3-(1,1-Dimethylethoxycarbonyl) propyl, 2-(Methoxycarbonyl)butyl, 2-(Ethoxycarbonyl)butyl, 2-(n-Propoxycarbonyl)butyl, 2-(1-Methylethoxycarbonyl)butyl, 2-(n-Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(n-Propoxycarbonyl) butyl, 3-(1-Methylethoxycarbonyl)butyl, 3-(n-Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)butyl, 4-(Ethoxycarbonyl)-butyl, 4-(n-Propoxycarbonyl)butyl, 4-(1-Methylethoxycarbonyl)-butyl, 4-(n-Butoxycarbonyl)butyl, 4-(1-Methylpropoxycarbonyl)-butyl, 4-(2-Methylpropoxycarbonyl)butyl oder 4-(1,1-Dimethylethoxycarbonyl)butyl, vorzugsweise für

$CH_2$-CO-$OCH_3$, $CH_2$-CO-$OC_2H_5$, 1-(Methoxycarbonyl)ethyl oder 1-(Ethoxycarbonyl)ethyl;

- ($C_1$-$C_4$-Alkylthio)carbonyl für: CO-$SCH_3$, CO-$SC_2H_5$, CO-$SCH_2$-$C_2H_5$, CO-$SCH(CH_3)_2$, CO-$SCH_2CH_2$-$C_2H_5$, CO-$SCH(CH_3)$-$C_2H_5$, CO-$SCH_2$-$CH(CH_3)_2$ oder CO-$SC(CH_3)_3$, vorzugsweise für CO-$SCH_3$ oder CO-$SC_2H_5$;

- ($C_1$-$C_4$-Alkylthio)carbonyl-$C_1$-$C_4$-alkyl für: durch ($C_1$-$C_4$-Alkylthio)carbonyl - wie vorstehend genannt - substituiertes $C_1$-$C_4$-Alkyl, also z.B. für $CH_2$-CO-$SCH_3$, $CH_2$-CO-$SC_2H_5$, $CH_2$-CO-$SCH_s$-$C_2H_5$, $CH_2$-CO-$SCH(CH_3)_2$, $CH_2$-CO-$SCH_2CH_2$-$C_2H_5$, $CH_2$-CO-$SCH(CH_3)$-$C_2H_5$, $CH_2$-CO-$SCH_2$-$CH(CH_3)$, $CH_2$-CO-$SC(CH_3)$3. 1-(CO-$SCH_3$) ethyl, 1-(CO-$SC_2H_5$)ethyl, 1-(CO-$SCH_2$-$C_2H_5$)ethyl, 1-[CO-$SCH(CH_3)_2$]ethyl, 1-(CO-$SCH_2CH_2$-$C_2H_5$)ethyl, 1-[CO-SCH(CH)-$C_2H_5$]ethyl, 1-[CO-$SCH_2$-$CH(CH_3)_2$]ethyl, 1-[CO-$SC(CH_3)_3$]ethyl, 2-(CO-$SCH_3$)ethyl, 2-(CO-$SC_2H_5$)ethyl, 2-(CO-$SCH_2$-$C_2H_5$)ethyl, 2-[CO-$SCH(CH_3)_2$]ethyl, 2-(CO-$SCH_2CH_O$-$C_2H_5$)ethyl, 2-[CO-SCH ($CH_3$)-$C_2H_5$]ethyl, 2-[CO-$SCH_2$-$CH(CH_3)_2$)ethyl, 2-[CO-$SC(CH_3)_3$]ethyl, 2-(CO-$SCH_3$)-propyl, 2-(CO-$SC_2H_5$)propyl, 2-(CO-$SCH_2$-$C_2H_5$)propyl, 2-[CO-$SCH(CH_3)_2$]propyl, 2-(CO-$SCH_2CH_2$-$C_2H_5$)propyl , 2-[CO-$SCH(CH_3)$-$C_2H_5$] propyl, 2-[CO-$SCH_2$-$CH(CH_3)_2$]propyl, 2-[CO-$SC(CH_3)_3$)propyl, 3-(CO-$SCH_3$)propyl , 3-(CO-$SC_2H_5$)propyl, 3-(CO-$SCH_2$-$C_2H_5$)propyl, 3-[CO-$SCH(CH_3)_2$]propyl, 3-(CO-$SCH_2CH_2$-$C_2H_5$) propyl, 3-[CO-SCH ($CH_3$)-$C_2H_5$) propyl, 3-[CO-$SCH_2$-CH[$CH_3$)$_2$]propyl, 3-[CO-$SC(CH_3)_3$]propyl, 2-(CO-$SCH_3$)butyl, 2-(CO-$SC_2H_5$)butyl, 2-(CO-$SCH_2$-$C_2H_5$)butyl, 2-[CO-$SCH(CH_3)_2$]butyl, 2-(CO-$SCH_2CH_2$-$C_2H_5$)butyl, 2-[CO-$SCH(CH_3)$-$C_2H_5$]butyl, 2-[CO-$SCH_2$-$CH(CH_3)_2$]butyl, 2-(CO-$SC(CH_3)_3$)butyl, 3-(CO-$SCH_3$)butyl, 3-(CO-$SC_2H_5$)butyl, 3-(CO-$SCH_2$-$C_2H_5$)butyl, 3-[CO-$SCH(CH_3)_2$]butyl, 3-(CO-$SCH_2CH_2$-$C_2H_5$)butyl, 3-[CO-$SCH(CH_3)$-$C_2H_5$]butyl, 3-[CO-$SCH_2$-$CH(CH_3)_2$] butyl, 3-[CO-$SC(CH_3)_3$]butyl, 4-(CO-$SCH_3$)-butyl, 4-(CO-$SC_2H_5$)butyl, 4-(CO-$SCH_2$-$C_2H_5$)butyl, 4-[CO-$SCH(CH_3)_2$]butyl, 4-(CO-$SCH_2CH_2$-$C_2H_5$)butyl, 4-[CO-$SCH(CH_3)$-$C_2H_5$]butyl, 4-[CO-$SCH_2$-$CH(CH)_2$]butyl oder 4-[CO-$SC(CH_3)_3$]butyl, vorzugsweise für $CH_2$-CO-$SCH_3$, $CH_2$-CO-$SC_2H_5$, 1-(CO-$SCH_3$)ethyl oder 1-(CO-$SC_2H_5$)ethyl;

- $C_1$-$C_4$-Alkylsulfinyl für: SO-$CH_3$, SO-$C_2H_5$, SO-$CH_2$-$C_2H_5$, SO-$CH(CH_3)_2$, n-Butylsulfinyl, SO-$CH(CH_3)$-$C_2H_5$, SO-$H_2$-$CH(CH_3)_2$ oder SO-$C(CH_3)_3$, vorzugsweise für SO-$CH_3$ oder SO-$C_2H_5$;

- $C_1$-$C_4$-Halogenalkylsulfinyl für: einen $C_1$-$C_4$-Alkylsulfinylrest - wie vorstehend genannt - der partiell oder vollständig durch Fluor, Chlor, Brom und/oder Iod substituiert ist, also z.B. SO-$CH_2F$, SO-$CHF_2$, SO-$CF_3$, SO-$CH_2Cl$, SO-CH $(Cl)_2$, SO-$C(Cl)_3$, Chlorfluormethylsulfinyl, Dichlorfluormethylsulfinyl, Chlordifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2-Chlor-2-fluorethylsulfinyl, 2-Chlor-2,2-difluorethylsulfinyl, 2,2-Dichlor-2-fluorethylsulfinyl, 2, 2, 2-Trichlorethylsulfinyl, SO-$C_2F_5$, 2-Fluorpropylsulfinyl, 3-Fluorpropylsulfinyl, 2,2-Difluorpropylsulfinyl, 2,3-Difluorpropylsulfinyl, 2-Chlorpropyl-5 sulfinyl, 3-Chlorpropylsulfinyl, 2,3-Dichlorpropylsulfinyl, 2-Brompropylsulfinyl, 3-Brompropylsulfinyl, 3,3,3-Trifluorpropylsulfinyl, 3,3,3-Trichlorpropylsulfinyl, SO-$CH_2$-$C_2F_5$, SO-$CF_2$-$C_2F_5$, 1-($CH_2F$)-2-fluorethylsulfinyl, 1-($CH_2Cl$)-2-chlorethylsulfinyl, 1-($CH_2Br$)-2-bromethylsulfinyl, 4-Fluorbutylsulfinyl, 4-Chlorbutylsulfinyl, 4-Brombutylsulfinyl oder Nonafluorbutylsulfinyl, vorzugsweise für SO-$CF_3$, SO-$CH_2Cl$ oder 2,2,2-Trifluorethylsulfinyl;

- $C_1$-$C_4$-Alkylsulfinyl-$C_1$-$C_4$-alkyl für: durch $C_2$-$C_4$-Alkylsulfinyl wie vorstehend genannt substituiertes $C_1$-$C_4$-Alkyl, also z.B. für $CH_2SOCH_3$, $CH_2SOC_2H_5$, n-Propylsulfinylmethyl, $CH_2SOCH(CH_3)_2$, n-Butylsulfinylmethyl, (1-Methylpropylsulfinyl)methyl, (2-Methylpropylsulfinyl)methyl, (1,1-Dimethylethylsulfinyl)-methyl, 2-Methylsulfinylethyl, 2-Ethylsulfinylethyl, 2-(n-Propylsulfinyl)ethyl, 2-(1-Methylethylsulfinyl)ethyl, 2-(n-Butylsulfinyl)ethyl, 2-(1-Methylpropylsulfinyl)ethyl, 2-(2-Methylpropylsulfinyl)ethyl, 2-(1,1-Dimethylethylsulfinyl)ethyl, 2-($SOCH_3$)propyl, 3-($SOCH_3$)propyl, 2-($SOC_2H_5$)-propyl, 3-($SOC_2H_5$)propyl, 3-(Propylsulfinyl)propyl, 3-(Butylsulfinyl)propyl, 4-($SOCH_3$)butyl, 4-($SOC_2H_5$)butyl, 4-(n-Propylsulfinyl)butyl oder 4-(n-Butylsulfinyl)butyl, insbesondere für 2-($SOCH_3$)ethyl;

- $C_1$-$C_4$-Halogenalkylsulfinyl-$C_1$-$C_4$-alkyl für: durch $C_1$-$C_4$-Halogenalkylsulfinyl wie vorstehend genannt substituiertes $C_1$-$C_4$-Alkyl, also z.B. für 2-(2,2,2-Trifluorethylsulfinyl)-ethyl;

- $C_1$-$C_4$-Alkylsulfonyl für: $SO_2$-$CH_3$, $SO_2$-$C_2H_5$, $SO_2$-$CH_2$-$C_2H_5$, $SO_2$-CH $(CH_3)_2$, n-Butylsulfonyl, $SO_2$-$CH(CH_3)$ -$C_2H_5$, $SO_2$-$CH_2$-CH $(CH_3)_2$ oder $SO_2$-$C(CH_3)_3$, vorzugsweise für $SO_2$-$CH_3$ oder $SO_2$-$C_2H_5$;

- $C_1$-$C_4$-Halogenalkylsulfonyl für: einen $C_1$-$C_4$-Alkylsulfonylrest - wie vorstehend genannt - der partiell oder vollständig durch Fluor, Chlor, Brom und/oder Iod substituiert ist, also z.B. $SO_2$-$CH_2F$, $SO_2$-$CHF_2$, $SO_2$-$CF_3$, $SO_2$-$CH_2Cl$, $SO_2$-$CH(Cl)_2$, $SO_2$-$C(Cl)_3$, Chlorfluormethylsulfonyl, Dichlorfluormethylsulfonyl, Chlordifluormethylsulfonyl, 2-Fluorethylsulfonyl, 2-Chlorethyl-sulfonyl, 2-Bromethylsulfonyl, 2-Iodethylsulfonyl, 2,2-Difluorethylsulfonyl,

2,2,2-Trifluorethylsulfonyl, 2-Chlor-2-fluorethylsulfonyl, 2-Chlor-2;2-difluorethylsulfonyl, 2,2-Dichlor-2-fluorethylsulfonyl, 2,2,2-Trichlorethylsulfonyl, $SO_2$-$C_2F_5$, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, $SO_2$-$CH_2$-$C_2F_5$, $SO_2$-$CF_2$-$C_2F_5$, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, vorzugsweise für $SO_2$-$CH_2Cl$, $SO_2$-$CF_3$ oder 2,2,2-Trifluorethylsulfonyl;

- $C_1$-$C_4$-Alkylsulfonyl-$C_1$-$C_4$-alkyl für: durch $C_1$-$C_4$-Alkylsulfonyl wie vorstehend genannt substituiertes $C_1$-$C_4$-Alkyl, also z.B. für $CH_2SO_2$-$CH_3$, $CH_2SO_2$-$C_2H_5$, $CH_2SO_2$-$CH_2$-$C_2H_5$, $CH_2SO_2$-$CH(CH_3)_2$, $CH_2SO_2$-$CH_2CH_2$-$C_2H_5$, (1-Methylpropylsulfonyl)methyl, (2-Methylpropylsulfonyl)methyl, $CH_2SO_2$-$C(CH_3)_3$, $CH(CH_3)SO_2$-$CH_3$, $CH(CH_3)$ $SO_2$-$C_2H_5$, $CH_2CH_2SO_2$-$CH_3$, $CH_2CH_2SO_2$-$C_2H_5$, $CH_2CH_2SO_2$-$CH_2$-$C_2H_5$, $CH_2CH_2SO_2$-$CH(CH_3)_2$, $CH_2CH_2SO_2$-$CH_2CH_2$-$C_2H_5$, 2-(1-Methylpropylsulfonyl)ethyl, 2-(2-Methylpropylsulfonyl)ethyl, $CH_2CH_2SO_2$-C $(CH_3)_3$, 2-($SO_2$-$CH_3$)propyl, 2-($SO_2$-$C_2H_5$)propyl, 2-($SO_2$-$CH_2$-$C_2H_5$)propyl, 2-[$SO_2$-$CH(CH_3)_2$]propyl, 2-($SO_2$-$CH_2CH_2$-$C_2H_5$)propyl, 2-(1-Methylpropylsulfonyl)propyl, 2-(2-Methylpropylsulfonyl)propyl, 2-[$SO_2$-C $(CH_3)_3$]propyl, 3-($SO_2$-$CH_3$)propyl, 3-($SO_2$-$C_5$)$H_5$)propyl, 3-($SO_2$-$CHO_2$-$CH_2H_5$)propyl, 3-[$SO_2$-$CH(CH_3)_2$]propyl, 3-($SO_2$-$CH_2CH_2$-$C_2H_5$)propyl, 3-(1-Methylpropylsulfonyl)propyl, 3-(2-Methylpropylsulfonyl)propyl, 3-[$SO_2$-C $(CH_3)_3$]propyl, 2-($SO_2$-$CH_3$)butyl, 2-($SO_2$-$C_2H_5$)butyl, 2-($SO_2$-$CH_2$-$C_2H_5$)butyl, 2-[$SO_2$-$CH(CH_3)_2$]butyl , 2-($SO_2$-$CH_2CH_2$-$C_2H_5$)butyl, 2-(1-Methylpropylsulfonyl)butyl, 2-(2-Methylpropylsulfonyl)butyl, 2-[$SO_2$-$C(CH_3)_3$] butyl, 3-($SO_2$-$CH_3$)butyl, 3-i$SO_2$-$C_2H_5$)butyl, 3-($SO_2$-$CH_2$-$C_2H_5$)butyl, 3- [$SO_2$-$CH(CH_3)_2$]butyl, 3-($SO_2$-$CH_2CH_2$-$C_2H_5$)butyl, 3-(1-Methylpropylsulfonyl)butyl, 3-(2-Methylpropylsulfonyl)butyl, 3-(SO2-$C(CH_3)_3$] butyl, 4-($SO_2$-$CH_3$)butyl, 4-($SO_2$-$C_2H_5$)butyl, 4-($SO_2$-$CH_2$-$C_2H_5$)butyl, 4-[$SO_2$-$CH(CH_3)_2$]butyl, 4-($SO_2$-$CH_2CH_2$-$C_2H_5$)butyl, 4-(1-Methylpropylsulfonyl)butyl, 4-(2-Methylpropylsulfonyl)butyl oder 4-[$SO_2$-C $(CH_3)_3$]butyl, insbesondere für $CH_2CH_2SO_2$-$CH_3$ oder $CH_2CH_2SO_2$-$C_2H_5$;

- $C_1$-$C_4$-Halogenalkylsulfonyl-$C_1$-$C_4$-alkyl für: durch $C_1$-$C_4$-Halogenalkylsulfonyl wie vorstehend genannt substituiertes $C_1$-$C_4$-Alkyl, also z.B. für 2-(2,2,2-Trifluorethylsulfonyl)-ethyl;

- $C_1$-$C_4$-Alkylamino-$C_1$-$C_4$-alkyl für: durch $C_1$-$C_4$-Alkylamino wie $H_3C$-NH-, $H_5C_2$-NH-, n-Propyl-NH-, 1-Methylethyl-NH-, n-Butyl-NH-, 1-Methylpropyl-NH-, 2-Methylpropyl-NH- und 1,1-Dimethylethyl-NH-, vorzugsweise $H_3C$-NH- oder $H_3C_2$-NH-, substituiertes $C_1$-$C_4$-Alkyl, also beispielsweise für $CH_2CH_2$-NH-$CH_3$, $CH_2CH_2$-$N(CH_3)_2$, $CH_2CH_2$-NH·$C_2H_5$ oder $CH_2CH_2$-$N(C_2H_5)_2$;

- $C_1$-$C_4$-Alkylaminocarbonyl für: CO-NH-$CH_3$, CO-NH-$C_2H_5$, n-Propyl-amino, CO-NH-$CH(CH_3)_2$, CO-NH-$CH_2CH_2$-$C_2H_5$, CO-NH-$CH(CH_3)$-$C_2H_5$, CO-NH-$CH_2$-$CH(CH_3)_2$ oder CO-NH-$C(CH_3)_3$, vorzugsweise für CO-NH-$CH_3$ oder CO-NH-$C_2H_5$;

- $C_1$-$C_4$-Alkylaminocarbonyl-$C_1$-$C_4$-alkyl für: $C_1$-$C_4$-Alkylamino-carbonyl wie vorstehend genannt, vorzugsweise CO-NH-$CH_3$ oder CO-NH-$C_2H_5$, substituiertes $C_1$-$C_4$-Alkyl, als z.B. für $CH_2$-CO-NH-$CH_3$, $CH_2$-CO-NH-$C_2H_5$, $CH_2$-CO-NH-$CH_2$-$C_2H_5$, $CH_2$-CO-NH-$CH(CH_3)_2$, $CH_2$-CO-NH-$CH_2CH_2$-$C_2H_5$, $CH_2$-CO-NH-$CH(CH_3)$-$C_2H_5$, $CH_2$-CO-NH-$CH_2$-$CH(CH_3)_2$, $CH_2$-CO-NH-$C(CH_3)_3$, $CH(CH_3)$-CO-NH-$CH_3$, $CH(CH_3)$-CO-NH-$C_2H_5$, 2-(CO-NH-$CH_3$)ethyl, 2-(CO-NH-$C_2H_5$)ethyl, 2-(CO-NH-$CH_2$-$C_2H_5$)-ethyl, 2-[$CH_2$-CO-NH-$CH(CH_3)_2$]ethyl, 2-(CO-NH-$CH_2CH_2$-$C_2H_5$)ethyl, 2-[CO-NH-$CH(CH_3)$-$C_2H_5$]ethyl, 2-[CO-NH-$CH_2$-$CH(CH_3)_2$]ethyl, 2-[CO-NH-C $(CH_3)_3$]ethyl, 2-(CO-NH-$CH_3$)propyl, 2-(CO-NH-$C_2H_5$)propyl, 2-(CO-NH-$CH_2$-$C_2H_5$)propyl, 2-[$CH_2$-CO-NH-CH $(CH_3)_2$]propyl, 2-(CO-NH-$CH_2CH_2$-$C_2H_5$)propyl, 2-[CO-NH-$CH(CH_3)$-$C_2H_5$]propyl, 2-[CO-NH-$CH_2$-$CH(CH_3)_2$] propyl, 2-[CO-NH-$C(CH_3)_3$]propyl, 3-(CO-NH-$CH_3$)propyl, 3-(CO-NH-$C_2H_5$)propyl, 3-(CO-NH-$CH_2$-$C_2H_5$)propyl, 3-[$CH_2$-CO-NH-$CH(CH_3)_2$]propyl, 3-(CO-NH-$CH_2CH_2$-$C_2H_5$)propyl, 3-[CO-NH-$CH(CH_3)$-$C_2H_5$]propyl, 3-[CO-NH-$CH_2$-$CH(CH_3)_2$]propyl, 3-[CO-NH-$C(CH_3)_3$]propyl, 2-(CO-NH-$CH_3$)butyl, 2-(CO-NH-$C_2H_5$)-butyl, 2-(CO-NH-$CH_2$-$C_2H_5$)butyl, 2-[$CH_2$-CO-NH-$CH(CH_3)_2$]butyl, 2-(CO-NH-$CH_2CH_2$-$C_2H_5$)butyl, 2-[CO-NH-$CH(CH_3)$ -$C_2H_5$]butyl, 2-[CO-NH-$CH_2$-$CH(CH_3)_2$]butyl, 2-[CO-NH-$C(CH_3)_3$]butyl, 3-(CO-NH-$CH_3$)butyl, 3-(CO-NH-$C_2H_5$)butyl, 3-(CO-NH-$CH_2$-$C_2H_5$)-butyl, 3-[$CH_2$-CO-NH-$CH(CH_3)_2$]butyl, 3-(CO-NH-$CH_2CH_2$-$C_2H_5$)butyl, 3-[CO-NH-CH $(CH_3)$-$C_2H_5$]butyl, 3-[CO-NH-$CH_2$-$CH(CH_3)_2$]butyl, 3-[CO-NH-$C(CH_3)_3$]butyl, 4-(CO-NH-$CH_3$)butyl, 4-(CO-NH-$C_2H_5$)butyl, 4-(CO-NH-$CH_2$-$C_2H_5$)butyl, 4-[$CH_2$-CO-NH-$CH(CH_3)_2$]butyl, 4-(CO-NH-$CH_2CH_2$-$C_2H_5$)butyl, 4-[CO-NH-$CH(CH_3)$-$C_2H_5$]butyl, 4-[CO-NH-$CH_2$-$CH(CH_3)_2$]butyl oder 4-[CO-NH-$C(CH_3)_3$]butyl, vorzugsweise für $CH_2$-CO-NH-$CH_3$, $CH_2$-CO-NH-$C_2H_5$, $CH(CH_3)$-CO-NH-$CH_3$ oder $CH(CH_3)$-CO-NH-$C_2H_5$;

- Di($C_1$-$C_4$-alkyl)amino für: $N(CH_3)_2$, $N(C_2H_5)_2$, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl) amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N- (1-methylethyl) amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl) amino, N-Butyl-N-(2-methylpropyl)amino, N-Butyl-N-(1,1-dimethylethyl)-amino, N-(1-Methylpropyl)-N-(2-methylpropyl) amino, N-(1,1-Dimethylethyl)-N-(1-methylpropyl)amino oder N-(1,1-Dimethylethyl)-N-(2-methylpropyl)amino, vorzugsweise für $N(CH_3)_2$ oder $N(C_2H_5)2$;

- Di($C_1$-$C_4$-alkyl)amino-$C_1$-$C_4$-alkyl für: durch Di($C_1$-$C_4$-alkyl)-amino wie vorstehend genannt substituiertes $C_1$-$C_4$-Alkyl, also z.B. für $CH_2N(CH_3)_2$, $CH_2N(C_2H_5)_2$, N,N-Dipropylaminomethyl, N,N-Di(CH$(CH_3)_2$]aminomethyl, N,N-Dibutylaminomethyl, N,N-Di-(1-methylpropyl)aminomethyl, N,N-Di (2-methylpropyl) aminomethyl, N,N-Di($C(CH_3)_3$] aminomethyl, N-Ethyl-N-methylaminomethyl, N-Methyl-N-propylaminomethyl, N-Methyl-N-($CH(CH_3)_2$]aminomethyl, N-Butyl-N-methylaminomethyl, N-Methyl-N-(1-methylpropyl)aminomethyl, N-Methyl-N-(2-methylpropyl)aminomethyl, N-[$C(CH_3)_3$]-N-methylaminomethyl, N-Ethyl-N-propylaminomethyl, N-Ethyl-N-[$CH(CH_3)_2$]aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-[$C(CH_3)_3$]aminomethyl, N-($CH(CH_3)_2$]-N-propylaminomethyl, N-Butyl-N-propylaminomethyl, N-(1-Methylpropyl)-N-propylaminomethyl, N-(2-Methylpropyl)-N-propylaminomethyl, N-[$C(CH_3)_3$]-N-propylaminomethyl, N-Butyl-N-(1-methylethyl)-aminomethyl, N-[$CH(CH_3)_2$]-N-(1-methylpropyl)aminomethyl, N-[$CH(CH_3)_2$]-N-(2-methylpropyl)aminomethyl, N-[$C(CH_3)_3$]-N-[$CH(CH_3)_2$]aminomethyl, N-Butyl-N-(1-methylpropyl)aminomethyl, N-Butyl-N-(2-methylpropyl)aminomethyl, N-Butyl-N-[$C(CH_3)_3$]-aminomethyl, N-(1-Methylpropyl)-N-(2-methylpropyl) aminomethyl, N-[$C(CH_3)_3$]-N-(1-methylpropyl)aminomethyl, N-[$C(CH_3)_3$]-N-(2-methylpropyl)aminomethyl, N,N-Dimethylaminoethyl, N,N-Diethylaminoethyl, N,N-Di(n-propyl)aminoethyl, N,N-Di-[$CH(CH_3)_2$]-aminoethyl, N,N-Dibutylaminoethyl, N,N-Di(1-methylpropyl)aminoethyl, N,N-Di(2-methylpropyl)aminoethyl, N,N-Di-[$C(CH_3)_3$]-aminoethyl, N-Ethyl-N-methylaminoethyl, N-Methyl-N-propylaminoethyl, N-Methyl-N-[$CH(CH_3)_2$]aminoethyl, N-Butyl-N-methylaminoethyl, N-Methyl-N-(1-methylpropyl)aminoethyl, N-Methyl-N-(2-methylpropyl)aminoethyl, N-[$C(CH_3)_3$] -N-methylaminoethyl, N-Ethyl-N-propylaminoethyl, N-Ethyl-N-[$CH(CH_3)_2$]aminoethyl, N-Butyl-N-ethylaminoethyl, N-Ethyl-N-(1-methylpropyl)aminoethyl, N-Ethyl-N-(2-methylpropyl)aminoethyl, N-Ethyl-N-[$C(CH_3)_3$]aminoethyl, N-[$CH(CH_3)_2$]-N-propylaminoethyl, N-Butyl-N-propylaminoethyl, N-(1-Methylpropyl)-N-propylaminoethyl, N-(2-Methylpropyl)-N-propylaminoethyl, N-[$C(CH_3)_3$]-N-propylaminoethyl, N-Butyl-N-[$CH(CH_3)_2$]aminoethyl, N-[CH $(CH_3)_2$]-N-(1-methylpropyl)aminoethyl, N-[$CH(CH_3)_2$]-N-(2-methylpropyl)-aminoethyl, N-[$C(CH_3)_3$]-N-[$CH(CH_3)_2$] aminoethyl, N-Butyl-N-(1-methylpropyl)aminoethyl, N-Butyl-N-(2-methylpropyl)aminoethyl, N-Butyl-N-[$C(CH_3)_3$] aminoethyl, N-(1-Methylpropyl)-N-(2-methylpropyl)aminoethyl, N-[$C(CH_3)_3$]-N-(1-methylpropyl)-aminoethyl oder N-[$C(CH_3)_3$]-N-(2-methylpropyl)aminoethyl, insbesondere für N,N-Dimethylaminoethyl oder N,N-Diethylaminoethyl;

- Di($C_1$-$C_4$-alkyl)aminocarbonyl für: CO-$N(CH_3)_2$, CO-$N(C_2H_5)$, CO-N($CH_2$-$C_2H_5)_2$, CO-N[$CH(CH_3)_2]_2$, N,N-Dibutylaminocarbonyl, CO-N[$CH(CH_3)$-$C_2H_5]_2$, CO-N[$CH_2$-$CH(CH_3)_2]_2$, CO-N[$C(CH_3)_3]_2$, N-Ethyl-N-methylaminocarbonyl, N-Methyl-N-propylaminocarbonyl, N-Methyl-N-[$CH(CH_3)_2$]aminocarbonyl, N-Butyl-N-methylaminocarbonyl, N-Methyl-N-(1-methylpropyl)aminocarbonyl, N-Methyl-N-(2-methylpropyl)aminocarbonyl, N-[$C(CH_3)_3$]-N-methylamino-carbonyl, N-Ethyl-N-propylaminocarbonyl, N-Ethyl-N-[$CH(CH_3)_2$]-aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-[C $(CH_3)_3$]aminocarbonyl, N-[$CH(CH_3)_2$]-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-[$C(CH_3)_3$]-N-propylaminocarbonyl, N-Butyl-N-[$CH(CH_3)_2$]aminocarbonyl,N-[$CH(CH_3)_2$]-N-(1-methylpropyl)aminocarbonyl,N-[$CH(CH_3)_2$]-N-(2-methylpropyl)aminocarbonyl, N-[$C(CH_3)_3$]-N-[$CH(CH_3)_2$]aminocarbonyl, N-Butyl-N-(1-methylpropyl)aminocarbonyl, N-Butyl-N-(2-methylpropyl)-aminocarbonyl, N-Butyl-N-[$C(CH_3)_3$]aminocarbonyl, N-(1-Methylpropyl)-N-(2-methylpropyl)aminocarbonyl, N-[$C(CH_3)_3$]-N-(1-methylpropyl)aminocarbonyl oder N-[$C(CH_3)_3$]-N-(2-methylpropyl)aminocarbonyl, vorzugsweise für CO-$N(CH_3)_2$ oder CO-$N(C_2H_5)_2$;

- Di($C_1$-$C_4$-alkyl)aminocarbonyl-$C_1$-$C_4$-alkyl für: durch Di($C_1$-$C_4$alkyl)aminocarbonyl wie vorstehend genannt, vorzugsweise CO-$N(CH_3)_2$ oder CO-$N(C_2H_5)$, substituiertes $C_1$-$C_4$-Alkyl, also z.B. für $CH_2$-CO-$N(CH_3)_2$, $CH_2$-CO-N $(C_2H_5)_2$, $CH(CH_3)$-CO-$N(CH_3)_2$ oder $CH(CH_3)$-CO-$N(C_2H_5)_2$, vorzugsweise für $CH_2$-CO-$N(CH_3)_2$ oder $CH(CH_3)$ -CO-$N(CH_3)_2$;

- Di($C_1$-$C_4$-alkyl)phosphonyl für: -$PO(OCH_3)_2$, -$PO(OC_2H_5)_2$, N,N-Dipropylphosphonyl, N,N-Di-(1-methylethyl)phosphonyl, N,N-Dibutylphosphonyl, N,N-Di-(1-methylpropyl)phosphonyl, N,N-Di-(2-methylpropyl)phosphonyl, N,N-Di-(1,1-dimethylethyl)phosphonyl, N-Ethyl-N-methylphosphonyl, N-Methyl-N-propylphosphonyl, N-Methyl-N-(1-methylethyl)phosphonyl, N-Butyl-N-methylphosphonyl, N-Methyl-N-(1-methylpropyl)-phosphonyl, N-Methyl-N-(2-methylpropyl)phosphonyl, N-(1,1-Dimethylethyl)-N-methylphosphonyl, N-Ethyl-N-propylphosphonyl, N-Ethyl-N-(1-methylethyl)phosphonyl, N-Butyl-N-ethylphosphonyl, N-Ethyl-N-(1-methylpropyl)phosphonyl, N-Ethyl-N-(2-methylpropyl)phosphonyl, N-Ethyl-N-(1,1-dimethylethyl)phosphonyl, N-(1-Methylethyl)-N-propylphosphonyl, N-Butyl-N-propylphosphonyl, N-(1-Methylpropyl)-N-propylphosphonyl, N-(2-Methylpropyl)-N-propylphosphonyl, N-(1,1-Dimethylethyl)-N-propylphosphonyl, N-Butyl-N-(1-methylethyl)phosphonyl, N-(1-Methylethyl) -N-(1-methylpropyl)phosphonyl,N-(1-Methylethyl)-N-(2-methylpropyl)phosphonyl,N-(1,1-Dimethylethyl)-N-(1-methylethyl)phosphonyl, N-Butyl-N-(1-methylpropyl)phosphonyl, N-Butyl-N-(2-methylpropyl)phosphonyl, N-Butyl-N-(1,1-dimethylethyl)phosphonyl, N-(1-Methylpropyl)-N-(2-methylipropyl)-phosphonyl, N-(1,1-Dimethylethyl) -N-(1-methypropyl)phosphonyl oder N-(1,1-Dimethylethyl)-N-(2-methylpropyl)phosphonyl, vorzugsweise für -PO$(OCH_3)_2$ oder -PO$(OC_2H_5)_2$;

- Di($C_1$-$C_4$-alkyl)phosphonyl-$C_1$-$C_4$-alkyl für: durch Di($C_1$-$C_4$-alkyl)phosphonyl wie vorstehend genannt, vorzugsweise -PO$(OCH_3)_2$ oder -PO$(OC_2H_5)_2$, substituiertes $C_1$-$C_4$-Alkyl, also z.B. für $CH_2$-PO$(OCH_3)_2$, $CH_2$-PO$(OC_2H_5)_2$, $CH(CH_3)$-PO$(OCH_3)_2$ oder $CH(CH_3)$ -PO$(OC_2H_5)_2$;

- $C_3$-$C_6$-Alkenyl für: Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl-, 1-Methylprop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methylbut-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-2-en-1-yl, 1-Methylpent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methylpent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut -1- en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;

- $C_3$-$C_6$-Halogenalkenyl für: $C_3$-$C_6$-Alkenyl wie vorstehend genannt, das partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;

- Cyano-$C_3$-$C_6$-alkenyl für: z.B. 2-Cyanoallyl, 3-Cyanoallyl, 4-Cyanobut-2-enyl, 4-Cyanobut-3-enyl oder 5-Cyanopent-4-enyl;

- $C_3$-$C_6$-Alkinyl für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für Prop-2-in-1-yl;

- $C_3$-$C_6$-Halogenalkinyl für: $C_3$-$C_6$-Alkinyl wie vorstehend genannt, das partiell oder vollständig durch Fluor, Chlor, Brom und/oder Iod substituiert ist, also z.B. 1,1-Difluorprop-2-in-1-yl, 4-Fluorbut-2-in-1-yl, 4-Chlorbuc-2-in-1-yl, 1,1-Difluorbut-2-in-1-yl, 5-Fluorpent-3-in-1-yl oder 6-Fluorhex-4-in-1-yl;

- Cyano-$C_3$-$C_6$-alkinyl für: z.B. 3-Cyanopropargyl, 4-Cyanobut-2-in-1-yl, 5-Cyanopent-3-in-1-yl und 6-Cyanohex-4-in-1-yl;

- $C_3$-$C_4$-Alkenyloxy-$C_1$-$C_4$-alkyl für: durch $C_3$-$C_4$-Alkenyloxy wie Allyloxy, But-1-en-3-yloxy, But-1-en-4-yloxy, But-2-en-1-yloxy. 1-Methylprop-2-enyloxy oder 2-Methylprop-2-enyloxy substituiertes $C_1$-$C_4$-Alkyl, also beispielsweise für Allyloxymethyl, 2-Allyloxyethyl oder But-1-en-4-yloxymethyl, insbesondere für 2-Allyloxyethyl;

- $C_3$-$C_4$-Alkinyloxy-$C_1$-$C_4$-alkyl für: durch $C_3$-$C_4$-Alkinyloxy wie Propargyloxy, But-1-in-3-yloxy, But-1-in-4-yloxy, But-2-in-1-yloxy, 1-Methylprop-2-inyloxy oder 2-Methylprop-2-inyloxy, vorzugsweise Propargyloxy, substituiertes $C_1$-$C_4$-Alkyl, also beispielsweise für Propargyloxymethyl oder 2-Propargyloxyethyl, insbesondere für 2-Propargyloxyethyl;

- ($C_3$-$C_4$-Alkenyloxy)imino-$C_1$-$C_4$-alkyl für: durch ($C_3$-$C_4$-Alkenyloxy)imino wie Allyloxyimino, But-1-en-3-yloxyimino, But-1-en-4-yloxyimino, But-2-en-1-yloxyimino, 1-Methylprop-2-enyloxyimino oder 2-Methylprop-2-enyloxyimino substituiertes $C_1$-$C_4$-Alkyl, also beispielsweise für Allyloxy-N=CH-$CH_2$ oder But-1-en-4-yloxy-N=CH, insbesondere für Allyloxy-N=CH-$CH_2$;

- $C_3$-$C_4$-Alkenylthio-$C_1$-$C_4$-alkyl für: durch $C_3$-$C_4$-Alkenylthio wie Allylthio, But-1-en-3-ylthio, But-1-en-4-ylthio, But-2-en-1-ylthio, 1-Methylprop-2-enylthio oder 2-Methylprop-2-enylthio substituiertes $C_1$-$C_4$-Alkyl, also beispielsweise für Allylthiomethyl, 2-Allylthioethyl oder But-1-en-4-ylthiomethyl, insbesondere für 2-(Allylthio)ethyl;

- $C_3$-$C_4$-Alkinylthio-$C_1$-$C_4$-alkyl für: durch $C_3$-$C_4$-Alkinylthio wie Propargylthio, But-1-in-3-ylthio, But-1-in-4-ylthio, But-2-in-1-ylthio, 1-Methylprop-2-inylthio oder 2-Methylprop-2-inylthio, vorzugsweise Propargylthio, substituiertes $C_1$-$C_4$-Alkyl, also beispielsweise für Propargylthiomethyl oder 2-Propargylthioethyl, insbesondere für 2-(Propargylthio)-ethyl;

- $C_3$-$C_4$-Alkenylsulfinyl-$C_1$-$C_4$-alkyl für: durch $C_3$-$C_4$-Alkenylsulfinyl wie Allylsulfinyl, But-1-en-3-ylsulfinyl, But-1-en-4-ylsulfinyl, But-2-en-1-ylsulfinyl, 1-Methylprop-2-enylsulfinyl oder 2-Methylprop-2-enylsulfinyl substituiertes $C_1$-$C_4$-Alkyl, also beispielsweise für Allylsulfinylmethyl, 2-Allylsulfinylethyl oder But-1-en-4-ylsulfinylmethyl, insbesondere für 2-(Allylsulfinyl)ethyl;

- $C_3$-$C_4$-Alkinylsulfinyl-$C_1$-$C_4$-alkyl für: durch $C_3$-$C_4$-Alkinylsulfinyl wie Propargylsulfinyl, But-1-in-3-ylsulfinyl, But-1-in-4-ylsulfinyl, But-2-in-1-ylsulfinyl, 1-Methylprop-2-inylsulfinyl oder 2-Methylprop-2-inylsulfinyl, vorzugsweise Propargylsulfinyl, substituiertes $C_1$-$C_4$-Alkyl, also beispielsweise für Propargylsulfinylmethyl oder 2-Propargylsulfinylethyl, insbesondere für 2-(Propargylsulfinyl)ethyl;

- $C_3$-$C_4$-Alkenylsulfonyl-$C_1$-$C_4$-alkyl für: durch $C_3$-$C_4$-Alkenylsulfonyl wie Allylsulfonyl, But-1-en-3-ylsulfonyl, But-1-en-4-ylsulfonyl, But-2-en-1-ylsulfonyl, 1-Methylprop-2-enylsulfonyl oder 2-Methylprop-2-enylsulfonyl substituiertes $C_1$-$C_4$-Alkyl, also beispielsweise für Allylsulfonyimethyl, 2-Allylsulfonylethyl oder But-1-en-4-ylsuffonylmethyl, insbesondere für 2-(Allylsulfonyl)ethyl;

- $C_3$-$C_4$-Alkinylsulfonyl-$C_1$-$C_4$-alkyl für: durch $C_3$-$C_4$-Alkinylsulfonyl wie Propargylsulfonyl, But-1-in-3-ylsulfonyl, But-1-in-4-ylsulfonyl, But-2-in-1-ylsulfonyl, 1-Methylprop-2-inylsulfonyl oder 2-Methylprop-2-inylsulfonyl, vorzugsweise Propargyl-sulfonyl, substituiertes $C_1$-$C_4$-Alkyl, also beispielsweise für Propargylsulfonylmethyl oder 2-Propargylsulfonylethyl, insbesondere für 2-(Propargylsulfonyl)ethyl;

- $C_3$-$C_8$-Cycloalkyl für: Cyclopropyl, Cyclobutyl, Cyclopentyl, Cyclohexyl, Cycloheptyl oder Cyclooctyl;

- $C_3$-$C_8$-Cycloalkyl-$C_1$-$C_6$-alkyl für: z.B. Cyclopropylmethyl, Cyclobutylmethyl, Cyclopentylmethyl, Cyclohexylmethyl, Cycloheptylmethyl, Cyclooctylmethyl, 2-(Cyclopropyl)ethyl, 2-(Cyclobutyl)-ethyl, 2-(Cyclopentyl)ethyl, 2-(Cyclohexyl)ethyl, 2-(Cycloheptyl)ethyl, 2-(Cyclooctyl)ethyl, 3-(Cyclopropyl)propyl, 3-(Cyclobutyl)propyl, 3-(Cyclopentyl)propyl, 3-(Cyclohexyl)-propyl, 3-(Cycloheptyl)propyl, 3-(Cyclooctyl)propyl, 4-(Cyclopropyl)butyl, 9-(Cyclobutyl) butyl, 4-(Cyclopentyl)butyl, 4-(Cyclohexyl)butyl, 4-(Cycloheptyl)butyl, 4-(Cyclooctyl)butyl, 5-(Cyclopropyl)pentyl, 5-(Cyclobutyl)pentyl, 5-(Cyclopentyl)-pentyl, 5-(Cyclohexyl)pentyl, 5-(Cycloheptyl)pentyl, 5-(Cyclooctyl)pentyl, 6-(Cyclopropyl)hexyl, 6-(Cyclobutyl)hexyl, 6-(Cyclopentyl)hexyl, 6-(Cyclohexyl)hexyl, 6-(Cycloheptyl)hexyl oder 6-(Cyclooctyl)hexyl;

- $C_3$-$C_8$-Cycloalkyl, das ein Carbonyl- oder Thiocarbonyl-Ringglied enthält, z.B. für Cyclobutanon-2-yl, Cyclobutanon-3-yl, Cyclopentanon-2-yl, Cyclopentanon-3-yl, Cyclohexanon-2-yl, Cyclohexanon-4-yl, Cycloheptanon-2-yl, Cyclooctanon-2-yl, Cyclobutanthion-2-yl, Cyclobutanthion-3-yl, Cyclopentanthion-2-yl, Cyclopentanthion-3-yl, Cyclohexanthion-2-yl, Cyclohexanthion-4-yl, Cycloheptanthion-2-yl oder Cyclooctanthion-2-yl, vorzugsweise für Cyclopentanon-2-yl oder Cyclohexanon-2-yl;

- $C_3$-$C_8$-Cycloalkyl-$C_1$-$C_4$-alkyl, das ein Carbonyl- oder Thiocarbonyl-Ringglied enthält, für: z.B. Cyclobutanon-2-ylmethyl, Cyclobutanon-3-ylmethyl, Cyclopentanon-2-ylmethyl, Cyclopentanon-3-ylmethyl, Cyclohexanon-2-ylmethyl, Cyclohexanon-4-ylmethyl, Cycloheptanon-2-ylmethyl, Cyclooctanon-2-ylmethyl, Cyclobutanthion-2-ylmethyl, Cyclobutanthion-3-ylmethyl, Cyclopentanthion-2-ylmethyl, Cyclopentanthion-3-ylmethyl, Cyclohexanthion-2-ylmethyl, Cyclohexanthion-4-ylmethyl, Cycloheptanthion-2-ylmethyl, Cyclooctanthion-2-ylmethyl, 1-(Cyclobutanon-2-yl)ethyl, 1-(Cyclobutanon-3-yl)ethyl, 1-(Cyclopentanon-2-yl)-ethyl, 1-(Cyclopentanon-3-yl)ethyl, 1-(Cyclohexanon-2-yl)ethyl, 1-(Cyclohexanon-4-yl)ethyl, 1-(Cycloheptanon-2-yl)ethyl, 1-(Cyclooctanon-2-yl)ethyl, 1-(Cyclobutanthion-2-yl)ethyl, 1-(Cyclobutanthion-3-yl)ethyl, 1-(Cyclopentanthion-2-yl)ethyl, 1-(Cyclopentanthion-3-yl)ethyl, 1-(Cyclohexanthion-2-yl)ethyl, 1-(Cyclohexanthion-4-yl)ethyl, 1-(Cycloheptanthion-2-yl)ethyl, 1-(Cyclooctanthion-2-yl)ethyl, 2-(Cyclobutanon-2-yl)ethyl, 2-(Cyclobutanon-3-yl)ethyl, 2-(Cyclopentanon-2-yl)ethyl, 2-(Cyclopentanon-3-yl)ethyl, 2-(Cyclohexanon-2-yl)ethyl, 2-(Cyclohexanon-4-yl)ethyl, 2-(Cycloheptanon-2-yl)ethyl, 2-(Cyclooctanon-2-yl)ethyl, 2-(Cyclobutanthion-2-yl)ethyl, 2-(Cyclobutanthion-3-yl)ethyl, 2-(Cyclopentanthion-2-yl)ethyl, 2-(Cyclopentanthion-3-yl)ethyl, 2-(Cyclohexanthion-2-yl)ethyl, 2-(Cyclohexanthion-4-yl)ethyl, 2-(Cycloheptanthion-2-yl)ethyl, 2-(Cyclooctanthion-2-yl) ethyl, 3-(Cyclobutanon-2-yl)propyl, 3-(Cyclobutanon-3-yl)propyl, 3-(Cyclopentanon-2-yl)propyl, 3-(Cyclopentanon-3-yl)propyl, 3-(Cyclohexanon-2-yl)propyl, 3-(Cyclohexanon-4-yl)propyl, 3-(Cycloheptanon-2-yl)propyl, 3-(Cyclooctanon-2-yl)propyl, 3-(Cyclobutanthion-2-yl)propyl, 3-(Cyclobutanthion-3-yl)propyl, 3-(Cyclopentanthion-2-yl)-propyl, 3-(Cyclopentanthion-3-yl)propyl, 3-(Cyclohexanthion-2-yl)propyl, 3-(Cyclohexanthion-4-yl)propyl, 3-(Cycloheptanthion-2-yl)propyl, 3-(Cyclooctanthion-2-yl)propyl, 4-(Cyclobutanon-2-yl)butyl, 4-(Cyclobutanon-3-yl)butyl, 4-(Cyclopentanon-2-yl)butyl, 4-(Cyclopentanon-3-yl)butyl, 4-(Cyclohexanon-2-yl)butyl, 4-(Cyclohexanon-4-yl)butyl, 4-(Cyclohep-tanon-2-yl)butyl, 4-(Cyclooctanon-2-yl)butyl, 4-(Cyclobutanthion-2-yl)butyl, 4-(Cyclobutanthion-3-yl)butyl, 4-(Cyclopentanthion-2-yl)butyl, 4-(Cyclopentanthion-3-yl)butyl, 4-(Cyclonexanthion-2-yl)butyl, 4-(Cyclohexanthion-4-yl)butyl, 4-(Cycloneptanthion-2-yl)butyl oder 4-(Cyclooctanthion-2-yl)butyl;

- $C_3$-$C_8$-Cycloalkyloxy-$C_1$-$C_4$-alkyl für: Cyclopropyloxymethyl, 1-Cyclopropyloxy-ethyl, 2-Cyclopropyloxy-ethyl, 1-Cyclopropyloxy-prop-1-yl, 2-Cyclopropyloxy-prop-1-yl, 3-Cyclopropyloxyprop-1-yl, 1-Cyclopropyloxy-but-1-yl, 2-Cyclopropyloxy-but-1-yl, 3-Cyclopropyloxy-but-1-yl, 4-Cyclopropyloxy-but-1-yl, 1-Cyclopropyloxy-but-2-yl, 2-Cyclopropyloxy-but-2-yl, 3-Cyclopropyloxy-but-2-yl, 3-Cyclopropyloxy-but-2-yl, 4-Cyclopropyloxy-but-2-yl, 1-(Cyclopropyloxymethyl)-eth-1-yl, 1-(Cyclopropyloxymetlhyl)-1-($CH_3$) -eth-1-yl, 1-(Cyclopropylmethyloxy)-prop-1-yl, Cyclobutyloxymethyl, 1-Cyclobutyloxy-ethyl, 2-Cyclobutyloxy-ethyl, 1-Cyclobutyloxy-prop-1-yl, 2-Cyclobutyloxyprop-1-yl, 3-Cyclobutyloxy-prop-1-yl, 1-Cyclobutyloxy-but-1-yl, 2-Cyclobutyloxy-but-1-yl, 3-Cyclobutyloxy-but-1-yl, 4-Cyclobutyloxy-but-1-yl, 1-Cyclobutyloxy-but-2-yl, 2-Cyclobutyloxybut-2-yl, 3-Cyclobutyloxy-but-2-yl, 3-Cyclobutyloxy-but-2-yl, 4-Cyclobutyloxy-but-2-yl, 1-(Cyclobutyloxymethyl)eth-1-yl, 1-(Cyclobutyloxymethyl)-1-($CH_3$)-eth-1-yl, 1-(Cyclobutyloxymethyl)prop-1-yl, Cyclopentyloxymethyl, 1-Cyclopentyloxy-ethyl, 5 2-Cyclopentyloxy-ethyl, 1-Cyclopentyloxy-grop-1-yl, 2-Cyclopentyloxy-prop-1-yl, 3-Cyclopentyloxy-prop-1-yl, 1-Cyclopentyloxy-but-1-yl, 2-Cyclopentyloxy-but-1-yl, 3-Cyclopentyloxy-but-1-yl, 4-Cyclopentyloxy-but-1-yl, 1-Cyclopentyloxy-but-2-yl, 2-Cyclopentyloxy-but-2-yl, 3-Cyclopentyloxy-but-2-yl, 3-Cyclo-0 pentyloxy-but-2-yl, 4-Cyclopentyloxy-but-2-yl, 1-(Cyclopentyloxymethyl)eth-1-yl, 1-(Cyclopentyloxymethyl)-1-($CH_3$)-eth-1-yl, 1-(Cyclopentyloxymethyl)prop-1-yl, Cyclohexyloxymethyl, 1-Cyclohexyloxy-ethyl, 2-Cyclohexyloxy-ethyl, 1-Cyclohexyloxyprop-1-yl, 2-Cyclohexyloxy-prop-1-yl, 3-Cyclohexyloxy-prop-5 1-yl, 1-Cyclohexyloxy-but-1-yl, 2-Cyclohexyloxy-but-1-yl, 3-Cyclohexyloxy-but-1-yl, 9-Cyclohexyloxy-but-1-yl, 1-Cyclohexyloxy-but-2-yl, 2-Cyclohexyloxy-but-2-yl, 3-Cyclohexyloxybut-2-yl, 3-Cyclohexyloxy-but-2-yl, 4-Cyclohexyloxy-but-2-yl, 1-(Cyclohexylommethyl)eth-1-yl, 1-(Cyclohexyloxymethyl)-3-($CH_3$)-eth-1-yl, 1-(Cyclohexyloxymethyl)-prop-1-yl, Cycloheptyloxymethyl, 1-Cycloheptyloxy-ethyl, 2-Cycloheptyloxy-ethyl, 1-Cycloheptyloxy-prop-1-yl, 2-Cycloheptyloxy-prop-1-yl, 3-Cycloheptyloxy-prop-1-yl, 1-Cycloheptyloxy-but-3-yl, 2-Cycloheptyloxy-but-1-yl, 3-Cycloheptyloxy-but-1-yl, 4-Cycloheptyloxy-but-1-yl, 1-Cycloheptyloxy-but-2-yl, 2-Cycloheptyloxy-but-2-yl, 3-Cycloheptyloxy-but-2-yl, 3-Cycloheptyloxy-but-2-yl, 4-Cycloheptyloxy-but-2-yl, 1-(Cycloheptyloxymethyl)eth-1-yl, 2-(Cycloheptyloxyrnethyl)-1-($CH_3$)-eth-1-yl, 1-(Cycloheptyloxymethyl)prop-1-yl, Cyclooctyloxymethyl, 1-Cyclooctyloxy-ethyl, 2-Cyclooctyloxy-ethyl, 1-Cyclooctyloxy-prop-1-yl, 2-Cyclooctyloxy-prop-1-yl, 3-Cyclooctyloxy-prop-1-yl, 1-Cyclooctyloxy-but-1-yl, 2-Cyclooctyloxy-but-1-yl, 3-Cyclooctyloxy-but-1-yl, 4-Cyclooctyloxy-but-1-yl, 1-Cyclooctyloxy-but-2-yl, 2-Cyclooctyloxy-but-2-yl, 3-Cyclooctyloxy-but-2-yl, 3-Cyclooctyloxy-but-2-yl, 4-Cyclooctyloxy-but-2-yl, 1-(Cyclooctyloxymethyl)-eth-1-yl, 1-(Cyclooctyloxymethyl)-1-($CH_3$)-eth-1-yl oder 1-(Cyclooctyloxymethyl)prop-1-yl, insbesondere für $C_3$-$C_6$-Cycloalkoxymethyl oder 2-($C_3$-$C_6$-Cycloalkoxy) ethyl.

Unter 3- bis 7gliedrigem Heterocyclyl sind sowohl gesättigte, partiell oder vollständig ungesättigte als auch aromatische Heterocyclen mit ein bis drei Heteroatomen, ausgewählt aus einer Gruppe bestehend aus

- ein bis drei Stickstoffatomen,

- einem oder zwei Sauerstoff- und
- einem oder zwei Schwefelatomen,

zu verstehen.

Beispiele für gesättigte Heterocyclen, die ein Carbonyl- oder Thiocarbonyl-Ringglied enthalten können, sind: Oxiranyl, Thiiranyl, Aziridin-1-yl, Aziridin-2-yl, Diaziridin-1-yl, Diaziridin-3-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl.

Beispiele für ungesättigte Heterocyclen, die ein Carbonyloder Thiocarbonyl-Ringglied enthalten können, sind: Dihydrofuran-2-yl, 1,2-Oxazolin-3-yl, 1,2-Oxazolin-5-yl, 1,3-Oxazolin-2-yl;

Unter den Heteroaromaten sind die 5- und 6-gliedrigen bevorzugt, also z.B.
Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Alle Phenyl-, carbocyclischen und heterocyclischen Ringe sind vorzugsweise unsubstituiert oder tragen einen Substituenten.

Im Hinblick auf die Verwendung der substituierten Pyrazol-3-ylbenzazole I als Herbizide oder Desikkantien/Defoliantien sind diejenigen Verbindungen I bevorzugt, bei denen die Variablen folgende Bedeutungen haben, und zwar jeweils für sich allein oder in Kombination:

$R^1$ $C_1$-$C_4$-Alkyl, insbesondere Methyl;

$R^2$ $C_1$-$C_4$-Halogenalkyl, $C_1$-$C_4$-Halogenalkoxy oder $C_1$-$C_4$-Alkylsulfonyl, insbesondere Trifluormethyl, Difluormethoxy oder Methylsulfonyl, besonders bevorzugt Difluormethoxy;

$R^3$ Halogen, insbesondere Chlor;

$R^4$ Wasserstoff, Fluor oder Chlor, insbesondere Fluor oder Chlor;

$R^5$ Halogen, besonders bevorzugt Chlor;

A eine über Sauerstoff oder Schwefel an $\alpha$ gebundene Gruppe -N=C($XR^6$)-O- oder -N=C($XR^6$)-S-, insbesondere über den Schwefel an $\alpha$ gebundenes -N=C($XR^6$)-S-;

X eine chemische Bindung, Sauerstoff, Schwefel, -NH- oder -N($R^7$)-;

$R^6$, $R^7$ unabhängig voneinander
$C_1$-$C_6$-Alkyl, Cyano-$C_1$-$C_4$-alkyl, Hydroxy-$C_1$-$C_4$-alkyl, $C_3$-$C_6$-Alkenyl, $C_3$-$C_6$-Alkinyl, $C_1$-$C_4$-Alkoxy-$C_1$-$C_4$-alkyl,

$C_1$-$C_4$-Alkylthio-$C_1$-$C_4$-alkyl, $C_1$-$C_4$-Alkylsulfonyl-$C_1$-$C_4$-alkyl, ($C_1$-$C_4$-Alkoxy)carbonyl-$C_1$-$C_4$-alkyl, Di($C_1$-$C_4$-alkyl)aminocarbonyl-$C_1$-$C_4$-alkyl, $C_3$-$C_8$-Cycloalkyl, $C_3$-$C_8$-Cycloalkyl-$C_1$-$C_4$-alkyl, Phenyl oder Phenyl-$C_1$-$C_4$ - alkyl; sofern X eine chemische Bindung, Sauerstoff, Schwefel, -NH- oder -N($R^7$)- ist, kann $R^6$ auch ($C_1$-$C_4$-Alkyl)carbonyl oder $C_1$-$C_4$-Alkylsulfonyl bedeuten; sofern X eine chemische Bindung ist, kann $R^6$ außerdem Wasserstoff, Cyano, Amino, Halogen oder -CH=CH-$R^8$ bedeuten;

$R^6$, $R^7$ jeweils insbesondere $C_1$-$C_6$-Alkyl, Hydroxy-$C_1$-$C_4$-alkyl, $C_3$-$C_6$-Alkenyl, $C_3$-$C_6$-Alkinyl oder ($C_1$-$C_4$-Alkoxy)carbonyl-$C_1$-$C_4$-alkyl;
sofern X eine chemische Bindung ist, kann $R^6$ außerdem insbesondere Wasserstoff oder -CH=CH-$R^8$ bedeuten;

$R^8$ ($C_1$-$C_4$-Alkoxy) carbonyl.

Ganz besonders bevorzugt sind die substituierten Pyrazol-3-ylbenzazole der Formel Ia ($\hat{=}$ I mit $R^1$ = Methyl, $R^2$ = Difluormethoxy, $R^3$ und $R^5$ = Chlor, $R^4$ = wasserstoff, Z = über den Schwefel an α gebundenes -N=C(X$R^6$) -S-), insbesondere die in der folgenden Tabelle 1 aufgeführten Verbindungen:

Tabelle 1

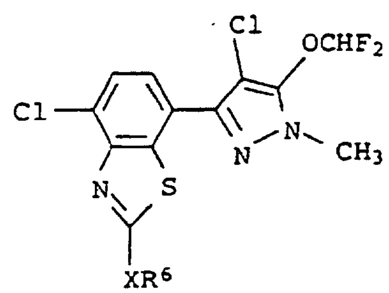

Ia

| Nr. | -XR$^6$ |
|---|---|
| Ia.001 | -H |
| Ia.002 | $-CH_3$ |
| Ia.003 | $-C_2H_5$ |
| Ia.004 | $-(n-C_3H_7)$ |
| Ia.005 | $-CH(CH_3)_2$ |
| Ia.006 | $-(n-C_4H_9)$ |
| Ia.007 | $-CH_2-CH(CH_3)_2$ |
| Ia.008 | $-CH(CH_3)-C_2H_5$ |
| Ia.009 | $-C(CH_3)_3$ |
| Ia.010 | $-CH_2-CH=CH_2$ |
| Ia.011 | $-CH_2-CH=CH-CH_3$ |
| Ia.012 | $-CH_2-CH_2-CH=CH_2$ |

| Nr. | $-XR^6$ |
|---|---|
| Ia.013 | $-CH_2-C\equiv CH$ |
| Ia.014 | $-CH_2-OCH_3$ |
| Ia.015 | $-CH_2-CH_2-OCH_3$ |
| Ia.016 | $-CH_2-CN$ |
| Ia.017 | $-CH_2-CO-OCH_3$ |
| Ia.018 | $-CH_2-CO-OC_2H_5$ |
| Ia.019 | $-CH_2-CO-N(CH_3)_2$ |
| Ia.020 | $-CH_2-CH(=N-OCH_3)$ |
| Ia.021 | $-CH_2-CH(=N-OC_2H_5)$ |
| Ia.022 | $-CH_2-CH[=N-O(n-C_3H_7)]$ |
| Ia.023 | $-CH_2-CH[=N-OCH(CH_3)_2]$ |
| Ia.024 | $-CH_2-CH[=N-O(n-C_4H_9)]$ |
| Ia.025 | $-CH_2-CH(=N-OCH_2-CH=CH_2)$ |
| Ia.026 | -Cyclobutyl |
| Ia.027 | -Cyclopentyl |
| Ia.028 | -Cyclohexyl |
| Ia.029 | -Phenyl |
| Ia.030 | $-CH_2$-Cyclobutyl |
| Ia.031 | $-CH_2$-Cyclopentyl |
| Ia.032 | $-CH_2$-Cyclohexyl |
| Ia.033 | $-CH_2$-Phenyl |
| Ia.034 | $-NO_2$ |
| Ia.035 | -CN |
| Ia.036 | -F |
| Ia.037 | -Cl |
| Ia.038 | -Br |
| Ia.039 | $-OCH_3$ |
| Ia.040 | $-OC_2H_5$ |
| Ia.041 | $-O(n-C_3H_7)$ |
| Ia.042 | $-OCH(CH_3)_2$ |
| Ia.043 | $-O(n-C_4H_9)$ |
| Ia.044 | $-OCH_2-CH(CH_3)_2$ |
| Ia.045 | $-OCH(CH_3)-C_2H_5$ |
| Ia.046 | $-OC(CH_3)_3$ |
| Ia.047 | $-OCH_2-CH=CH_2$ |
| Ia.048 | $-OCH_2-CH=CH-CH_3$ |

| Nr. | $-XR^6$ |
|---|---|
| Ia.049 | $-OCH_2-CH_2-CH=CH_2$ |
| Ia.050 | $-OCH(CH_3)-CH=CH_2$ |
| Ia.051 | $-OCH_2-C{\equiv}CH$ |
| Ia.052 | $-OCH(CH_3)-C{\equiv}CH$ |
| Ia.053 | $-OCH_2-OCH_3$ |
| Ia.054 | $-OCH_2-CH_2-OCH_3$ |
| Ia.055 | $-OCH_2-CN$ |
| Ia.056 | $-OCH_2-CO-OCH_3$ |
| Ia.057 | $-OCH_2-CO-OC_2H_5$ |
| Ia.058 | $-OCH_2-CO-N(CH_3)_2$ |
| Ia.059 | $-OCH_2-CH(=N-OCH_3)$ |
| Ia.060 | $-OCH_2-CH(=N-OC_2H_5)$ |
| Ia.061 | $-OCH_2-CH[=N-O(n-C_3H_7)]$ |
| Ia.062 | $-OCH_2-CH[=N-OCH(CH_3)_2]$ |
| Ia.063 | $-OCH_2-CH[=N-O(n-C_4H_9)]$ |
| Ia.064 | $-OCH_2-CH(=N-OCH_2-CH=CH_2)$ |
| Ia.065 | -O-Cyclobutyl |
| Ia.066 | -O-Cyclopentyl |
| Ia.067 | -O-Cyclohexyl |
| Ia.068 | -O-Phenyl |
| Ia.069 | $-OCH_2$-Cyclobutyl |
| Ia.070 | $-OCH_2$-Cyclopentyl |
| Ia.071 | $-OCH_2$-Cyclohexyl |
| Ia.072 | $-OCH_2$-Phenyl |
| Ia.073 | $-CH_2-OH$ |
| Ia.074 | $-CH_2-OCH_3$ |
| Ia.075 | $-NH_2$ |
| Ia.076 | $-NH-CH_3$ |
| Ia.077 | $-N(CH_3)_2$ |
| Ia.078 | $-NH-C_2H_5$ |
| Ia.079 | $-N(C_2H_5)_2$ |
| Ia.080 | $-NH-(n-C_3H_7)$ |
| Ia.081 | $-N(n-C_3H_7)_2$ |
| Ia.082 | $-NH-(n-C_4H_9)$ |
| Ia.083 | $-N(n-C_4H_9)_2$ |
| Ia.084 | $-NH-CH(CH_3)_2$ |
| Ia.085 | $-N[CH(CH_3)_2]_2$ |

| Nr. | $-XR^6$ |
|---|---|
| Ia.086 | $-NH-CH_2-CH(CH_3)_2$ |
| Ia.087 | $-N[CH_2-CH(CH_3)_2]_2$ |
| Ia.088 | $-NH-CH_2-CH=CH_2$ |
| Ia.089 | $-N(CH_2-CH=CH_2)_2$ |
| Ia.090 | $-NH-CH_2-C{\equiv}CH$ |
| Ia.091 | $-N(CH_2-C{\equiv}CH)_2$ |
| Ia.092 | $-CH_2-N(CH_3)_2$ |
| Ia.093 | -SH |
| Ia.094 | $-SCH_3$ |
| Ia.095 | $-SC_2H_5$ |
| Ia.096 | $-S(n-C_3H_7)$ |
| Ia.097 | $-S(n-C_4H_9)$ |
| Ia.098 | $-SCH(CH_3)_2$ |
| Ia.099 | $-SCH_2-CH(CH_3)_2$ |
| Ia.100 | $-SCH(CH_3)-C_2H_5$ |
| Ia.101 | $-SC(CH_3)_3$ |
| Ia.102 | $-SCH_2-CH=CH_2$ |
| Ia.103 | $-SCH_2-CH=CH-CH_3$ |
| Ia.104 | $-SCH_2-CH_2-CH=CH_2$ |
| Ia.105 | $-SCH(CH_3)-CH=CH_2$ |
| Ia.106 | $-SCH_2-C{\equiv}CH$ |
| Ia.107 | $-SCH(CH_3)-C{\equiv}CH$ |
| Ia.108 | $-SCH_2-OCH_3$ |
| Ia.109 | $-SCH_2-CH_2-OCH_3$ |
| Ia.110 | $-SCH_2-CN$ |
| Ia.111 | $-SCH_2-CO-OCH_3$ |
| Ia.112 | $-SCH_2-CO-OC_2H_5$ |
| Ia.113 | $-SCH_2-CO-N(CH_3)_2$ |
| Ia.114 | $-SCH_2-CH(=N-OCH_3)$ |
| Ia.115 | $-SCH_2-CH(=N-OC_2H_5)$ |
| Ia.116 | $-SCH_2-CH[=N-O(n-C_3H_7)]$ |
| Ia.117 | $-SCH_2-CH[=N-OCH(CH_3)_2]$ |
| Ia.118 | $-SCH_2-CH[=N-O(n-C_4H_9)]$ |
| Ia.119 | $-SCH_2-CH(=N-OCH_2-CH=CH_2)$ |
| Ia.120 | -S-Cyclobutyl |
| Ia.121 | -S-Cyclopentyl |

| Nr. | $-XR^6$ |
|---|---|
| Ia. 122 | -S-Cyclohexyl |
| Ia. 123 | -S-Phenyl |
| Ia. 124 | $-SCH_2$-Cyclobutyl |
| Ia. 125 | $-SCH_2$-Cyclopentyl |
| Ia. 126 | $-SCH_2$-Cyclohexyl |
| Ia. 127 | $-SCH_2$-Phenyl |
| Ia. 128 | $-CH_2-SCH_3$ |
| Ia. 129 | $-SO-CH_3$ |
| Ia. 130 | $-SO-C_2H_5$ |
| Ia. 131 | $-SO-(n-C_3H_7)$ |
| Ia. 132 | $-SO-(n-C_4H_9)$ |
| Ia. 133 | $-SO-CH(CH_3)_2$ |
| Ia. 134 | $-SO-CH_2-CH(CH_3)_2$ |
| Ia. 135 | $-SO-CH(CH_3)-C_2H_5$ |
| Ia. 136 | $-SO-C(CH_3)_3$ |
| Ia. 137 | $-SO-CH_2-CH=CH_2$ |
| Ia. 138 | $-SO-CH_2-CH=CH-CH_3$ |
| Ia. 139 | $-SO-CH_2-CH_2-CH=CH_2$ |
| Ia. 140 | $-SO-CH(CH_3)-CH=CH_2$ |
| Ia. 141 | $-SO-CH_2-C\equiv CH$ |
| Ia. 142 | $-SO-CH(CH_3)-C\equiv CH$ |
| Ia. 143 | $-SO-CH_2-OCH_3$ |
| Ia. 144 | $-SO-CH_2-CH_2-OCH_3$ |
| Ia. 145 | $-SO-CH_2-CN$ |
| Ia. 146 | $-SO-CH_2-CO-OCH_3$ |
| Ia. 147 | $-SO-CH_2-CO-OC_2H_5$ |
| Ia. 148 | $-SO-CH_2-CO-N(CH_3)_2$ |
| Ia. 149 | $-SO-CH_2-CH(=N-OCH_3)$ |
| Ia. 150 | $-SO-CH_2-CH(=N-OC_2H_5)$ |
| Ia. 151 | $-SO-CH_2-CH[=N-O(n-C_3H_7)]$ |
| Ia. 152 | $-SO-CH_2-CH[=N-OCH(CH_3)_2]$ |
| Ia. 153 | $-SO-CH_2-CH[=N-O(n-C_4H_9)]$ |
| Ia. 154 | $-SO-CH_2-CH(=N-OCH_2-CH=CH_2)$ |
| Ia. 155 | -SO-Cyclobutyl |
| Ia. 156 | -SO-Cyclopentyl |
| Ia. 157 | -SO-Cyclohexyl |

| Nr. | $-XR^6$ |
|---|---|
| Ia. 158 | -SO-Phenyl |
| Ia. 159 | $-SO-CH_2-$Cyclobutyl |
| Ia. 160 | $-SO-CH_2-$Cyclopentyl |
| Ia. 161 | $-SO-CH_2-$Cyclohexyl |
| Ia. 162 | $-SO-CH_2-$Phenyl |
| Ia. 163 | $-CH_2-SO-CH_3$ |
| Ia. 164 | $-SO_2-CH_3$ |
| Ia. 165 | $-SO_2-C_2H_5$ |
| Ia. 166 | $-SO_2-(n-C_3H_7)$ |
| Ia. 167 | $-SO_2-(n-C_4H_9)$ |
| Ia. 168 | $-SO_2-CH(CH_3)_2$ |
| Ia. 169 | $-SO_2-CH_2-CH(CH_3)_2$ |
| Ia. 170 | $-SO_2-CH(CH_3)-C_2H_5$ |
| Ia. 171 | $-SO_2-C(CH_3)_3$ |
| Ia. 172 | $-SO_2-CH_2-CH=CH_2$ |
| Ia. 173 | $-SO_2-CH_2-CH=CH-CH_3$ |
| Ia. 174 | $-SO_2-CH_2-CH_2-CH=CH_2$ |
| Ia. 175 | $-SO_2-CH(CH_3)-CH=CH_2$ |
| Ia. 176 | $-SO_2-CH_2-C\equiv CH$ |
| Ia. 177 | $-SO_2-CH(CH_3)-C\equiv CH$ |
| Ia. 178 | $-SO_2-CH_2-OCH_3$ |
| Ia. 179 | $-SO_2-CH_2-CH_2-OCH_3$ |
| Ia. 180 | $-SO_2-CH_2-CN$ |
| Ia. 181 | $-SO_2-CH_2-CO-OCH_3$ |
| Ia. 182 | $-SO_2-CH_2-CO-OC_2H_5$ |
| Ia. 183 | $-SO_2-CH_2-CO-N(CH_3)_2$ |
| Ia. 184 | $-SO_2-CH_2-CH(=N-OCH_3)$ |
| Ia. 185 | $-SO_2-CH_2-CH(=N-OC_2H_5)$ |
| Ia. 186 | $-SO_2-CH_2-CH[=N-O(n-C_3H_7)]$ |
| Ia. 187 | $-SO_2-CH_2-CH[=N-OCH(CH_3)_2]$ |
| Ia. 188 | $-SO_2-CH_2-CH[=N-O(n-C_4H_9)]$ |
| Ia. 189 | $-SO_2-CH_2-CH(=N-OCH_2-CH=CH_2)$ |
| Ia. 190 | $-SO_2-$Cyclobutyl |
| Ia. 191 | $-SO_2-$Cyclopentyl |
| Ia. 192 | $-SO_2-$Cyclohexyl |
| Ia. 193 | $-SO_2-$Phenyl |

| Nr. | $-XR^6$ |
|---|---|
| Ia.194 | $-SO_2-CH_2-$Cyclobutyl |
| Ia.195 | $-SO_2-CH_2-$Cyclopentyl |
| Ia.196 | $-SO_2-CH_2-$Cyclohexyl |
| Ia.197 | $-SO_2-CH_2-$Phenyl |
| Ia.198 | $-CH_2-SO_2-CH_3$ |
| Ia.199 | $-CH_2-CH(Cl)-CO-OH$ |
| Ia.200 | $-CH_2-CH(Cl)-CO-OCH_3$ |
| Ia.201 | $-CH_2-CH(Cl)-CO-OC_2H_5$ |
| Ia.202 | $-CH_2-CH(Cl)-CO-O(n-C_3H_7)$ |
| Ia.203 | $-CH_2-CH(Cl)-CO-O(n-C_4H_9)$ |
| Ia.204 | $-CH_2-CH(Cl)-CO-OCH(CH_3)_2$ |
| Ia.205 | $-CH_2-CH(Cl)-CO-OCH_2-CH(CH_3)_2$ |
| Ia.206 | $-CH_2-CH(Cl)-CO-OCH(CH_3)-C_2H_5$ |
| Ia.207 | $-CH_2-CH(Cl)-CO-OC(CH_3)_3$ |
| Ia.208 | $-CH_2-CH(Br)-CO-OH$ |
| Ia.209 | $-CH_2-CH(Br)-CO-OCH_3$ |
| Ia.210 | $-CH_2-CH(Br)-CO-OC_2H_5$ |
| Ia.211 | $-CH_2-CH(Br)-CO-O(n-C_3H_7)$ |
| Ia.212 | $-CH_2-CH(Br)-CO-O(n-C_4H_9)$ |
| Ia.213 | $-CH_2-CH(Br)-CO-OCH(CH_3)_2$ |
| Ia.214 | $-CH_2-CH(Br)-CO-OCH_2-CH(CH_3)_2$ |
| Ia.215 | $-CH_2-CH(Br)-CO-OCH(CH_3)-C_2H_5$ |
| Ia.216 | $-CH_2-CH(Br)-CO-OC(CH_3)_3$ |
| Ia.217 | $-CH=CH-CO-OH$ |
| Ia.218 | $-CH=CH-CO-OCH_3$ |
| Ia.219 | $-CH=CH-CO-OC_2H_5$ |
| Ia.220 | $-CH=CH-CO-O(n-C_3H_7)$ |
| Ia.221 | $-CH=CH-CO-O(n-C_4H_9)$ |
| Ia.222 | $-CH=CH-CO-OCH(CH_3)_2$ |
| Ia.223 | $-CH=CH-CO-OCH_2-CH(CH_3)_2$ |
| Ia.224 | $-CH=CH-CO-OCH(CH_3)-C_2H_5$ |
| Ia.225 | $-CH=CH-CO-OC(CH_3)_3$ |
| Ia.226 | $-CH=C(Cl)-CO-OH$ |
| Ia.227 | $-CH=C(Cl)-CO-OCH_3$ |
| Ia.228 | $-CH=C(Cl)-CO-OC_2H_5$ |
| Ia.229 | $-CH=C(Cl)-CO-O(n-C_3H_7)$ |
| Ia.230 | $-CH=C(Cl)-CO-O(n-C_4H_9)$ |
| Ia.231 | $-CH=C(Cl)-CO-OCH(CH_3)_2$ |
| Ia.232 | $-CH=C(Cl)-CO-OCH_2-CH(CH_3)_2$ |

| Nr. | $-XR^6$ |
|---|---|
| Ia.233 | $-CH=C(Cl)-CO-OCH(CH_3)-C_2H_5$ |
| Ia.234 | $-CH=C(Cl)-CO-OC(CH_3)_3$ |
| Ia.235 | $-CH=C(Br)-CO-OH$ |
| Ia.236 | $-CH=C(Br)-CO-OCH_3$ |
| Ia.237 | $-CH=C(Br)-CO-OC_2H_5$ |
| Ia.238 | $-CH=C(Br)-CO-O(n-C_3H_7)$ |
| Ia.239 | $-CH=C(Br)-CO-O(n-C_4H_9)$ |
| Ia.240 | $-CH=C(Br)-CO-OCH(CH_3)_2$ |
| Ia.241 | $-CH=C(Br)-CO-OCH_2-CH(CH_3)_2$ |
| Ia.242 | $-CH=C(Br)-CO-OCH(CH_3)-C_2H_5$ |
| Ia.243 | $-CH=C(Br)-CO-OC(CH_3)_3$ |
| Ia.244 | $-CH_2-CH(Cl)-CO-NH_2$ |
| Ia.245 | $-CH_2-CH(Cl)-CO-NH-CH_3$ |
| Ia.246 | $-CH_2-CH(Cl)-CO-N(CH_3)_2$ |
| Ia.247 | $-CH_2-CH(Cl)-CO-NH-C_2H_5$ |
| Ia.248 | $-CH_2-CH(Cl)-CO-N(C_2H_5)_2$ |
| Ia.249 | $-CH_2-CH(Cl)-CO-NH-(n-C_3H_7)$ |
| Ia.250 | $-CH_2-CH(Cl)-CO-N(n-C_3H_7)_2$ |
| Ia.251 | $-CH_2-CH(Cl)-CO-NH-(n-C_4H_9)$ |
| Ia.252 | $-CH_2-CH(Cl)-CO-N(n-C_4H_9)_2$ |
| Ia.253 | $-CH_2-CH(Br)-CO-NH_2$ |
| Ia.254 | $-CH_2-CH(Br)-CO-NH-CH_3$ |
| Ia.255 | $-CH_2-CH(Br)-CO-N(CH_3)_2$ |
| Ia.256 | $-CH_2-CH(Br)-CO-NH-C_2H_5$ |
| Ia.257 | $-CH_2-CH(Br)-CO-N(C_2H_5)_2$ |
| Ia.258 | $-CH_2-CH(Br)-CO-NH-(n-C_3H_7)$ |
| Ia.259 | $-CH_2-CH(Br)-CO-N(n-C_3H_7)_2$ |
| Ia.260 | $-CH_2-CH(Br)-CO-NH-(n-C_4H_9)$ |
| Ia.261 | $-CH_2-CH(Br)-CO-N(n-C_4H_9)_2$ |
| Ia.262 | $-CH=CH-CO-NH_2$ |
| Ia.263 | $-CH=CH-CO-NH-CH_3$ |
| Ia.264 | $-CH=CH-CO-N(CH_3)_2$ |
| Ia.265 | $-CH=CH-CO-NH-C_2H_5$ |
| Ia.266 | $-CH=CH-CO-N(C_2H_5)_2$ |
| Ia.267 | $-CH=CH-CO-NH-(n-C_3H_7)$ |
| Ia.268 | $-CH=CH-CO-N(n-C_3H_7)_2$ |
| Ia.269 | $-CH=CH-CO-NH-(n-C_4H_9)$ |
| Ia.270 | $-CH=CH-CO-N(n-C_4H_9)_2$ |
| Ia.271 | $-CH=C(Cl)-CO-NH_2$ |

| Nr. | $-XR^6$ |
|---|---|
| Ia. 272 | $-CH=C(Cl)-CO-NH-CH_3$ |
| Ia. 273 | $-CH=C(Cl)-CO-N(CH_3)_2$ |
| Ia. 274 | $-CH=C(Cl)-CO-NH-C_2H_5$ |
| Ia. 275 | $-CH=C(Cl)-CO-N(C_2H_5)_2$ |
| Ia. 276 | $-CH=C(Cl)-CO-NH-(n-C_3H_7)$ |
| Ia. 277 | $-CH=C(Cl)-CO-N(n-C_3H_7)_2$ |
| Ia. 278 | $-CH=C(Cl)-CO-NH-(n-C_4H_9)$ |
| Ia. 279 | $-CH=C(Cl)-CO-N(n-C_4H_9)_2$ |
| Ia. 280 | $-CH=C(Br)-CO-NH_2$ |
| Ia. 281 | $-CH=C(Br)-CO-NH-CH_3$ |
| Ia. 282 | $-CH=C(Br)-CO-NH(CH_3)_2$ |
| Ia. 283 | $-CH=C(Br)-CO-NH-C_2H_5$ |
| Ia. 284 | $-CH=C(Br)-CO-N(C_2H_5)_2$ |
| Ia. 285 | $-CH=C(Br)-CO-NH-(n-C_3H_7)$ |
| Ia. 286 | $-CH=C(Br)-CO-N(n-C_3H_7)_2$ |
| Ia. 287 | $-CH=C(Br)-CO-NH-(n-C_4H_9)$ |
| Ia. 288 | $-CH=C(Br)-CO-N(n-C_4H_9)_2$ |
| Ia. 289 | $-CH(CH_3)-OCH_3$ |
| Ia. 290 | $-CH_2OH$ |
| Ia. 291 | $-CH(CH_3)OH$ |
| Ia. 292 | $-CH_2-CH_2OH$ |
| Ia. 293 | -O-Phenyl |
| Ia. 294 | $-OCH_2$-Phenyl |
| Ia. 295 | $-OCH_2-CO-O(n-C_3H_7)$ |
| Ia. 296 | $-OCH_2-CO-OCH(CH_3)_2$ |
| Ia. 297 | $-OCH_2-CO-O(n-C_4H_9)$ |
| Ia. 298 | $-OCH_2-CO-OCH_2-CH(CH_3)_2$ |
| Ia. 299 | $-OCH_2-CO-OCH(CH_3)-C_2H_5$ |
| Ia. 300 | $-OCH_2-CO-OC(CH_3)_3$ |
| Ia. 301 | $-O-CO-CH_3$ |
| Ia. 302 | $-O-CO-C_2H_5$ |
| Ia. 303 | $-O-CO-(n-C_3H_7)$ |
| Ia. 304 | $-O-CO-(n-C_4H_9)$ |
| Ia. 305 | $-OCH(CH_3)-CO-OCH_3$ |
| Ia. 306 | $-OCH(CH_3)-CO-OC_2H_5$ |
| Ia. 307 | $-OCH(CH_3)-CO-O(n-C_3H_7)$ |
| Ia. 308 | $-OCH(CH_3)-CO-OCH(CH_3)_2$ |

| Nr. | $-XR^6$ |
|---|---|
| Ia.309 | $-OCH(CH_3)-CO-O(n-C_4H_9)$ |
| Ia.310 | $-OCH(CH_3)-CO-OCH_2-CH(CH_3)_2$ |
| Ia.311 | $-OCH(CH_3)-CO-OCH(CH_3)-C_2H_5$ |
| Ia.312 | $-OCH(CH_3)-CO-OC(CH_3)_3$ |
| Ia.313 | $-NH-CH_2-CH_2-CN$ |
| Ia.314 | $-N(CH_2-CH_2-CN)_2$ |
| Ia.315 | $-NH-CH_2-CO-OCH_3$ |
| Ia.316 | $-N(CH_2-CO-OCH_3)_2$ |
| Ia.317 | $-NH-CH_2-CO-OC_2H_5$ |
| Ia.318 | $-N(CH_2-CO-OC_2H_5)_2$ |
| Ia.319 | $-N(CH_3)-C_2H_5$ |
| Ia.320 | $-N(CH_3)-(n-C_3H_7)$ |
| Ia.321 | $-N(CH_3)-(n-C_4H_9)$ |
| Ia.322 | $-N(CH_3)-CH(CH_3)_2$ |
| Ia.323 | $-N(CH_3)-CH_2-CH(CH_3)_2$ |
| Ia.324 | $-N(CH_3)-CH_2-CH=CH_2$ |
| Ia.325 | $-N(CH_3)-CH_2-C\equiv CH$ |
| Ia.326 | $-N(CH_3)-CH_2-CH_2-CN$ |
| Ia.327 | $-N(CH_3)-CH_2-CO-OCH_3$ |
| Ia.328 | $-N(CH_3)-CH_2-CO-OC_2H_5$ |
| Ia.329 | $-NH-CO-CH_3$ |
| Ia.330 | $-NH-CO-C_2H_5$ |
| Ia.331 | $-NH-CO-(n-C_3H_7)$ |
| Ia.332 | $-NH-CO-(n-C_4H_9)$ |
| Ia.333 | $-NH-SO_2-CH_3$ |
| Ia.334 | $-NH-SO_2-C_2H_5$ |
| Ia.335 | $-NH-SO_2-(n-C_3H_7)$ |
| Ia.336 | $-NH-SO_2-(n-C_4H_9)$ |
| Ia.337 | -S-Phenyl |
| Ia.338 | $-SCH_2$-Phenyl |
| Ia.339 | $-SCH_2-CO-O(n-C_3H_7)$ |
| Ia.340 | $-SCH_2-CO-OCH(CH_3)_2$ |
| Ia.341 | $-SCH_2-CO-O(n-C_4H_9)$ |
| Ia.342 | $-SCH_2-CO-OCH_2-CH(CH_3)_2$ |
| Ia.343 | $-SCH_2-CO-OCH(CH_3)-C_2H_5$ |
| Ia.344 | $-SCH_2-CO-OC(CH_3)_3$ |
| Ia.345 | $-S-CO-CH_3$ |
| Ia.346 | $-SCH(CH_3)-CO-OCH_3$ |
| Ia.347 | $-SCH(CH_3)-CO-OC_2H_5$ |

| Nr. | $-XR^6$ |
|---|---|
| Ia.348 | $-SCH(CH_3)-CO-O(n-C_3H_7)$ |
| Ia.349 | $-SCH(CH_3)-CO-O(n-C_4H_9)$ |
| Ia.350 | $-CH_2-PO(OCH_3)_2$ |
| Ia.351 | $-CH_2-PO(OC_2H_5)_2$ |
| Ia.352 | $-OCH_2-PO(OCH_3)_2$ |
| Ia.353 | $-OCH_2-PO(OC_2H_5)_2$ |
| Ia.354 | $-SCH_2-PO(OCH_3)_2$ |
| Ia.355 | $-SCH_2-PO(OC_2H_5)_2$ |
| Ia.356 | $-CH_2-CH(Cl)-PO(OCH_3)_2$ |
| Ia.357 | $-CH_2-CH(Cl)-PO(OC_2H_5)_2$ |
| Ia.358 | $-CH_2-CH(Br)-PO(OCH_3)_2$ |
| Ia.359 | $-CH_2-CH(Br)-PO(OC_2H_5)_2$ |
| Ia.360 | $-CH=CH-PO(OCH_3)_2$ |
| Ia.361 | $-CH=CH-PO(OC_2H_5)_2$ |
| Ia.362 | $-CH(CO-OCH_3)_2$ |
| Ia.363 | $-CH(CO-OC_2H_5)_2$ |
| Ia.364 | $-CH(CO-OCH_3)[CO-OC(CH_3)_3]$ |
| Ia.365 | $-CH(CO-OC_2H_5)[CO-OC(CH_3)_3]$ |
| Ia.366 | $-CH(CN)-CO-OCH_3$ |
| Ia.367 | $-CH(CN)-CO-OC_2H_5$ |
| Ia.368 | $-CH(CN)-CO-OC(CH_3)_3$ |

Des weiteren sind die substituierten Pyrazol-3-ylbenzazole der Formeln Ib bis It und IA bis IT besonders bevorzugt, insbesondere

- die Verbindungen Ib.001 bis Ib.368, die sich von den entsprechenden Verbindungen Ia.001 bis Ia.368 lediglich dadurch unterscheiden, daß $R^4$ für Chlor steht:

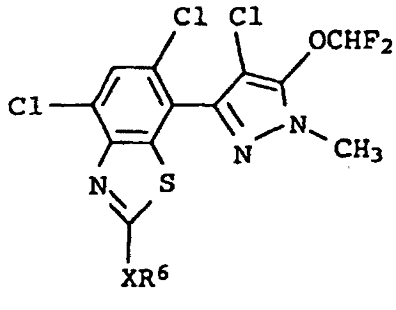

Ib

- die Verbindungen Ic.001 bis Ic.368, die sich von den entsprechenden Verbindungen Ia.001 bis Ia.368 lediglich dadurch unterscheiden, daß $R^4$ für Fluor steht:

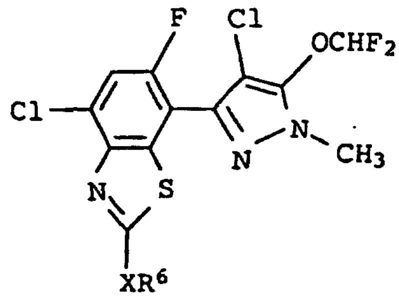

Ic

- die Verbindungen Id.001 bis Id.368, die sich von den entsprechenden Verbindungen Ia.001 bis Ia.368 lediglich dadurch unterscheiden, daß $R^2$ für Trifluormethyl steht:

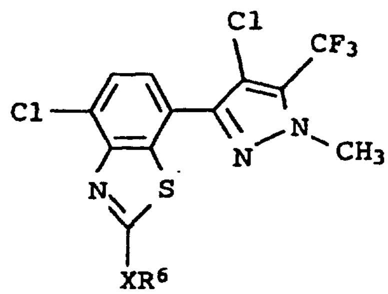

Id

- die Verbindungen Ie.001 bis Ie. 368, die sich von den entsprechenden Verbindungen Ia.001 bis Ia.368 lediglich dadurch unterscheiden, daß $R^2$ für Trifluormethyl und $R^4$ für Chlor stehen:

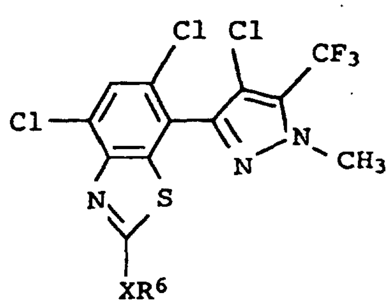

Ie

- die Verbindungen If.001 bis If.368, die sich von den entsprechenden Verbindungen Ia.001 bis Ia.368 lediglich dadurch unterscheiden, daß $R^2$ für Trifluormethyl und $R^4$ für Fluor stehen:

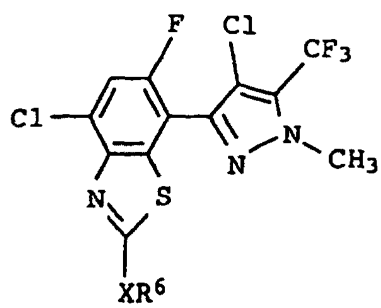

If

- die verbindungen Ig.001 bis Ig.368, die sich von den entsprechenden Verbindungen Ia.001 bis Ia.368 lediglich dadurch unterscheiden, daß $R^2$ für Methylsulfonyl steht:

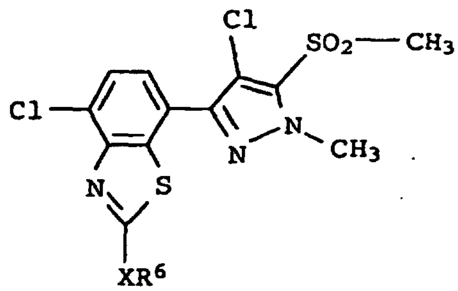

Ig

- die Verbindungen Ih.001 bis Ih.368, die sich von den entsprechenden Verbindungen Ia.001 bis Ia.368 lediglich dadurch. unterscheiden, daß $R^2$ für Methylsulfonyl und $R^4$ für Chlor stehen:

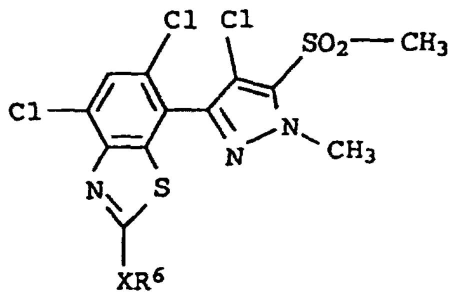

Ih

- die Verbindungen Ij. 001 bis Ij.368, die sich von den entsprechenden Verbindungen Ia.001 bis Ia.368 lediglich dadurch unterscheiden, daß $R^2$ für Methylsulfonyl und $R^4$ für Fluor stehen:

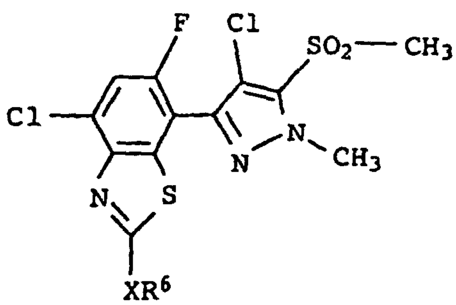

Ij

- die Verbindungen Ik.001 bis Ik.368, die sich von den entsprechenden Verbindungen Ia.001 bis Ia.368 lediglich dadurch unterscheiden, daB Z eine Gruppe -N=C($XR^6$)-O- bedeutet, die über den Sauerstoff an $\alpha$ gebunden ist:

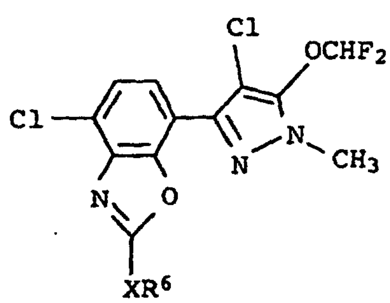

Ik

- die Verbindungen Im. 001 bis Im.368, die sich von den entsprechenden Verbindungen Ia.001 bis Ia.368 dadurch unterscheiden, daß $R^4$ für Chlor steht und Z eine Gruppe -N=C ($XR^6$) -Obedeutet, die über den Sauerstoff an $\alpha$ gebunden ist:

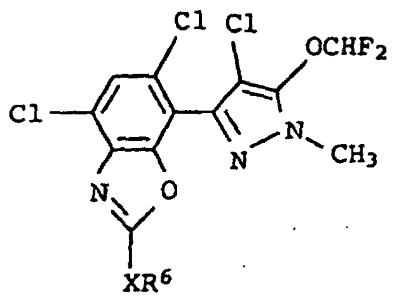

Im

- die Verbindungen In.001 bis In.368, die sich von den entsprechenden Verbindungen Ia.001 bis Ia.368 dadurch unterscheiden, daß $R^4$ für Fluor steht und Z eine Gruppe -N=C($XR^6$)-Obedeutet, die über den Sauerstoff an $\alpha$ gebunden ist:

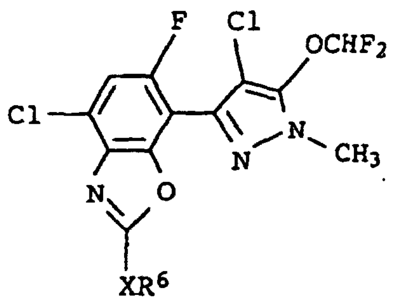

In

- die Verbindungen Io.001 bis Io.368, die sich von den entsprechenden Verbindungen Ia.001 bis Ia.368 dadurch unterscheiden, daß $R^2$ für Trifluormethyl steht und Z eine Gruppe -N=C($XR^6$)-O- bedeutet, die über den Sauerstoff an $\alpha$ gebunden ist:

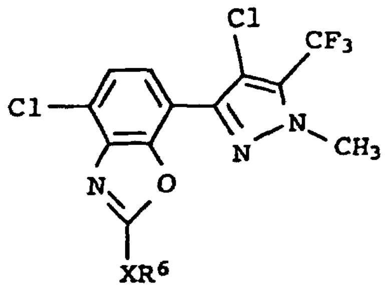

Io

- die Verbindungen Ip.001 bis Ip.368, die sich von den entsprechenden Verbindungen Ia.001 bis Ia.368 dadurch unterscheiden, daß $R^2$ für Trifluormethyl und $R^4$ für Chlor stehen und Z eine Gruppe -N=C($XR^6$)-O- bedeutet, die über den Sauerstoff an $\alpha$ gebunden ist:

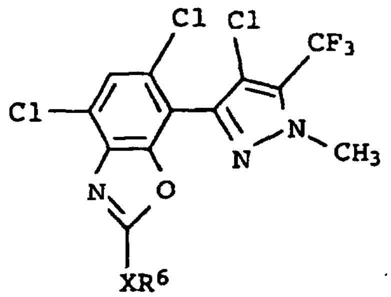

Ip

- die Verbindungen Iq.001 bis Iq.368, die sich von den entsprechenden Verbindungen Ia.001 bis Ia.368 dadurch unterscheiden, daß $R^2$ für Trifluormethyl und $R^4$ für Fluor stehen und Z eine Gruppe -N=C($XR^6$)-O- bedeutet, die über den Sauerstoff an $\alpha$ gebunden ist:

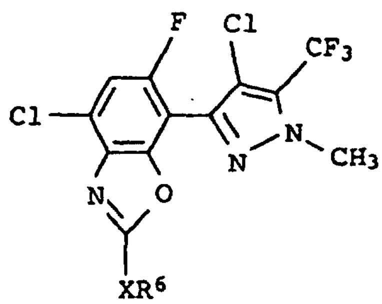

Iq

- die Verbindungen Ir.001 bis Ir.368, die sich von den entsprechenden Verbindungen Ia.001 bis Ia.368 dadurch unterscheiden, daß $R^2$ für Methylsulfonyl steht und Z eine Gruppe -N=C($XR^6$)-O- bedeutet, die über den Sauerstoff an $\alpha$ gebunden ist:

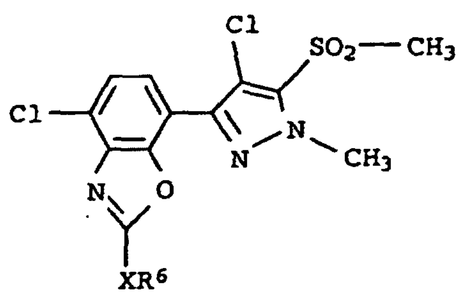

Ir

- die Verbindungen Is.001 bis Is.368, die sich von den entsprechenden Verbindungen Ia.001 bis Ia.368 dadurch unterscheiden, daß $R^2$ für Methylsulfonyl und $R^4$ für Chlor stehen und Z eine Gruppe -N=C($XR^6$)-O- bedeutet, die über den Sauerstoff an $\alpha$ gebunden ist:

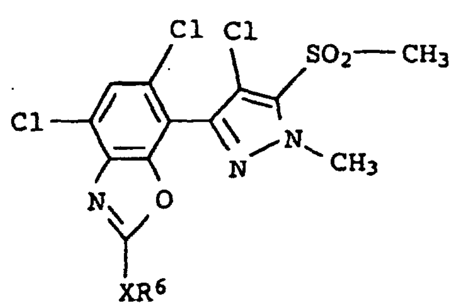

Is

- die Verbindungen It.001 bis It.368, die sich von den entsprechenden Verbindungen Ia.001 bis Ia.368 dadurch unterscheiden, daß $R^2$ für Methylsulfonyl und $R^4$ für Fluor stehen und Z eine Gruppe -N=C($XR^6$)-O- bedeutet, die über den Sauerstoff an α gebunden ist:

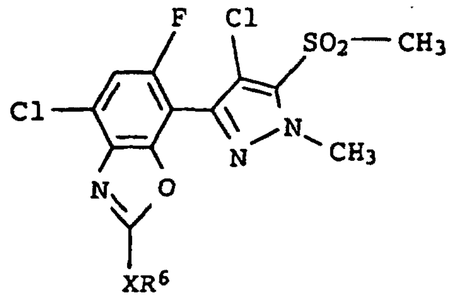

It

- die Verbindungen IA.001 bis IA.368, die sich von den entsprechenden Verbindungen Ia.001 bis Ia.368 lediglich dadurch unterscheiden, daß die Gruppe -N=C($XR^6$)-S- über den Stickstoff an α gebunden ist:

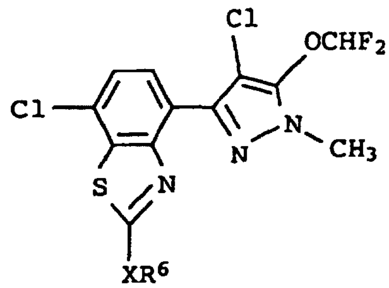

IA

- die Verbindungen IB.001 bis IH.368, die sich von den entsprechenden Verbindungen Ia.001 bis Ia.368 lediglich dadurch unterscheiden, daß $R^4$ für Chlor steht und die Gruppe -N=C($xR^6$)-S- über den Stickstoff an α gebunden ist:

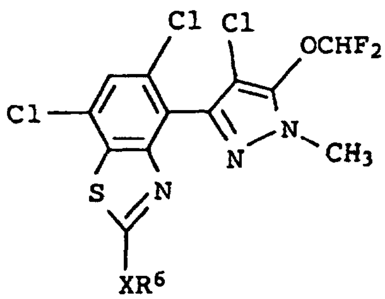

IB

- die Verbindungen IC.001 bis IC.368, die sich von den entsprechenden Verbindungen Ia.001 bis Ia.368 lediglich dadurch unterscheiden, daß $R^4$ für Fluor steht und die Gruppe -N=C($XR^6$)-S- über den Stickstoff an α gebunden ist:

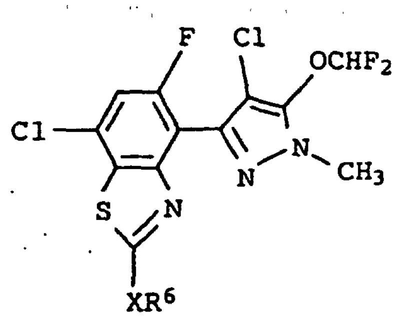

IC

- die Verbindungen ID.001 bis ID.368, die sich von den entsprechenden Verbindungen Ia.001 bis Ia.368 lediglich dadurch unterscheiden, daß $R^2$ für Trifluormethyl steht und die Gruppe -N=C(X$R^6$)-S- über den Stickstoff an $\alpha$ gebunden ist:

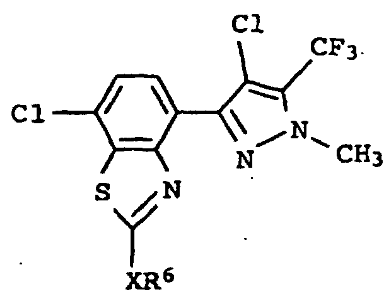

ID

- die Verbindungen IE.001 bis IE.368, die sich von den entsprechenden Verbindungen Ia.001 bis Ia.368 lediglich dadurch unterscheiden, daß $R^2$ für Trifluormethyl und $R^4$ für Chlor stehen und die Gruppe -N=C(X$R^6$)-S- über den Stickstoff an $\alpha$ gebunden ist:

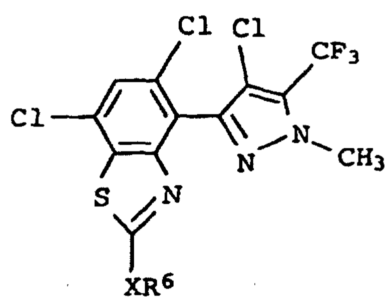

IE

- die Verbindungen IF.001 bis IF.368, die sich von den entsprechenden Verbindungen Ia.001 bis Ia.368 lediglich dadurch unterscheiden, daß $R^2$ für Trifluormethyl und $R^4$ für Fluor stehen und die Gruppe -N=C(X$R^6$)-S- über den Stickstoff an $\alpha$ gebunden ist:

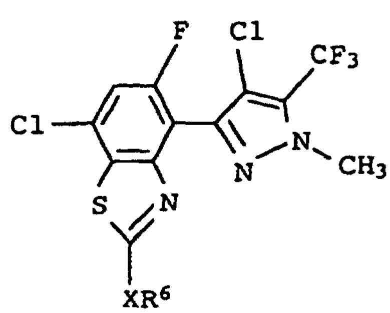

IF

- die Verbindungen IG.001 bis IG.368, die sich von den entsprechenden Verbindungen Ia.001 bis Ia.368 lediglich dadurch unterscheiden, daß $R^2$ für Methylsulfonyl steht und die Gruppe -N=C($XR^6$)-S- über den Stickstoff an α gebunden ist:

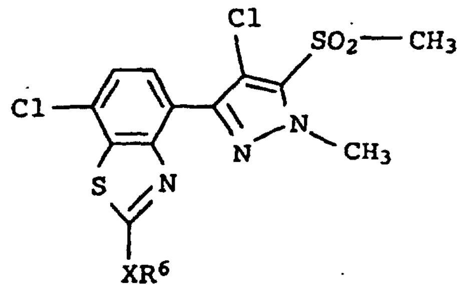

IG

- die Verbindungen IH.001 bis IH.368, die sich von den entsprechenden Verbindungen Ia.001 bis Ia.368 lediglich dadurch unterscheiden, daß $R^2$ für Methylsulfonyl und $R^4$ für Chlor stehen und die Gruppe -N=C($XR^6$)-S- über den Stickstoff an α gebunden ist:

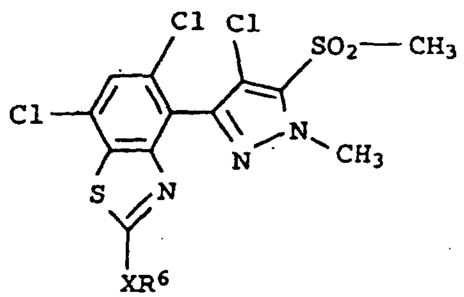

IH

- die Verbindungen IJ.001 bis IJ.368, die sich von den entsprechenden Verbindungen Ia.001 bis Ia.368 lediglich dadurch unterscheiden, daß $R^2$ für Methylsulfonyl und $R^4$ für Fluor stehen und die Gruppe -N=C($XR^6$)-S- über den Stickstoff an α gebunden ist:

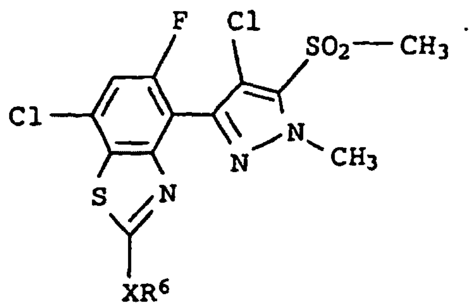

IJ

- die Verbindungen IK.001 bis IK.368, die sich von den entsprechenden Verbindungen Ia.001 bis Ia.368 lediglich dadurch unterscheiden, daß Z eine Gruppe -N=C($XR^6$)-O- bedeutet, die über den Stickstoff an α gebunden ist:

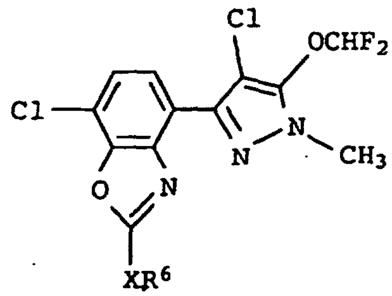

IK

- die Verbindungen IM.001 bis IM. 368, die sich von den entsprechenden Verbindungen Ia.001 bis Ia.368 dadurch unterscheiden, daß $R^4$ für Chlor steht und Z eine Gruppe -N=C(XR$^6$)-Obedeutet, die über den Stickstoff an α gebunden ist:

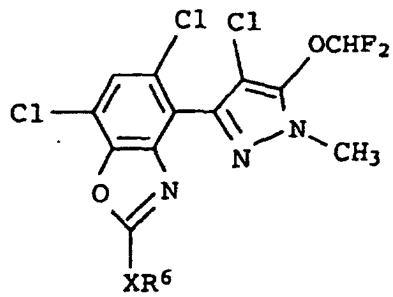

IM

- die Verbindungen IN.001 bis IN.368 die sich von den entsprechenden Verbindungen Ia.001 bis Ia.368 dadurch unterscheiden, daß $R^4$ für Fluor steht und Z eine Gruppe -N=C(XR$^6$)-Obedeutet, die über den Stickstoff an α gebunden ist:

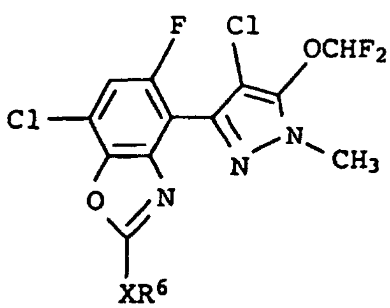

IN

- die Verbindungen IO.001 bis IO.368, die sich von den entsprechenden Verbindungen Ia.001 bis Ia.368 dadurch unterscheiden, daß $R^2$ für Trifluormethyl steht und Z eine Gruppe -N=C(XR$^6$)-O- bedeutet, die über den Stickstoff an α gebunden ist:

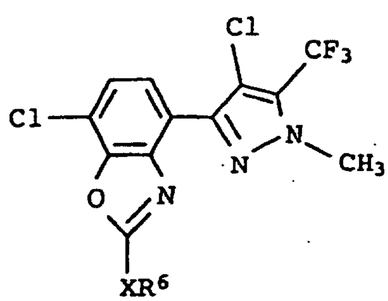

IO

- die Verbindungen IP.001 bis IP.368, die sich von den entsprechenden Verbindungen Ia.001 bis Ia.368 dadurch unterscheiden, daß $R^2$ für Trifluormethyl und $R^4$ für Chlor stehen und Z eine Gruppe -N=C($XR^6$)-O- bedeutet, die über den Stickstoff an α gebunden ist:

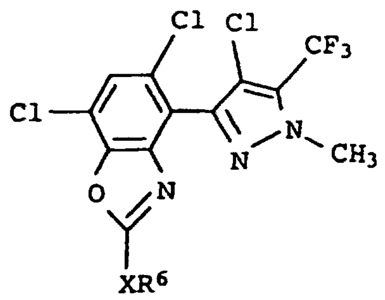

IP

- die Verbindungen IQ.001 bis IQ.368, die sich von den entsprechenden Verbindungen Ia.001 bis Ia.368 dadurch unterscheiden, daß $R^2$ für Trifluormethyl und $R^4$ für Fluor stehen und Z eine Gruppe -N=C($XR^6$)-O- bedeutet, die über den Stickstoff an α gebunden ist:

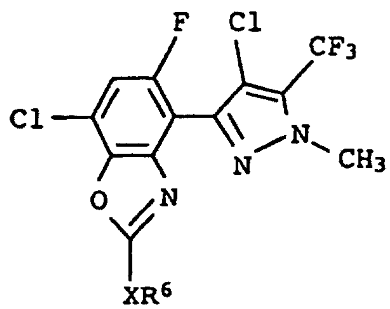

IQ

- die Verbindungen IR.001 bis IR.368, die sich von den entsprechenden Verbindungen Ia.001 bis Ia.368 dadurch unterscheiden, daß $R^2$ für Methylsulfonyl steht und Z eine Gruppe -N=C($XR^6$)-O- bedeutet, die über den Stickstoff an α gebunden ist:

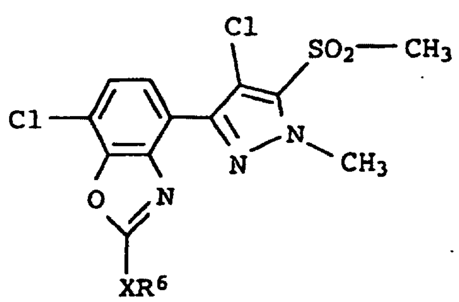

IR

- die Verbindungen IS.001 bis IS.368, die sich von den entsprechenden Verbindungen Ia.001 bis Ia.368 dadurch unterscheiden, daß $R^2$ für Methylsulfonyl und $R^4$ für Chlor stehen und Z eine Gruppe -N=C($XR^6$)-O- bedeutet, die über den Stickstoff an α gebunden ist:

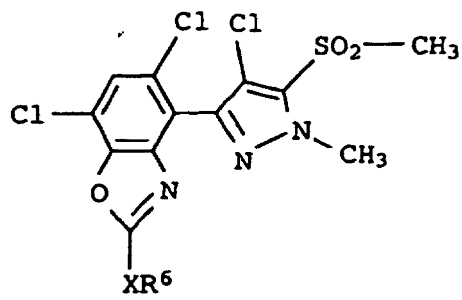

IS

- die Verbindungen IT.001 bis IT.368, die sich von den entsprechenden verbindungen Ia.001 bis Ia.368 dadurch unterscheiden, daß $R^2$ für Methylsulfonyl und $R^4$ für Fluor stehen und Z eine Gruppe -N=C(XR$^6$)-O- bedeutet, die über den Stickstoff an α gebunden ist:

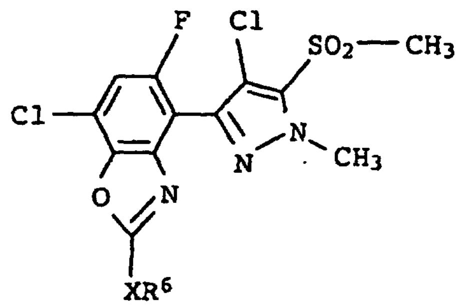

IT

Die substituierten Pyrazol-3-ylbenzazole der Formel I sind auf verschiedene Weise erhältlich, insbesondere nach einem der folgenden Verfahren:

A) Umsetzung eines Aminophenylpyrazols der Formel IIIa oder IIIb mit einem Halogen und Ammoniumthiocyanat oder mit einem Alkalioder Erdalkalimetallthiocyanat:

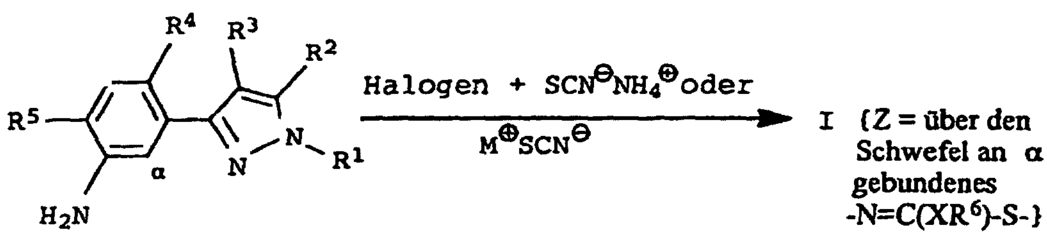


IIIa

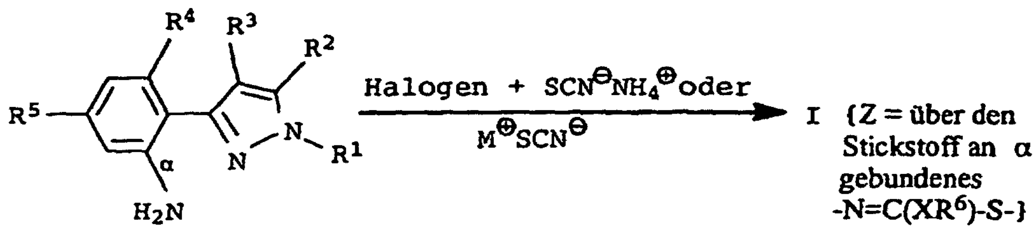


IIIb

$M^{\oplus}$ = Alkali- oder 1/2 Erdalkalimetallion

Bevorzugtes Halogen ist Chlor oder Brom; unter den Alkali-/Erdalkalimetallthiocyanaten ist Natriumthiocyanat bevorzugt.

In der Regel arbeitet man in einem inerten Lösungs-/Verdünnungsmittel, z.B. in einem Kohlenwasserstoff wie Toluol und Hexan, in einem halogenierten Kohlenwasserstoff wie Dichlormethan, in einem Ether wie Tetrahydrofuran, in einem Alkohol wie Ethanol, in einer Carbonsäure wie Essigsäure, oder in einem aprotischen Solvens wie Dimethylformamid, Acetonitril und Dimethylsulfoxid.

Die Reaktionstemperatur liegt normalerweise zwischen Schmelzund Siedepunkt des Reaktionsgemisches, vorzugsweise bei 0 bis 150°C.

Um eine möglichst hohe Ausbeute an Wertprodukt zu erzielen verwendet man Halogen und Ammoniumthiocyanat bzw. Alkali-/Erdalkalimetallthiocyanat in etwa äquimolarer Menge oder im Überschuß, bis etwa zur 5fachen molaren Menge, bezogen auf die Menge an IIIa oder IIIb.

Eine Variante des Verfahrens besteht darin, das Aminophenylpyrazol IIIa oder IIIb zunächst mit Ammoniumthiocyanat oder einem Alkali- oder Erdalkalimetallthiocyanat zu einem Thioharnstoff IVa oder IVb

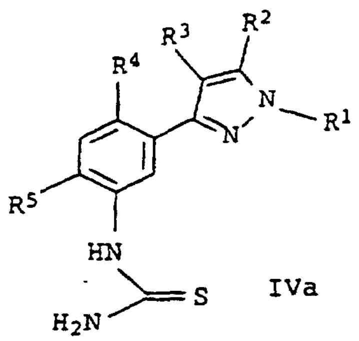
IVa

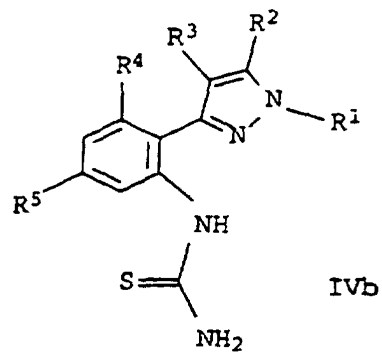
IVb

umzusetzen, und IVa oder IVb durch Behandlung mit Halogen anschließend in I mit Z = -N=C(XR$^6$)-S- zu überführen.

B) Diazotierung eines Aminophenylpyrazols der Formel IIIa oder IIIb, Überführung des jeweiligen Diazoniumsalzes in ein Azidophenylpyrazol der Formel Va oder Vb und dessen Umsetzung entweder

B.1) mit einer Carbonsäure oder

B.2) zunächst mit einer Sulfonsäure (zu VIa oder VIb), Hydrolyse des gebildeten Sulfonats zum Aminophenol VIIa oder VIIb, und deren Überführung in I:

B.1)

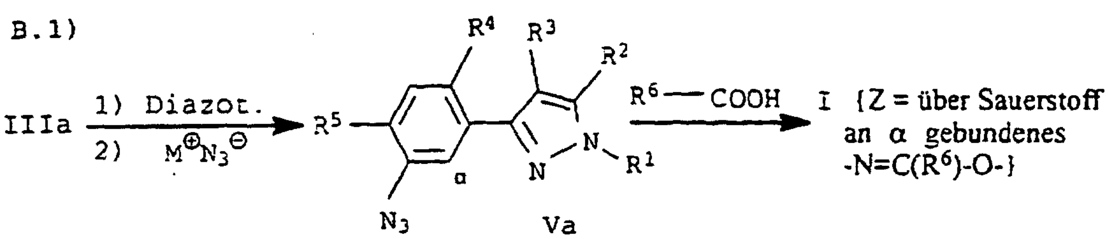


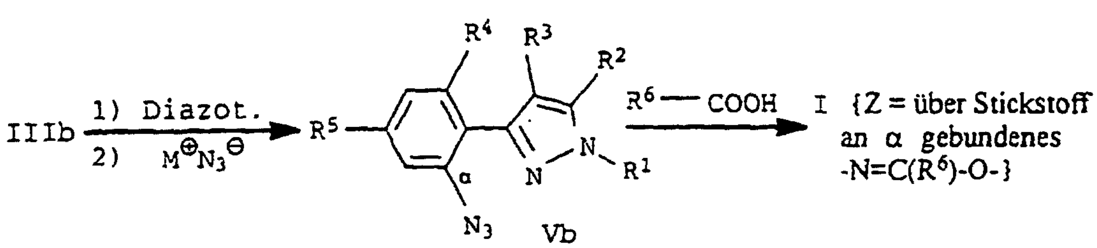


M⊕ steht für eine Alkalimetallion oder 1/2 Erdalkalimetallion.

B.2)

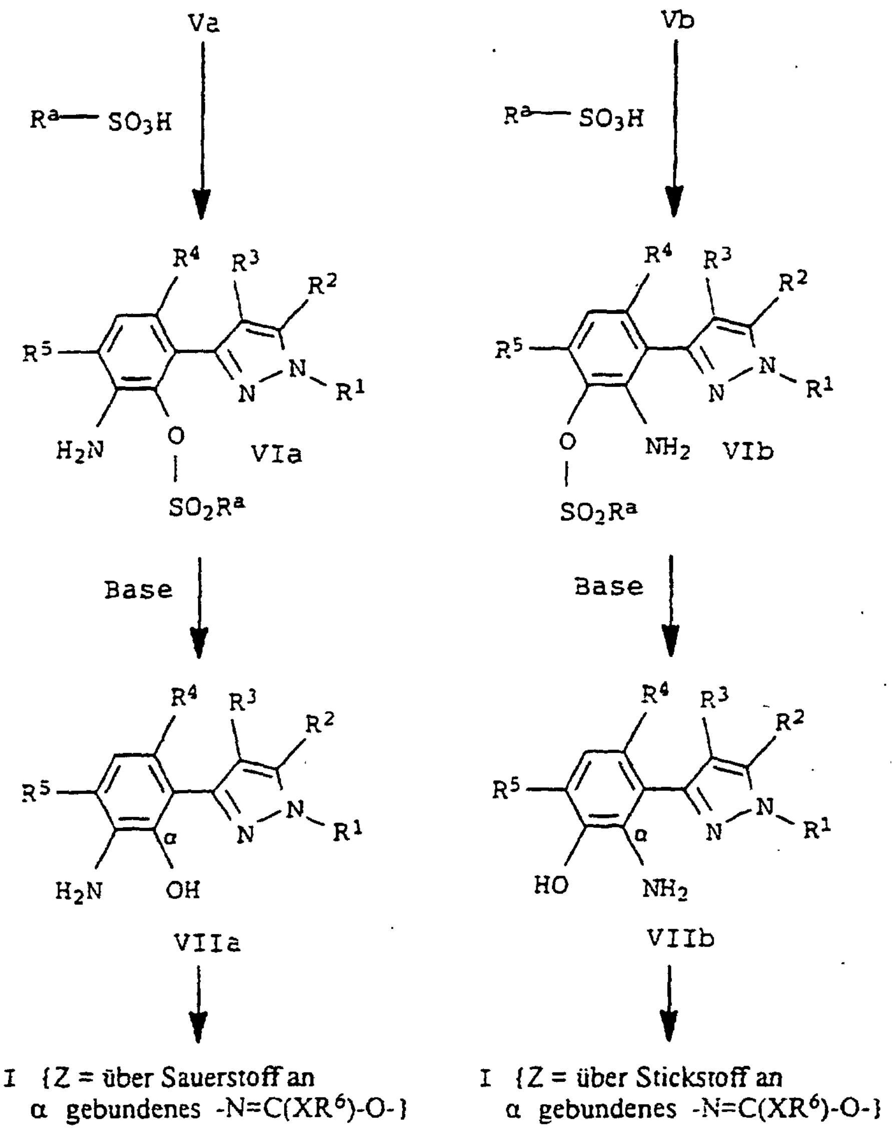


Für die Durchführung der Diazotierung gelten die bei Verfahren C) gemachten Angaben. Die Überführung in die Arylazide Va/Vb erfolgt vorzugsweise durch Umsetzung von IIIa/IIIb mit einem Alkali- oder Erdalkalimetallazid wie Natriumazid oder durch Umsetzung mit Trimethylsilylazid.

Bei der unter B.1) genannten Umsetzung mit einer Carbonsäure arbeitet man entweder in einem inerten Lösungsmittel, z.B. einem Ether wie Tetrahydrofuran und Dioxan, einem aprotischen Solvens wie Dimethylformamid und Acetonitril, einem Kohlenwasserstoff wie Toluol und Hexan, einem halogenierten Kohlenwasserstoff wie Dichlormethan, oder lösungsmittelfrei in einem Überschuß an Carbonsäure $R^6$-COOH. Im letzteren Fall kann der Zusatz einer Mineralsäure wie Phosphorsäure hilfreich sein.

Die Umsetzung wird vorzugsweise bei erhöhter Temperatur, beispielsweise bei der Siedetemperatur des Reaktionsgemisches, vorgenommen.

Für die unter B.2) zunächst genannte Umsetzung von Va/Vb mit einer Sulfonsäure $R^a$-$SO_3H$ (wobei $R^a$ für $C_1$-$C_4$-Alkyl- oder $C_1$-$C_4$-Halogenalkyl, vorzugsweise Methyl oder Trifluormethyl, steht) gelten die vorstehend für die Umsetzung von Va/Vb mit $R^6$-COOH gemachten Angaben.

Die anschließende Hydrolyse der Sulfonate VIa/VIb erfolgt vorzugsweise durch Umsetzung mit einer wäßrigen Base wie Natron- und Kalilauge, wobei gewünschtenfalls ein Lösungsmittel, z.B. ein Ether wie Dioxan und Tetrahydrofuran, oder ein Alkohol wie Methanol und Ethanol, zugesetzt werden kann.

Die abschließende Umsetzung zu I ist an sich bekannt und kann auf außerordentlich vielfältige Weise erfolgen. Hierzu sei auf die Angaben in Houben-Weyl, Methoden der Organischen Chemie, Georg Thieme Verlag Stuttgart,

Bd E8a 1993, S. 1032 ff., verweisen.

C) Diazotierung von substituierten Pyrazol-3-ylbenzazolen der Formel I, bei denen $XR^6$ für Amino steht, und anschließender Überführung des Diazoniumsalzes in Verbindungen I mit

- $-XR^6$ = Cyano oder Halogen (zur Sandmeyer-Reaktion vgl. beispielsweise Houben-Weyl, Methoden der Organischen Chemie, Georg Thieme Verlag Stuttgart, Bd. 5/4, 4. Auflage 1960, S. 438ff.},
- -X- = Schwefel {vgl. hierzu beispielsweise Houben-Weyl, Methoden der Organischen Chemie, Georg Thieme Verlag Stuttgart, Bd. E11 1984, S. 43 und 176},
- $-XR^6$ = z.B. $-CH_2-CH$ (Halogen)-$R^8$, $-CH=CH-R^8$, -CH=C(Halogen)-$R^8$ {allgemein handelt es sich hierbei um Produkte einer Meerwein-Arylierung; vgl. hierzu beispielsweise C.S. Rondestredt, Org. React. 11, 189 (1960) und H.P. Doyle et al., J. Org. Chem. 42, 2431 (1977)}:

$$I\ \{-XR^6 = NH_2\} \xrightarrow{\text{Diazot.}} \longrightarrow I\ \{-XR^6 = \text{z.B. CN, Halogen, }-SR^6,\ -CH_2-CH(\text{Halogen})-R^8,\ -CH=CH-R^8,\ -CH=C(\text{Halogen})-R^8\}$$

Im allgemeinen erhält man das Diazoniumsalz auf an sich bekannte Weise durch Umsetzung von I mit $-XR^6$ = Amino in einer wäßrigen Säurelösung, z.B. in Salzsäure, Bromwasserstoffsäure oder Schwefelsäure, mit einem Nitrit wie Natriumnitrit und Kaliumnitrit.

Es besteht aber auch die Möglichkeit, wasserfrei, z.B. in Chlorwasserstoff haltigem Eisessig, in absolutem Alkohol, in Dioxan oder Tetrahydrofuran, in Acetonitril oder in Aceton zu arbeiten und hierbei die Ausgangsverbindung (I mit $-XR^6 = NH_2$) mit einem Salpetrigsäureester wie tert.-Butylnitrit und Isopentylnitrit zu behandeln.

Die Überführung des so erhaltenen Diazoniumsalzes in die entsprechende Verbindung I mit $-XR^6$ = Cyano, Chlor, Brom oder Iod erfolgt besonders bevorzugt durch Behandeln mit einer Lösung oder Suspension eines Kupfer(I)salzes wie Kupfer(I)cyanid, -chlorid, -bromid und iodid, oder mit einer Alkalimetallsalz-Lösung.

Verbindungen I mit -X- = Schwefel erhält man normalerweise durch Umsetzung des Diazoniumsalzes mit einem Dialkyldisulfid wie Dimethyldisulfid und Diethyldisulfid, oder mit z.B. Diallyldisulfid oder Dibenzyldisulfid.

Bei der Meerwein-Arylierung handelt es sich üblicherweise um die Umsetzung der Diazoniumsalze mit Alkenen (hier $H_2C=CH-R^8$) oder Alkinen (hier $HC\equiv C-R^8$). Das Alken oder Alkin wird dabei vorzugsweise im Überschuß, bis etwa 3000 mol-%, bezogen auf die Menge des Diazoniumsalzes, eingesetzt.

Die vorstehend beschriebenen Umsetzungen des Diazoniumsalzes können z.B. in Wasser, in wässriger Salzsäure oder Bromwasserstoffsäure, in einem Keton wie Aceton, Diethylketon und Methylethylketon, in einem Nitril wie Acetonitril, in einem Ether wie Dioxan und Tetrahydrofuran oder in einem Alkohol wie Methanol und Ethanol erfolgen.

Sofern nicht bei den einzelnen Umsetzungen anders angegeben liegen die Reaktionstemperaturen normalerweise bei (-30) bis +50°C.

Bevorzugt werden alle Reaktionspartner in etwa stöchiometrischen Mengen eingesetzt, jedoch kann auch ein Überschuß der einen oder anderen Komponente, bis etwa 3000 mol-%, von Vorteil sein.

D) Oxidation eines substituierten Pyrazol-3-ylbenzazols I, bei dem X für Schwefel steht, zu I mit X = -SO- auf an sich bekannte Weise (vgl. z.B. Houben-Weyl, Methoden der Organischen Chemie, Georg Thieme Verlag Stuttgart, Bd. E 11/1, 1985, S. 702 ff., Bd. IX, 4. Auflage, 1955, S. 211):

$$I\ \{X = S\} \xrightarrow{\text{Oxidationsmittel}} I\ \{X = SO\}$$

Geeignete Oxidationsmittel sind z.B. wasserstoffperoxid, organische Peroxide wie Essigsäureperoxid, Trifluoressigsäureperoxid, m-Chlorperbenzoesäure, tert.-Butylhydroperoxid und tert.-Butylhypochlorit, sowie anorganische Verbindungen wie Natriummetaiodat, Chromsäure und Salpetersäure.

Üblicherweise arbeitet man - je nach Oxidationsmittel - in einer organischen Säure wie Essigsäure und Trichloressigsäure, in einem chlorierten Kohlenwasserstoff wie Methylenchlorid, Chloroform und 1,2-Dichlorethan, in einem aromatischen Kohlenwasserstoff wie Benzol, Chlorbenzol und Toluol oder in einem protischen Lösungsmittel wie Methanol und Ethanol. Auch Gemische der genannten Solventien kommen in Betracht.

Die Reaktionstemperatur liegt im allgemeinen bei (-30)°C bis zur Siedetemperatur des jeweiligen Reaktionsgemisches, wobei normalerweise der untere Temperaturbereich bevorzugt ist.

Zweckmäßigerweise setzt man Ausgangsverbindung und Oxidationsmittel in etwa stöchiometrischem Verhältnis ein, jedoch kann auch die eine oder andere Komponente im Überschuß eingesetzt werden.

E) Oxidation eines substituierten Pyrazol-3-ylbenzazols I, bei dem X für Schwefel oder -SO- steht, zu I mit X = -$SO_2$- auf an sich bekannte Weise (vgl. z.B. Houben-Weyl, Methoden der Organischen Chemie. Georg Thieme Verlag Stuttgart, Bd. E 11/2, 1985, S. 1132 ff. und Bd. IX, 4. Auflage, 1955, S. 222 ff.):

$$\text{I } \{X = S,\ SO\} \xrightarrow{\text{Oxidationsmittel}} \text{I } \{X = SO_2\}$$

Als Oxidationsmittel eignen sich beispielsweise Wasserstoffperoxid, organische Peroxide wie Essigsäureperoxid. Trifluoressigsäureperoxid und m-Chlorperbenzoesäure, ferner anorganische Oxidationsmittel wie Kaliumpermanganat. Die Anwesenheit eines Katalysators, z.B. Wolframat, kann sich förderlich auf den Reaktionsverlauf auswirken.

In der Regel nimmt man die Umsetzung in einem inerten Lösungsmittel vor, wobei je nach Oxidationsmittel z. B. organische Säuren wie Essigsäure und Propionsäure, chlorierte Kohlenwasserstoffe wie Methylenchlorid, Chloroform und 1,2-Dichlorethan, aromatische Kohlenwasserstoffe oder Halogenkohlenwasserstoffe wie Benzol, Chlorbenzol und Toluol, oder Wasser brauchbar sind. Auch Mischungen der genannten Lösungsmittel kommen in Betracht.

Normalerweise arbeitet man bei (-30)°C bis zur Siedetemperatur des jeweiligen Reaktionsgemisches, vorzugsweise bei 10°C bis zur Siedetemperatur.

Zweckmäßigerweise werden die Ausgangsverbindung I mit X = Schwefel oder SO und das Oxidationsmittel in etwa stöchiometrischen Mengen eingesetzt. Zur Optimierung des Umsatzes an Ausgangsverbindung kann aber ein Überschuß an Oxidationsmittel empfehlenswert sein.

F) Umsetzung eines substituierten Pyrazol-3-ylbenzazols I, bei dem die Gruppierung -$XR^6$ für Chlor, Brom, Alkylsulfonyl oder Halogenalkylsulfonyl steht, in Gegenwart einer Base mit einem Alkohol, Mercapcan, Amin oder einer CH-aciden Verbindung (VIII):

$$\text{I } \{-XR^6 = Cl,\ Br,\ -SO_2\text{-Alkyl},\ -SO_2\text{-Halogenalkyl}\} + \left\{\begin{array}{l} HOR^6, \\ HSR^6, \\ H_2NR^6, \\ HN(R^6)R^7 \\ \text{oder} \\ H_2C(R^b)R^c \end{array}\right. \xrightarrow{\text{Base}} \text{I } \{-XR^6 = OR^6,\ SR^6,\ NHR^6,\ N(R^6)R^7,\ CH(R^b)R^c\}$$

VIII

$R^b$ und $R^c$ stehen unabhängig voneinander für Cyano oder ($C_1$-$C_4$-Alkoxy)carbonyl.

Zweckmäßigerweise nimmt man die Umsetzung in einem inerten Lösungsmittel vor, beispielsweise in einem Ether wie Diethylether, Methyl-tert.-butylether, Dimethoxyethan, Diethylenglycoldimethylether, Tetrahydrofuran und Dioxan, einem Keton wie Aceton, Diethylketon, Ethylmethylketon und Cyclohexanon, einem dipolaren aprotischen Lösungsmittel wie Acetonitril, Dimethylformamid. N-Methylpyrrolidon und Dimethylsulfoxid, einem protischen Lösungsmittel wie Methanol und Ethanol, einem aromatischen, gewünschtenfalls halogenierten Kohlenwasserstoff wie Benzol, Chlorbenzol und 1,2-Dichlorbenzol, einem heteroaromatischen Lösungsmittel wie Pyridin und Chinolin oder in einem Gemisch solcher Lösungsmittel. Tetrahydrofuran, Aceton, Diethylketon und Dimethylformamid sind bevorzugt.

Als Basen können hierbei z.B. die Hydroxide, Hydride, Alkoxide, Carbonate oder Hydrogencarbonate von Alkalimetall- und Erdalkalimetallkationen, tertiäre aliphatische Amine wie Triethylamin, N-Methylmorpholin und N-Ethyl-N,N-diisopropyl-amin, biund tricyclische Amine wie Diazabicycloundecan (DBU) und Diazabicyclooctan (DABCO), oder aromatische Stickstoffbasen wie pyridin, 4-Dimethylaminopyridin und Chinolin, dienen. Auch Kombinationen verschiedener Basen kommen in Betracht. Bevorzugte Basen sind Natriumhydrid, Natriumhydroxid, Natriumcarbonat, Kaliumcarbonat, Natriummethylat, Natriumethylat und Kaliumtert. butylat.

Die Amine $H_2NR^6$ oder $HN(R^6)R^7$ können als Reaktionspartner und gleichzeitig als Base dienen, wobei dann das Amin in mindestens doppeltem Überschuß, bezogen auf die Menge an Ausgangsverbindung I, vorliegen sollte. Selbstverständlich ist auch ein größerer Überschuß an Amin möglich, bis etwa zur 10fachen molaren Menge, bezogen auf die Menge an I mit $-XR^6$ = Cl, Br, $-SO_2$-Alkyl oder $-SO_2$-Halogenalkyl.

Üblicherweise setzt man die Ausgangsstoffe in etwa stöchiometrischen Mengen ein, jedoch kann auch ein Überschuß der einen oder anderen Komponente im Hinblick auf die verfahrensführung oder eine möglichst vollständige Umsetzung der Ausgangsverbindung I {$-XR^6$ = Cl, Br, $-SO_2$-Alkyl, $-SO_2$-Halogenalkyl} vorteilhaft sein.

Das molare Verhältnis von Alkohol, Mercaptan, Amin oder CH-acider Verbindung (VIII) zu Base beträgt im allgemeinen 1:1 bis 1:3.

Die Konzentration der Ausgangsstoffe im Lösungsmittel liegt normalerweise bei 0,1 bis 5,0 mol/l.

Die Umsetzung kann bei Temperaturen von 0°C bis zur Rückflußtemperatur des jeweiligen Reaktionsgemisches durchgeführt werden.

G) Umsetzung eines substituierten Pyrazol-3-ylbenzazols I, bei dem $-XR^6$ für Halogen steht, mit einer ($C_1$-$C_6$-Alkyl)-Grignard-Verbindung:

$$\text{I}\ \{-XR^6 = \text{Halogen}\} \xrightarrow{(C_1\text{-}C_6\text{-Alkyl})\text{-MgHal}} \text{I}\ \{-XR^6 = \text{H},\ C_1\text{-}C_6\text{-Alkyl}\}$$

Hal steht dabei für Chlorid oder Bromid.

In der Regel arbeitet man in einem inerten Lösungs-/verdünnungsmittel, beispielsweise einem Kohlenwasserstoff wie Hexan und Toluol, oder einem Ether wie Diethylether, Tetrahydrofuran und Dioxan.

Gewünschtenfalls kann dabei ein Übergangsmetallkatalysator, in Mengen von 0,0001 bis 10 mol-%, zugesetzt werden. Hierfür kommen beispielsweise Nickel- und Palladiumkatalysatoren wie Nickeldichlorid, Bis(triphenylphosphin)nickeldichlorid, [Bis-(1,2-diphenylphosphino)ethan]nickeldichlorid, [Bis-(1,3-diphenylphosphino)propan]nickeldichlorid, Palladiumdichlorid, Tetrakis(triphenylphosphin)palladium, Bis(triphenylphosphin)palladiumdichlorid, [Bis-(1,2-diphenylphosphino)-ethan]palladiumdichlorid, [Bis-(1,3-diphenylphosphino)propan]-palladiumdichlorid und [Bis(diphenylphospino)ferrocen]-palladiumdichlorid, aber auch Mischungen von Palladiumoder Nickeldichlorid und Phosphinen wie Triphenylphosphin, Bis-1,2-(Diphenylphosphino)ethan und Bis-1,3-(Diphenylphosphino)propan in Betracht.

Je nach Reaktionsführung entstehen dabei Verbindungen I mit $-XR^6$ = Wasserstoff oder $C_1$-$C_6$-Alkyl oder entsprechende Mischungen aus alkylierter und nicht-alkylierter Verbindung I, die jedoch auf übliche Weise getrennt werden können.

In der Regel arbeitet man bei (-100)°C bis zur Siedetemperatur des Reaktionsgemisches.

Die Menge an Grignard-Reagenz ist nicht kritisch; normalerweise verwendet man ($C_1$-$C_6$-Alkyl)-MgHal in ca. äquimolarer Menge oder im Überschuß, bis etwa zur 10fachen molaren Menge, bezogen auf die Menge an I mit $-XR^6$ = Halogen.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel auf an sich bekannte Weise. Sofern nicht bei den vorstehend beschriebenen Verfahren etwas anderes angegeben ist, erhält man die Wertprodukte z.B. nach Verdünnen der Reaktionslösung mit Wasser 5 durch Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten Pyrazol-3-ylbenzazole I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die weitgehend reinen Isomeren getrennt werden können. Reine optisch aktive Isomere lassen sich vorteilhaft aus entsprechenden optisch aktiven Ausgangsprodukten herstellen.

Landwirtschaftlich brauchbare Salze der Verbindungen I können durch Reaktion mit einer Base des entsprechenden Kations, vorzugsweise einem Alkalimetallhydroxid oder -hydrid, oder durch Reaktion mit einer Säure des entsprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium). Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwendet werden.

Des weiteren eignen sich die substituierten Pyrazol-3-ylbenzazole I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldisper-5 sionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen z.B. die Alkali-, Erdalkali-, Ammoniumsalze von aromatischen Sulfonsäuren wie Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, LöB, Ton, Dolomit und Diatomeenerde, Calciumund Magnesiumsulfat, Magnesiumoxid, gemahlene Kunststoffe, Düngemittel wie Ammoniumsulfat, Ammoniumphosphat und Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa 0,001 bis 98 Gew.-%, vorzugsweise 0,01 bis 95 Gew.-%, mindestens eines Wirkstoffs. Die Wirkstoffe I werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:

I. 20 Gewichtsteile der Verbindung Nr. Ic.001 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.

II. 20 Gewichtsteile der Verbindung Nr. Ic.305 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.

III. 20 Gewichtsteile des Wirkstoffs Nr. Ic.327 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungs-5 produktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.

IV. 20 Gewichtsteile des Wirkstoffs Nr. If.001 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.

V. 3 Gewichtsteile des Wirkstoffs Nr. Ic.094 werden mit 97 Gewichtsteilen feinteiligem Kaolin vermischt. Man erhält auf diese Weise ein Stäubemittel, das 3 Gew.-% des wirkstoffs enthält.

VI. 20 Gewichtsteile des Wirkstoffs Nr. If.056 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines phenol-Harnstoff-Formaldehyd-Kondensates und 68 Gewichtsteilen eines paraffinischen. Mineralöls innig vermischt. Man erhält eine stabile ölige Dispersion.

VII. 1 Gewichtsteil der Verbindung Nr. Ij.040 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Anschließend kann die Mischung mit Wasser auf die gewünschte Wirkstoffkonzentration verdünnt werden. Man erhält ein stabiles Emulsionskonzentrat.

VIII. 1 Gewichtsteil der Verbindung Nr. Iq.003 wird in einer Mischung gelöst, die aus 80 Gewichtsteilen Cyclohexan und 20 Gewichtsteilen Wettol® EM 31 (= nichtionischer Emulgator auf der Basis von ethoxyliertem Rizinusöl; BASF AG) besteht. Danach kann mit Wasser auf die gewünschte Wirkstoffkonzentration verdünnt werden. Man erhält ein stabiles Emulsionskonzentrat.

Die Applikation der Wirkstoffe I bzw. der herbiziden Mittel kann im Vorauflauf- oder im Nachauflaufverfahren

erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha, aktive Substanz (a.S.).

Zur Verbreiterung des Wirkungsspektrums und zur Erzielung synergistischer Effekte können die substituierten Pyrazol-3-ylbenz(ox/thi)azole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-$CF_3$-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxyund Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

Beispiel 1

2-Amino-4-chlor-7-(4-chlor-5-difluormethoxy-1-methyl-1H-pyrazol-3-yl)-6-fluorbenzthiazol (Nr. Ic.075)

Zu einer Lösung von 29,3 g (90 mmol) 2-Chlor-5-(4-chlor-5-difluormethoxy-1-methyl-1H-pyrazol-3-yl)-4-fluoranilin in 500 ml Essigsäure wurden 29 g (0,36 mol) Natriumthiocyanat gegeben. Nach 10 Minuten Rühren gab man 28,7 g (0,18 mol) Brom zu dem Reaktionsgemisch. Anschließend wurde noch 16 Std. gerührt. Dann goß man die Mischung auf 2 l Wasser. Das entstandene feste Wertprodukt wurde abfiltriert, mit Wasser gewaschen und schließlich getrocknet. Ausbeute: quantitativ;
$^1$H-NMR (270 MHz; in $CDCl_3$): $\delta$ [ppm] = 3,86 (s, 3H), 5,44 (s,2H), 6,74 (t. 1H), 7,24 (d, 1H).

Beispiel 2

4-Chlor-7-(4-chlor-5-difluormethoxy-1-methyl-1H-pyrazol-3-yl)-6-fluor-2-(methylthio)benzthiazol (Nr. Ic.094)

Zu einer Lösung von 0,5 g (1,3 mmol) 2-Amino-4-chlor-7-(4-chlor-5-difluormethoxy-1-methyl-1H-pyrazol-3-yl)-6-fluorbenzthiazol in 50 ml Dichlormethan wurden 0,4 g (3,9 mmol) Dimethyldisulfid und 1,3 g (12,7 mmol) tert. -Butylnitrit gegeben. Nach 16 Std. rühren wurde die Reaktionsmischung eingeengt. Die Reinigung des Rohproduktes erfolgte mittels Kieselgelchromatographie (Eluens: Hexan/Ethylacetat = 4:1). Ausbeute: 0,3 g;
$^1$H-NMR (270 MHz; in $CDCl_3$) : $\delta$ [ppm] = 2,81 (s, 3H) , 3, 88 (s, 3H) , 6,75 (t, 1H), 7,35 (d, 1H).

Beispiel 3

2-Brom-4-chlor-7-(4-chlor-5-difluormethoxy-1-methyl-1H-pyrazol-3-yl)-6-fluorbenzthiazol (Nr. Ic.038)

Zu einer Lösung von 26,4 g (68 mmol) 2-Amino-4-chlor-7-(4-chlor-5-difluormethoxy-1-methyl-1H-pyrazol-3-yl)-6-fluorbenzthiazol in 1 1 Acetonitril wurden zunächst 35,2 g (0,34 mol) Natriumbromid und 19,6 g (0,14 mol) Kupfer(I)bromid gegeben und dann 9,2 g (89 mmol) tert.-Butylnitrit getropft. Nach 16 Std. Rühren versetzte man die Reaktionsmischung mit 0,2 1 verdünnter Salzsäure. Anschließend wurde der Feststoffanteil abfiltriert und mit 200 ml Ethylacetat gewaschen. In der wässrigen Phase verbliebenes Wertprodukt extrahierte man noch mit 200 ml Ethylacetat. Die vereinigten organischen Phasen wurden mit Wasser gewaschen, über Magnesiumsulfat getrocknet und schließlich eingeengt. Die Reinigung des Rohproduktes erfolgte mittels Kieselgelchromatographie (Eluens: Ethylacetat); Ausbeute: 17,3 g.

Beispiel 4

2-Amino-7- (4-chlor-5-difluormethoxy-1-methyl-1H-pyrazol-3-yl)-4,6-dichlorbenzthiazol (Nr. Ib.075)

4,4 g (13 mmol) 5-(4-Chlor-5-difluormethoxy-1-methyl-1H-pyrazol-3-yl)-2,4-dichloranilin, 4,2 g (52 mmol) Natriumthiocyanat und 4,1 g (26 mmol) Brom wurden analog Beispiel 1 umgesetzt.
Ausbeute: quantitativ.

Beispiel 5

7-(4-Chlor-5-difluormethoxy-1-methyl-1H-pyrazol-3-yl)-4,6-dichlor-2-(ethylthio)benzthiazol (Nr. Ib.095)

0,8 g (2 mmol) 2-Amino-7-(4-chlor-5-difluormethoxy-1-methyl-1Hpyrazol-3-yl)-4,6-dichlorbenzthiazol, 0,7 g (6 mmol) Diethyldisulfid und 0,3 g (3 mmol) tert.-Butylnitrit wurden analog Beispiel 2 umgesetzt. Ausbeute: 0,2 g.

Beispiel 6

2-Brom-7-(4-chlor-5-difluormethoxy-1-methyl-1H-pyrazol-3-yl)-4,6-dichlorbenzthiazol (Nr. Ib.038)

3,6 g (9 mmol) 2-Amino-7-(4-chlor-5-difluormethoxy-1-methyl-1Hpyrazol-3-yl)-4,6-dichlorbenzthiazol, 4,6 g (45 mmol) Natriumbromid, 2,6 g (18 mmol) Kupfer(I)bromid und 1,2 g, (12 mmol) tert.-Butylnitrit wurden analog Beispiel 3 umgesetzt. Ausbeute: 1,8 g.

Beispiel 7

7-(4-Chlor-5-difluormethoxy-1-methyl-1H-pyrazol-3-yl)-4,6-dichlorbenzthiazol (Nr. Ib.001)

Zu einer Lösung von 0,45 g (1 mmol) 2-Brom-7-(4-chlor-5-difluormethoxy-1-methyl-1H-pyrazol-3-yl)-4,6-dichlorbenzthiazol in 6 ml Diethylether wurden 3 ml einer 2-molaren Lösung von Propylmagnesiumchlorid in Diethylether (= 6 mmol $C_3H_7MgCl$) gegeben. Nach 5 Std. Rühren versetzte man die Mischung mit 10 ml verdünnter Salzsäure. Anschließend wurde die organische Phase abgetrennt, noch zweimal mit Wasser gewaschen, dann über Magnesiumsulfat getrocknet und schließlich eingeengt. Die Reinigung des Rohproduktes erfolgte mittels Kieselgelchromatographie (Eluens: Hexan/Ethylacetat = 1:1). Ausbeute: 0,3 g.

Beispiel 8

2-Amino-4-chlor-7-(4-chlor-1-methyl-5-trifluormethyl-1H-pyrazol-3-yl)-6-fluorbenzthiazol (Nr. If.075)

80 g (0,24 mol) 2-Chlor-5-(4-chlor-1-methyl-5-trifluormethyl-1Hpyrazol-3-yl)-4-fluoranilin, 79 g (0,98 mol) Natriumthiocyanat und 78 g (0,49 mol) Brom wurden analog Beispiel 1 umgesetzt. Ausbeute: quantitativ.

Beispiel 9

4-Chlor-7-(4-chlor-1-methyl-5-trifluormethyl-1H-pyrazol-3-yl)-6-fluor-2-(methylthio)benzthiazol (Nr. If.094)

0,7 g (1,8 mmol) 2-Amino-4-chlor-7-(4-chlor-1-methyl-5-trifluor-methyl-1H-pyrazol-3-yl)-6-fluorbenzthiazol, 0,5 g (5,4 mmol) Dimethyldisulfid und 0,33 g (2,7 mmol) tert.-Butylnitrit wurden analog Beispiel 2 umgesetzt. Ausbeute: 0,3 g.

Beispiel 10

2-Brom-4-chlor-7-(4-chlor-1-methyl-5-trifluormethyl-1H-pyrazol-3-yl)-6-fluorbenzthiazol (Nr. If.038)

73 g (0,12 mol) 2-Amino-4-chlor-7-(4-chlor-1-methyl-5-trifluormethyl-1H-pyrazol-3-yl)-6-fluorbenzthiazol, 126 g (1,22 mmol) 5 Natriumbromid, 35 g (0,24 mol) Kupfer(I)bromid und 16,3 g (0,36 mol) tert.-Butylnitrit wurden analog Beispiel 3 umgesetzt. Ausbeute: 18,2 g.

Beispiel 11

N-(4-Chlor-7-(4-chlor-1-methyl-5-trifluormethyl-1H-pyrazol-3-yl)-6-fluorbenzthiazol-2-yl)acetamid (Nr. If.329)

Zu einer Lösung von 0,38 g (6,4 mmol) Acetamid in 20 ml Tetrahydrofuran wurden zunächst 0,12 g (4,7 mmol) Natriumhydrid und 5 dann 0,7 g (1,6 mmol) 2-Brom-4-chlor-7-(4-chlor-1-methyl-5-trifluormethyl-1H-pyrazol-3-yl)-6-fluorbenzthiazol gegeben. Anschließend rührte man 16 Std., wonach die Reaktionsmischung eingeengt wurde. Die Reinigung des so erhaltenen Rohproduktes erfolgte mittels Kieselgelchromatographie (Eluens: Hexan/Ethyl-acetat = 4:1 und 1:1). Ausbeute: 0,4 g.

Beispiel 12

3-(4-Chlor-7-(4-chlor-5-difluormethoxy-1-methyl-1H-pyrazol-3-yl)-6-fluorbenzthiazol-2-yl)acrylsäuremethylester (Nr. Ic.218)

1 g (3,1 mmol) 2-Amino-4-chlor-7-(4-chlor-5-difluormethoxy-1-methyl-1H-pyrazol-3-yl)-6-fluorbenzthiazol, 5,5 g (64 mmol) Acrylsäuremethylester, 0,45 g (3,4 mmol) Kupfer(II)chlorid und 0,33 g (3,2 mmol) tert.-Butylnitrit wurden nacheinander in 30 ml Acetonitril gelöst. Anschließend rührte man 16 Std., wonach die Reaktionsmischung mit 100 ml verdünnter Salzsäure versetzt wurde. Dann extrahierte man das entstandene Wertprodukt mit 200 ml Methyl-tert.-butylether. Die vereinigten organische Phasen wurden noch zweimal mit Wasser gewaschen, über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,13 g.

Beispiel 13

4-Chlor-7-(4-chlor-5-difluormethoxy-1-methyl-1H-pyrazol-3-yl)-6-fluorbenzthiazol (Nr. Ic.001) und 4-Chlor-7-(4-chlor-5-difluormethoxy-1-methyl-1H-pyrazol-3-yl)-6-fluor-2-methylbenzthiazol (Nr. Ic.002)

Zu einer auf Rückflußtemperatur erhitzten Lösung von 2 g (4,4 mmol) 2-Brom-4-chlor-7-(4-chlor-5-difluormethoxy-1-methyl-1H-pyrazol-3-yl)-6-fluorbenzthiazol und 0,15 g (0,22 mmol) Bis(triphenylphosphin)nickeldichlorid in 20 ml Tetrahydrofuran wurden 2,9 ml einer 3-molaren Lösung von Methylmagnesiumchlorid in Tetrahydrofuran (= 8,7 mmol $H_3CMgCl$) getropft. Anschließend rührte man noch 12 Std. bei Rückflußtemperatur, wonach die Reaktionsmischung mit 40 ml verdünnter Salzsäure versetzt wurde. Aus der wässrigen Phase extrahierte man das entstandene Produkt mit 100 ml Ethylacetat. Die vereinigten organischen Phasen wurden zweimal mit Wasser gewaschen, über Magnesiumsulfat getrocknet und dann eingeengt. Die Reinigung der Rohprodukte erfolgte mittels Kieselgelchromatographie (Eluens: Hexan/Ethylacetat = 4:1). Abschließend wurden die beiden Wertprodukte noch mittels MPLC an Kieselgel (Eluens: Cyclohexan/Ethylacetat = 20:1) getrennt. Ausbeute: 0,11 g (Verb. Ic.001) und 0,28 g (Verb. Ic.002).

Beispiel 14

4-Chlor-7-(4-chlor-5-difluormethoxy-1-methyl-1H-pyrazol-3-yl)-6-fluor-2-propylbenzthiazol (Nr. Ic.004)

Zu einer Lösung von 0,4 g (0,9 mmol) 2-Brom-4-chlor-7-(4-chlor-5-difluormethoxy-1-methyl-1H-pyrazol-3-yl)-6-fluorbenzthiazol in 30 ml Diethylether wurden 0,65 ml einer 2-molaren Lösung von Propylmagnesiumchlorid in Diethylether (= 1,3 mmol $C_3H_7MgCl$) getropft. Anschließend rührte man noch 16 Std., wonach die Reaktionsmischung mit 40 ml verdünnter Salzsäure versetzt wurde. Aus der wässrigen Phase extrahierte man das entstandene Produkt mit 100 ml Ethylacetat. Die vereinigten organischen Phasen wurden noch über Magnesiumsulfat getrocknet und dann eingeengt. Die Reinigung der Rohprodukte erfolgte mittels Kieselgelchromatographie (Eluens: Hexan/Ethylacetat = 9: 1). Ausbeute: 0,1 g.

Beispiel 15

4-Chlor-7-(4-chlor-5-difluormethoxy-1-methyl-1H-pyrazol-3-yl)-6-fluor-2-methoxybenzthiazol (Nr. Ic.039)

0,1 g (4,4 mmol) Natriumhydrid wurden in 20 ml Methanol gelöst. Nach beendeter Gasentwicklung versetzte man die Lösung mit 0,7 g (1,6 mmol) 2-Brom-4-chlor-7-(4-chlor-5-difluormethoxy-1-methyl-1H-pyrazol-3-yl)-6-fluorbenzthiazol. Anschließend wurde die Reaktionsmischung 16 Std. gerührt. Dann engte man ein. Das Rohprodukt wurde schließlich noch mittels Kieselgelchromatographie (Eluens: Hexan/Ethylacetat = 1:1) gereinigt. Ausbeute: 0,5 g.

Beispiel 16

4-Chlor-7-(4-chlor-5-difluormethoxy-1-methyl-1H-pyrazol-3-yl)-2-dimethylamino-6-fluorbenzthiazol (Nr. Ic.077)

In eine auf 70-80°C erwärmte Lösung von 0,7 g (1,6 mmol) 2-Brom-4-chlor-7-(4-chlor-5-difluormethoxy-1-methyl-1H-pyrazol-3-yl)-6-fluorbenzthiazol in 50 ml Dimethylformamid wurde ein langsamer Strom gasförmigen Dimethylamins eingeleitet, bis der Umsatz vollständig war (ca. 30 Minuten). Anschließend engte man ein, wonach der Rückstand mit 50 ml Wasser versetzt wurde. Aus der wäßrigen Phase extrahierte man das entstandene Produkt mit 100 ml Ethylacetat. Die vereinigten organische Phasen wurden noch zweimal mit Wasser gewaschen, dann über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,46 g.

Beispiel 17

N-Methyl-N-(4-chlor-7-(4-chlor-5-difluormethoxy-1-methyl-1Hpyrazol-3-yl)-6-fluorbenzthiazol-2-yl)aminoessigsäuremethylester (Nr. Ic.327)

Eine Lösung von 0,7 g (1,6 mmol) 2-Brom-4-chlor-7-(4-chlor-5-difluormethoxy-1-methyl-1H-pyrazol-3-yl)-6-fluorbenzthiazol, 0,43 g (3,1 mmol) Kaliumcarbonat und 0,43 g (3,1 mmol) Sarcosinmethylesterhydrochlorid in 30 ml Dimethylformamid wurde 2 Std. auf 100°C erwärmt. Anschließend engte man ein, wonach der Rückstand mit 50 ml Wasser versetzt wurde. Aus der wäßrige Phase extrahierte man das entstandene Produkt mit 100 ml Ethylacetat. Die vereinigten organischen Phasen wurden noch zweimal mit Wasser 5 gewaschen, über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,18 g.

Beispiel 18

2- (4-Chlor-7-(4-chlor-5-difluormethoxy-1-methyl-1H-pyrazol-3-yl)-6-fluorbenzthiazol-2-yloxy)propionsäuremethylester (Nr. Ic.305)

Zu einer Lösung von 0,65 g (6,2 mmol) 2-Hydroxypropionsäure-L5 methylester in 20 ml Tetrahydrofuran wurden 0,11 g (4,7 mmol) Natriumhydrid gegeben. Nach beendeter Gasentwicklung versetzte man die Mischung mit 0,7 g (1,6 mmol) 2-Brom-4-chlor-7-(4-chlor-5-difluormethoxy-1-methyl-1H-pyrazol-3-yl)-6-fluorbenzthiazol. Anschließend wurde die Mischung 3 Tage gerührt und dann mit 50 ml Wasser versetzt. Aus der wäßrigen Phase extrahierte man das entstandene Produkt mit 100 ml Ethylacetat. Die vereinigten organischen Phasen wurden noch zweimal mit Wasser gewaschen, über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,3 g.

Beispiel 19

2-Amino-4-chlor-7-(4-chlor-1-methyl-5-methylsulfonyl-1H-pyrazol-3-yl)-6-fluorbenzthiazol (Nr. Ij.075)

7,5 g (21 mmol) 2-Chlor-5-(4-chlor-1-methyl-5-methylsulfonyl-1Hpyrazol-3-yl)-4-fluoranilin, 6,9 g (85 mmol) Natriumthiocyanat und 6,9 g (43 mmol) Brom wurden analog Beispiel 1 umgesetzt. Ausbeute: quantitativ.

Beispiel 20

4-Chlor-7-(4-chlor-1-methyl-5-methylsulfonyl-1H-pyrazol-3-yl)-2-ethylthio-6-fluorbenzthiazol (Nr. Ij.095)

1g (2,5 mmol) 2-Amino-4-chlor-7-(4-chlor-1-methyl-5-methylsulfonyl-1H-pyrazol-3-yl)-6-fluorbenzthiazol; 0,66 g (7,3 mmol) Diethyldisulfid und 0,37 g (3,7 mmol) tert.-Butylnitrit wurden analog Beispiel 2 umgesetzt. Ausbeute: 0,4 g.

Beispiel 21

2-Brom-4-chlor-7-(4-chlor-1-methyl-5-methylsulfonyl-1H-pyrazol-3-yl)-6-fluorbenzthiazol (Nr. Ij.038)

5 6 g (14 mmol) 2-Amino-4-chlor-7-(4-chlor-1-methyl-5-methylsulfonyl-1H-pyrazol-3-yl)-6-fluorbenzthiazol,

7,6 g (70 mmol) Natriumbromid, 4,2 g (30 mmol) Kupfer(I)bromid und 2,2 g (22 mmol) tert.-Butylnitrit wurden analog Beispiel 3 umgesetzt. Ausbeute: 3,6 g.

Beispiel 22

2-(4-Chlor-7-(4-chlor-5-difluormethoxy-1-methyl-1H-pyrazol-3-yl)-6-fluorbenzthiazol-2-yl)malonsäuremonoethylmono-tert.butylester (Nr. Ic.365)

Zu einer Lösung von 0,9 g (5 mmol) Malonsäuremonoethylmono-tert.butylester in 10 ml Dimethylformamid wurden 0,1 g (4,7 mmol) Natriumhydrid gegeben. Nach beendeter Gasentwicklung versetzte man die Mischung mit 1,5 g (3,3 mmol) 2-Brom-4-chlor-7-(4-chlor-5-difluormethoxy-1-methyl-1H-pyrazol-3-yl)-6-fluorbenzthiazol. Es wurde noch eine Stunde gerührt, wonach man das Reaktionsgemisch einengte. Der Rückstand wurde mit 50 ml Wasser versetzt. Aus der wäßrigen Phase extrahierte man das Wertprodukt mit 100 ml Ethylacetat. Der Extrakt wurde über Magnesiumsulfat getrocknet und schließlich eingeengt. Ausbeute: 1 g.

Beispiel 23

2-(4-Chlor-7-(4-chlor-5-difluormethoxy-1-methyl-1H-pyrazol-3-yl-)-6-fluorbenzthiazol-2-yl)essigsäureethylester (Nr. Ic.018)

Zu einer Lösung von 1 g (1,8 mmol) 2-(4-Chlor-7-(4-chlor-5-difluormethoxy-1-methyl-1H-pyrazol-3-yl)-6-fluorbenzthiazol-2-yl)malonsäuremonoethylmono-tert.-butylester in 10 ml Dichlormethan wurden 0,30 g (2 mmol) Trifluormethansulfonsäure gegeben. Anschließend rührte man eine Stunde, wonach die Mischung eingeengt wurde. Den Rückstand versetzte man mit 10 ml Essigsäure. Dann wurde noch 8 Stunden auf Rückflußtemperatur erhitzt. Anschließend engte man die Reaktionsmischung ein. Der Rückstand wurde mit 50 ml Wasser versetzt. Aus der wässrigen Phase extrahierte man das Wertprodukt mit 100 ml Ethylacetat. Der Extrakt wurde mit verdünnter wässriger Natriumhydrogencarbonat-Lösung und Wasser gewaschen, über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,4 g.

In der folgenden Tabelle 2 sind neben den vorstehend beschriebenen Wirkstoffen noch weitere Pyrazol-3-ylbenzazole der Formel I aufgeführt, die auf analoge Weise hergestellt wurden oder herstellbar sind:

Tabelle 2

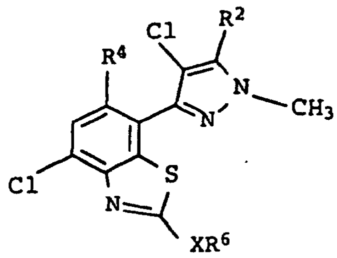

I {$R^1 = CH_3$; $R^3$, $R^5 = Cl$;
Z = über den Schwefel
an α gebundenes $-N=C(XR^6)-S-$}

| Nr. | $R^2$ | $R^4$ | $XR^6$ | Smp. / $^1H$-NMR [ppm] / MS [m/z] |
|---|---|---|---|---|
| Ib.001 | $OCHF_2$ | Cl | H | 3,89 (s,3H), 6,77 (t,1H), 7,71 (s,1H), 9,07 (s,1H) |
| Ib.002 | $OCHF_2$ | Cl | $CH_3$ | 2,85 (s,3H), 3,88 (s,3H), 6,76 (t,1H),7,63 (s,1H) |
| Ib.038 | $OCHF_2$ | Cl | Br | 3,88 (s,3H), 6,74 (t,1H), 7,66 (s,1H) |
| Ib.075 | $OCHF_2$ | Cl | $NH_2$ | 3,82 (s,3H), 7,41 (t,1H), 7,60 (s,1H), 8,04 (s,2H) {in $(CD_3)_2SO$} |
| Ib.094 | $OCHF_2$ | Cl | $SCH_3$ | 105–110°C |
| Ib.095 | $OCHF_2$ | Cl | $SC_2H_5$ | 76–77°C |
| Ic.001 | $OCHF_2$ | F | H | 3,88 (s,3H), 6,76 (t,1H), 7,47 (d,1H), 9,06 (s,1H) |
| Ic.002 | $OCHF_2$ | F | $CH_3$ | 108–110°C |
| Ic.004 | $OCHF_2$ | F | $n-C_3H_7$ | 1,06 (t,3H), 1,90 (sext.,2H), 3,10 (t,2H), 3,89 (s,3H), 6,76 (t,1H), 7,39 (d,1H) |
| Ic.018 | $OCHF_2$ | F | $CH_2-CO-OC_2H_5$ | 1,32 (t,3H), 4,10 (s,2H), 4,11 (s,3H), 4,26 (q,2H), 7,42 (d,1H) |
| Ic.038 | $OCHF_2$ | F | Br | 445 $[M]^+$, 366 $[M-Br]^+$ |
| Ic.039 | $OCHF_2$ | F | $OCH_3$ | 120°C |
| Ic.051 | $OCHF_2$ | F | $OCH_2-C\equiv CH$ | 117–118°C |
| Ic.075 | $OCHF_2$ | F | $NH_2$ | 3,82 (s,3H), 7,41 (t,1H), 7,49 (d,1H),7,89 (s,2H) {in $(CD_3)_2SO$} |
| Ic.077 | $OCHF_2$ | F | $N(CH_3)_2$ | 150°C |
| Ic.094 | $OCHF_2$ | F | $SCH_3$ | 96–98°C |
| Ic.218 | $OCHF_2$ | F | $CH=CH-CO-OCH_3$ | 158–160°C |
| Ic.291 | $OCHF_2$ | F | $CH(CH_3)-OH$ | 1,72 (d,3H), 3,11 (s,1H), 3,90 (s,3H), 5,29 (q,1H), 6,76 (t,1H), 7,41 (d,1H) |
| Ic.305 | $OCHF_2$ | F | $OCH(CH_3)-CO-OCH_3$ | 1,69 (d,3H), 3,80 (s,3H), 3,86 (s,3H), 5,63 (q,1H), 6,76 (t,1H), 7,25 (d,1H) |
| Ic.319 | $OCHF_2$ | F | $N(CH_3)-C_2H_5$ | 1,27 (t,3H), 3,19 (s,3H), 3,58 (q,2H), 3,87 (s,3H), 6,74 (t,1H), 7,20 (d,1H) |

| Nr. | $R^2$ | $R^4$ | $XR^6$ | Smp. / $^1H$-NMR [ppm] / MS [m/z] |
|---|---|---|---|---|
| Ic.324 | $OCHF_2$ | F | $N(CH_3)-CH_2-CH=CH_2$ | 3,15 (s,3H), 3,86 (s,3H), 4,14 (d,2H), 5,25 (d,1H), 5,26 (d,1H), 5,84 (m,1H), 6,75 (t,1H), 7,20 (d,1H) |
| Ic.325 | $OCHF_2$ | F | $N(CH_3)-CH_2-C{\equiv}CH$ | 113°C |
| Ic.327 | $OCHF_2$ | F | $N(CH_3)-CH_2-CO-OCH_3$ | 3,23 (s,3H), 3,78 (s,3H), 3,89 (s,3H), 4,41 (s,2H), 6,74 (t,1H), 7,21 (d,1H) |
| Ic.365 | $OCHF_2$ | F | $CH(COOC_2H_5)CO-OC(CH_3)_3$ | Sirup |
| If.001 | $CF_3$ | F | H | 4,13 (s,3H), 7,48 (d,1H), 9,07 (s,1H) |
| If.002 | $CF_3$ | F | $CH_3$ | 2,87 (s,3H), 4,11 (s,3H), 7,40 (d,1H) |
| If.038 | $CF_3$ | F | Br | 4,12 (s,3H), 7,44 (d,1H) |
| If.039 | $CF_3$ | F | $OCH_3$ | 399 $[M]^+$ |
| If.040 | $CF_3$ | F | $OC_2H_5$ | 1,48 (t,3H), 4,10 (s,3H), 4,67 (q,2H), 7,30 (d,1H) |
| If.051 | $CF_3$ | F | $OCH_2-C{\equiv}CH$ | 2,63 (t,1H), 4,09 (s,3H), 5,24 (d,2H), 7,30 (d,1H) |
| If.056 | $CF_3$ | F | $OCH_2-CO-OCH_3$ | 3,82 (s,3H), 4,10 (s,3H), 5,16 (s,2H), 7,31 (d,1H) |
| If.075 | $CF_3$ | F | $NH_2$ | 4,11 (s,3H), 7,51 (d,1H), 7,93 (s,2H) {in $(CD_3)_2SO$} |
| If.077 | $CF_3$ | F | $N(CH_3)_2$ | 3,21 (s,6H), 4,10 (s,3H), 7,22 (d,1H) |
| If.094 | $CF_3$ | F | $SCH_3$ | 2,81 (s,3H), 4,11 (s,3H), 7,35 (d,1H) |
| If.095 | $CF_3$ | F | $SC_2H_5$ | 1,51 (t,3H), 3,37 (q,2H), 4,10 (s,3H), 7,35 (d,1H) |
| If.218 | $CF_3$ | F | $CH=CH-CO-OCH_3$ | 3,86 (s,3H), 4,12 (s,3H), 6,68 (d,1H), 7,47 (d,1H), 7,91 (d,1H) |
| If.305 | $CF_3$ | F | $OCH(CH_3)-CO-OCH_3$ | 1,70 (d,3H), 3,80 (s,3H), 4,10 (s,3H), 5,63 (q,1H), 7,29 (d,1H) |
| If.319 | $CF_3$ | F | $N(CH_3)-CH_2-CH_3$ | 1,27 (t,3H), 3,19 (s,3H), 3,57 (q,2H), 4,10 (s,3H), 7,21 (d,1H) |
| If.324 | $CF_3$ | F | $N(CH_3)-CH_2-CH=CH_2$ | 3,17 (s,3H), 4,09 (s,3H), 4,15 (d,2H), 5,26 (d,1H), 5,28 (d,1H), 5,85 (m,1H), 7,22 (d,1H) |
| If.325 | $CF_3$ | F | $N(CH_3)-CH_2-C{\equiv}CH$ | 126°C |
| If.327 | $CF_3$ | F | $N(CH_3)-CH_2-CO-OCH_3$ | 3,22 (s,3H), 3,78 (s,3H), 4,10 (s,3H), 4,41 (s,2H), 7,22 (d,1H) |
| If.329 | $CF_3$ | F | $NH-CO-CH_3$ | 2,31 (s,3H), 4,12 (s,1H), 7,40 (d,1H), 9,25 (s,1H) |
| Ij.001 | $SO_2-CH_3$ | F | H | 379 $[M]^+$ |
| Ij.038 | $SO_2-CH_3$ | F | Br | 3,36 (s,3H), 4,30 (s,3H), 7,45 (d,1H) |
| Ij.039 | $SO_2-CH_3$ | F | $OCH_3$ | 3,35 (s,3H), 4,25 (s,3H), 4,28 (s,3H), 7,30 (d,1H) |
| Ij.040 | $SO_2-CH_3$ | F | $OC_2H_5$ | 1,48 (t,3H), 3,34 (s,3H), 4,26 (s,3H), 4,68 (q,2H), 7,29 (d,1H) |

| Nr. | $R^2$ | $R^4$ | $XR^6$ | Smp. / $^1H$-NMR [ppm] / MS [m/z] |
|---|---|---|---|---|
| Ij.051 | $SO_2-CH_3$ | F | $OCH_2-C\equiv CH$ | 2,67 (t,1H), 3,34 (s,3H), 4,27 (s,3H), 5,15 (d,2H), 7,31 (d,1H) |
| Ij.077 | $SO_2-CH_3$ | F | $N(CH_3)_2$ | 3,22 (s,6H), 3,35 (s,3H), 4,29 (s,3H), 7,22 (d,1H) |
| Ij.075 | $SO_2-CH_3$ | F | $NH_2$ | 3,54 (s,3H), 4,20 (s,3H), 7,51 (d,1H), 7,95 (s,2H) {in $(CD_3)_2SO$} |
| Ij.094 | $SO_2-CH_3$ | F | $SCH_3$ | 425 $[M]^+$ |
| Ij.095 | $SO_2-CH_3$ | F | $SC_2H_5$ | 1,51 (t,3H), 3,34 (s,3H), 3,38 (q,2H), 4,18 (s,3H), 7,35 (d,1H) |
| Ij.218 | $SO_2-CH_3$ | F | $CH=CH-CO-OCH_3$ | 3,36 (s,3H), 3,86 (s,3H), 4,31 (s,3H), 6,79 (d,1H), 7,47 (d,1H), 7,90 (d,1H) |
| Ij.329 | $SO_2-CH_3$ | F | $NH-CO-CH_3$ | 2,31 (s,3H), 3,36 (s,3H), 4,30 (s,3H),7,40 (d,1H), 9,71 (s,1H) |

Beispiel 24

4-Chlor-7-(4-chlor-1-methyl-5-trifluormethyl-1H-pyrazol-3-yl)-2-ethyl-6-fluorbenzoxazol (Nr. Iq.003)

Eine Lösung von 0,5 g (1,4 mmol) 2-Chlor-5-(4-chlor-1-methyl-5-trifluormethyl-1H-pyrazol-3-yl)-4-fluorphenylazid in 100 ml Propionsäure wurde 20 Std. auf Rückflußtemperatur erhitzt und dann eingeengt. Den Rückstand versetzte man mit 100 ml Ethylacetat. Die organische Phase wurde noch mit gesättigter wässriger Natriumhydrogencarbonat-Lösung gewaschen, dann über Magnesiumsulfat getrocknet und schließlich eingeengt. Die Reinigung des Rohproduktes erfolgte mittels Kieselgelchromatographie (Eluens: Hexan/Ethylacetat = 15:1). Ausbeute: 0,3 g;
$^1H$-NMR (400 MHz; in $CDCl_3$) : δ [ppm] = 1,44 (t,3H), 2,98. (q,2H), 4, 12 (s,3H), 7,25 (d, 1H).

Vorstufe: 2-Chlor-5-(4-chlor-1-methyl-5-trifluormethyl-1Hpyrazol-3-yl)-4-fluorphenylazid

Zu einer Lösung von 1 g (3 mmol) 2-Chlor-5-(4-chlor-1-methyl-5-trifluormethyl-1H-pyrazol-3-yl)-4-fluoranilin in 20 ml Trifluoressigsäure wurden 0,46 g (4,5 mmol) tert.-Butylnitrit und 0,3 g (4,5 mmol) Natriumazid gegeben. Anschließend rührte man 2 Std., wonach die Mischung mit 50 ml Wasser versetzt wurde. Aus der wäßrige Phase extrahierte man das entstandene Produkt mit 100 ml Methyl-tert.-butylether. Das Extrakt wurde noch mit 10 %iger Natronlauge gewaschen, dann über Magnesiumsulfat getrocknet und schließlich eingeengt. Die Reinigung des Rohproduktes erfolgte mittels Kieselgelchromatographie (Eluens: Hexan/Ethylacetat = 1:1). Ausbeute: 0,7 g;
$^1H$-NMR (400 MHz; in $(CD_3)_2SO$) : δ (ppmj = 4,10 (s,3H), 7,53 (d, 1H), 7,77 (d, 1H).

Anwendungsbeispiele (herbizide Wirksamkeit)

Die herbizide Wirkung der substituierten Pyrazol-3-ylbenzazole I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 31,2 oder 15,6 g/ha a.S. (aktive Substanz).

Die Pflanzen wurden artenspezifisch bei Temperaturen von 10 - 25°C bzw. 20 - 35°C gehalten. Die versuchsperiode erstreckte sich über 2 bis 4 Wochen. Während dieser Zeit wurden die Pflanzen gepflegt, und ihre Reaktion

auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Amaranthus retroflexus | Zurückgekrümmter Fuchsschwanz | redroot pigweed pigweed |
| Polygonum persicaria | Flohknöterich | ladysthumb |
| Solanum nigrum | Schwarzer Nachtschatten | black nightshade |
| Veronica subspecies | Ehrenpreisarten | speedwell |

Bei Aufwandmengen von 31,2 und 15,6 g/ha a.S. zeigte die Verbindung Nr. Ic.094 im Nachauflaufverfahren eine sehr gute herbizide Wirkung gegen die o.g. breitblättrigen Pflanzen.

Anwendungsbeispiele (desikkant/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der wirkstoffe (unter Zusatz von 0,15 Gew.-% des Fettalkoholalkoxylats Plurafac® LF 700[1]), bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

**1.** Substituierte Pyrazol-3-ylbenzazole der Formel I

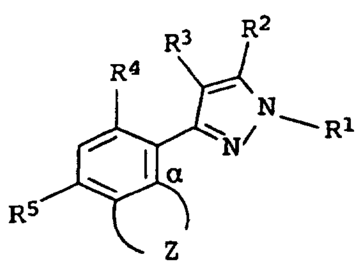

I,

in der die Variablen folgende Bedeutungen haben:

$R^1$ Wasserstoff, $C_1$-$C_4$-Alkyl oder $C_1$-$C_4$-Halogenalkyl;

$R^2$ $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl;

$R^3$ Wasserstoff, Halogen, Cyano, Nitro, $C_1$-$C_4$-Alkyl oder $C_1$-$C_4$-Halogenalkyl;

$R^4$ Wasserstoff oder Halogen;

$R^5$ Halogen, Cyano oder Trifluormethyl;

Z eine Gruppe -N=C($XR^6$)-O- oder -N=C($XR^6$)-S-, die über den Stickstoff, Sauerstoff oder Schwefel an α

gebunden sein kann;

X eine chemische Bindung, Sauerstoff, Schwefel, -S(O)-, $-SO_2-$, -NH- oder $-N(R^7)-$;

$R^6$, $R^7$ unabhängig voneinander $C_1$-$C_6$-Alkyl, Cyano-$C_1$-$C_4$-alkyl, Hydroxy-$C_1$-$C_4$-alkyl, $C_3$-$C_6$-Alkenyl, Cyano-$C_3$-$C_6$-alkenyl, $C_3$-$C_6$-Halogenalkenyl, $C_3$-$C_6$-Alkinyl, Cyano-$C_3$-$C_6$-alkinyl, $C_3$-$C_6$-Halogenalkinyl, $C_1$-$C_4$-Alkoxy-$C_1$-$C_4$-alkyl, $C_1$-$C_4$-Halogenalkoxy-$C_1$-$C_4$-alkyl, $C_3$-$C_4$-Alkenyloxy-$C_1$-$C_4$-alkyl, $C_3$-$C_4$-Alkinyloxy-$C_1$-$C_4$-alkyl, $C_3$-$C_8$-Cycloalkyloxy-$C_1$-$C_4$-alkyl, Amino-$C_1$-$C_4$-alkyl, $C_1$-$C_4$-Alkylamino-$C_1$-$C_4$-alkyl, Di($C_1$-$C_4$-alkyl)amino-$C_1$-$C_4$-alkyl, $C_1$-$C_4$-Alkylthio-$C_1$-$C_4$-alkyl, $C_1$-$C_4$-Halogen-alkylthio-$C_1$-$C_9$-alkyl, $C_3$-$C_4$-Alkenylthio-$C_1$-$C_4$-alkyl, $C_3$-$C_4$-Alkinylthio-$C_1$-$C_4$-alkyl, $C_1$-$C_4$-Alkylsulfinyl-$C_1$-$C_4$-alkyl, $C_1$-$C_4$-Halogenalkylsulfinyl-$C_1$-$C_4$-alkyl, $C_3$-$C_4$-Alkenylsulfinyl-$C_1$-$C_4$-alkyl, $C_3$-$C_4$-Alkinylsulfinyl-$C_1$-$C_4$-alkyl, $C_1$-$C_4$-Alkylsulfonyl-$C_1$-$C_4$-alkyl, $C_1$-$C_4$-Halogenalkylsulfonyl-$C_1$-$C_4$-alkyl, $C_3$-$C_4$-Alkenylsulfonyl-$C_1$-$C_4$-alkyl, $C_3$-$C_4$-Alkinylsulfonyl-$C_1$-$C_4$-alkyl, Hydroxycarbonyl-$C_1$-$C_4$-alkyl, ($C_1$-$C_9$-Alkoxy)-carbonyl-$C_1$-$C_4$-alkyl, das eine Cyano- oder ($C_1$-$C_4$-Alkoxy)carbonylgruppe tragen kann,
($C_1$-$C_4$-Alkylthio) carbonyl-$C_1$-$C_4$-alkyl, Aminocarbonyl-$C_1$-$C_4$-alkyl, $C_1$-$C_4$-Alkylaminocarbonyl-$C_1$-$C_4$-alkyl, Di($C_1$-$C_4$-alkyl)aminocarbonyl-$C_1$-$C_4$-alkyl, Di($C_1$-$C_4$-alkyl)-phosphonyl-$C_1$-$C_4$-alkyl, ($C_1$-$C_4$-Alkoxy)imino-$C_1$-$C_4$-alkyl, ($C_3$-$C_4$-Alkenyloxy)imino-$C_1$-$C_4$-alkyl,
$C_3$-$C_8$-Cycloalkyl, $C_3$-$C_8$-Cycloalkyl-$C_1$-$C_4$-alkyl, Phenyl, Phenyl-$C_1$-$C_4$-alkyl, 3- bis 7-gliedriges Heterocyclyl oder Heterocyclyl-$C_1$-$C_4$-alkyl, wobei jeder Cycloalkyl- und jeder Heterocyclyl-Ring ein Carbonyl- oder Thiocarbonyl-Ringglied enthalten kann,
und wobei jeder Cycloalkyl-, Phenyl- und Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl-sulfonyl, $C_1$-$C_4$-Halogenalkylsulfonyl, ($C_1$-$C_4$-Alkoxy)-carbonyl, ($C_1$-$C_4$-Alkyl)carbonyl, ($C_1$-$C_4$-Halogenalkyl)-carbonyl, ($C_1$-$C_4$-Alkyl)carbonyloxy, ($C_1$-$C_4$-Halogenalkyl)-carbonyloxy und Di($C_1$-$C_4$-alkyl)amino;

sofern X eine chemische Bindung, Sauerstoff, Schwefel, -NH- oder $-N(R^7)-$ ist, kann $R^6$ auch ($C_1$-$C_4$-Alkyl)carbonyl, ($C_1$-$C_4$-Halogenalkyl)carbonyl, ($C_1$-$C_4$-Alkoxy)carbonyl, $C_1$-$C_4$-Alkylsulfonyl oder $C_1$-$C_4$-Halogenalkylsulfonyl bedeuten;
sofern X eine chemische Bindung ist, kann $R^6$ außerdem Wasserstoff, Cyano, Mercapto, Amino, Halogen, $-CH_2$-CH(Halogen)-$R^8$, -CH=CH-$R^8$ oder -CH=C(Halogen)-$R^3$ bedeuten, wobei $R^8$ für Hydroxycarbonyl, ($C_1$-$C_4$-Alkoxy)carbonyl, ($C_1$-$C_4$-Alkylthio)carbonyl, Aminocarbonyl, $C_1$-$C_4$-Alkylaminocarbonyl, Di($C_1$-$C_4$-alkyl)aminocarbonyl oder Di($C_1$-$C_4$-alkyl)phosphonyl steht;
oder $R^6$ und $R^7$ stehen zusammen für eine 1,3-Propylen-, Tetramethylen-, Pentamethylen- oder Ethylenoxyethylen-Kette, die jeweils unsubstituiert sein oder ein bis vier $C_1$-$C_4$-Alkylgruppen oder ein oder zwei ($C_1$-$C_4$-Alkoxy)carbonylgruppen tragen kann,
sowie die landwirtschaftlich brauchbaren Salze dieser Verbindungen I.

**2.** Verwendung von substituierten Pyrazol-3-ylbenzazolen und deren landwirtschaftlich brauchbaren Salzen, gemäß Anspruch 1, als Herbizide oder zur Desikkation/Defoliation von Pflanzen.

**3.** Herbizides Mittel, enthaltend eine herbizid wirksame Menge mindestens eines substituierten Pyrazol-3-ylbenzazols der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

**4.** Mittel zur Desikkation und/oder Defoliation von Pflanzen, enthaltend eine desikkant und/oder defoliant wirksame Menge mindestens eines substituierten Pyrazol-3-ylbenzazols der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

**5.** Verfahren zur Herstellung von herbizid wirksamen Mitteln,
**dadurch gekennzeichnet, daß** man eine herbizid wirksame Menge mindestens eines substituierten Pyrazol-3-ylbenzazols der Formel I oder eines landwirtschaftlich brauchbaren Salzes von I, gemäß Anspruch 1, mit mindestens einem inerten flüssigen und/oder festen Trägerstoff sowie gewünschtenfalls mindestens einem oberflächenaktiven Stoff mischt.

**6.** Verfahren zur Herstellung von desikkant und/oder defoliant wirksamen Mitteln, **dadurch gekennzeichnet, daß** man eine desikkant/defoliant wirksame Menge mindestens eines substituierten Pyrazol-3-ylbenzazols der Formel I oder eines landwirtschaftlich brauchbaren Salzes von I, gemäß Anspruch 1, mit mindestens einem inerten flüssigen und/oder festen Trägerstoff sowie gewünschtenfalls mindestens einem oberflächenaktiven Stoff mischt.

**7.** Verfahren zur Bekämpfung von unerwünschtem Pflanzenwuchs,
**dadurch gekennzeichnet, daß** man eine herbizid wirksame Menge mindestens eines substituierten Pyrazol-3-ylbenzazols der Formel I oder eines landwirtschaftlich brauchbaren Salzes von I, gemäß Anspruch 1, auf Pflanzen, deren Lebensraum oder auf Saatgut einwirken läßt.

**8.** Verfahren zur Desikkation und/oder Defoliation von Pflanzen,
**dadurch gekennzeichnet, daß** man eine desikkant und/oder defoliant wirksame Menge mindestens eines substituierten Pyrazol-3-ylbenzazols der Formel I oder eines landwirtschaftlich brauchbaren Salzes von I, gemäß Anspruch 1, auf Pflanzen einwirken läßt.

**9.** Verfahren nach Anspruch 8, **dadurch gekennzeichnet, daß** man Baumwolle behandelt.

**10.** Verfahren zur Herstellung von substituierten Pyrazol-3-ylbenzazolen der Formel I gemäß Anspruch 1, wobei Z für $-N=C(NH_2)-S-$ steht, **dadurch gekennzeichnet, daß** man ein Aminophenylpyrazol der Formel IIIa oder IIIb

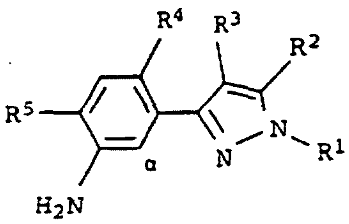

IIIa

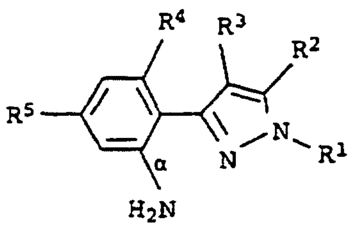

IIIb

entweder mit einem Halogen und Ammoniumthiocyanat oder einem Alkali- oder Erdalkalimetallthiocyanat, oder zunächst mit Ammoniumthiocyanat oder einem Alkali- oder Erdalkalimetallthiocyanat und danach mit einem Halogen, umsetzt.

**11.** Verfahren zur Herstellung von substituierten Pyrazol-3-ylbenzazolen der Formel I, bei denen $XR^6$ für Halogen oder Cyano steht, **dadurch gekennzeichnet, daß** man ein substituiertes Pyrazol-3-ylbenzazol I, bei dem $XR^6$ für Amino steht, diazotiert und das erhaltene Diazoniumsalz anschließend mit Kupfer (I) halogenid oder Kupfer (I) cyanid umsetzt.

**12.** Verfahren zur Herstellung von substituierten Pyrazol-3-ylbenzazolen der Formel I, bei denen X für Schwefel steht, **dadurch gekennzeichnet, daß** man ein substituiertes Pyrazol-3-ylbenzazol I, bei dem $XR^6$ für Amino steht, diazotiert und das erhaltene Diazoniumsalz anschließend mit einem Dialkyldisulfid der Formel $R^6S-SR^6$ umsetzt.

**13.** Verfahren zur Herstellung von substituierten Pyrazol-3-ylbenzazolen der Formel I, bei denen $XR^6$ für $-CH_2-CH(Cl)-R^8$, $-CH_2-CH(Br)-R^8$, $-CH=CH-R^8$, $-CH=C(Cl)-R^8$ oder $-CH=C(Br)-R^8$ steht, **dadurch gekennzeichnet, daß** man ein substituiertes Pyrazol-3-ylbenzazol I, bei dem $XR^6$ für Amino steht, diazotiert und das erhaltene Diazoniumsalz anschließend mit einem Alken der Formel $H_2C=CH-R^8$ oder einem Alkin der Formel $HC\equiv C-R^8$ und Kupfer(I)chlorid, Kupfer(I)bromid, Kupfer(II)chlorid oder Kupfer(II)bromid umsetzt.

**14.** Verfahren zur Herstellung von substituierten Pyrazol-3-ylbenzazolen der Formel I, bei denen X für Sauerstoff, Schwefel, -NH- oder $-N(R^7)-$ steht, oder bei denen $XR^6$ $CH(R^b)R^c$ bedeutet, wobei $R^b$ und $R^c$ unabhängig voneinander für Cyano oder ($C_1$-$C_4$-Alkoxy)carbonyl stehen, **dadurch gekennzeichnet, daß** man ein substituiertes Pyrazol-3-ylbenzazol I, bei dem $XR^6$ für Chlor, Brom, $-SO_2$-Alkyl oder $-SO_2$-Halogenalkyl steht, in Gegenwart einer Base mit einem Nukleophil $HOR^6$, $HSR^6$, $H_2NR^6$, $HN(R^6)R^7$ oder $H_2C(R^b)R^c$ umsetzt.

**15.** Verfahren zur Herstellung von substituierten Pyrazol-3-ylbenzazolen der Formel I, bei denen $XR^6$ für $C_1$-$C_6$-Alkyl steht, **dadurch gekennzeichnet, daß** man ein substituiertes Pyrazol-3-ylbenzazol I, bei dem $XR^6$ für Halogen

steht, gewünschtenfalls in Gegenwart eines Katalysators, mit einer ($C_1$-$C_6$-Alkyl)-Grignard-Verbindung umsetzt.

**16.** Verfahren zur Herstellung von substituierten Pyrazol-3-ylbenzazolen der Formel I gemäß Anspruch 1, wobei Z für -N=C($R^6$)-O- steht, **dadurch gekennzeichnet, daß** man ein Aminophenylpyrazol der Formel IIIa oder IIIb

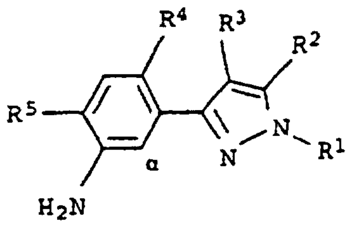

IIIa

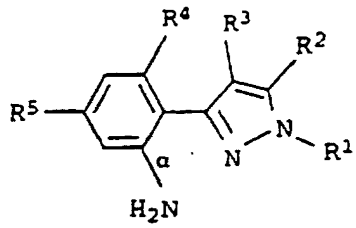

IIIb

diazotiert, das gebildete Diazoniumsalz mit einem Alkalimetallazid zu einem Azidophenylpyrazol der Formel Va oder Vb

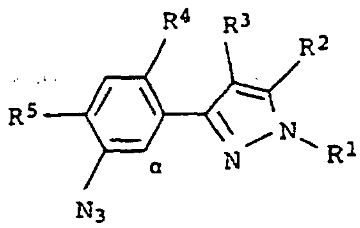

Va

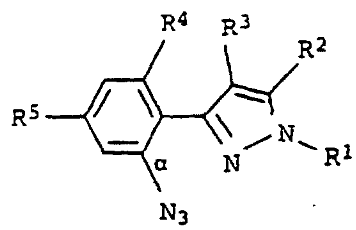

Vb

umsetzt und dieses schließlich mit einer Carbonsäure der Formel $R^6$-COOH reagieren läßt.

## Claims

**1.** A substituted pyrazol-3-ylbenzazole of the formula I

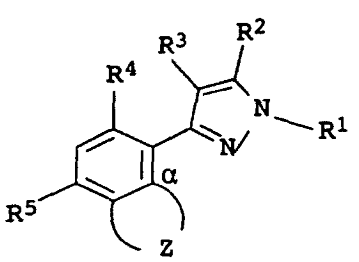

I,

where:

| | |
|---|---|
| $R^1$ | is hydrogen, $C_1$-$C_4$-alkyl or $C_1$-$C_4$-haloalkyl; |
| $R^2$ | is $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfinyl, $C_1$-$C_4$-haloalkylsulfinyl, $C_1$-$C_4$-alkylsulfonyl or $C_1$-$C_4$-haloalkylsulfonyl; |
| $R^3$ | is hydrogen, halogen, cyano, nitro, $C_1$-$C_4$-alkyl or $C_1$-$C_4$-haloalkyl; |
| $R^4$ | is hydrogen or halogen; |

$R^5$ is halogen, cyano or trifluoromethyl;

Z is a group -N=C($XR^6$)-O- or -N=C($XR^6$)-S- which may be bonded to $\alpha$ via the nitrogen, oxygen or sulfur;

X is a chemical bond, oxygen, sulfur, -S(O)-, -$SO_2$-, -NH-or -N($R^7$)-;

$R^6$ and $R^7$ independently of one another are each $C_1$-$C_6$-alkyl, cyano-$C_1$-$C_4$-alkyl, hydroxy-$C_1$-$C_4$-alkyl, $C_3$-$C_6$-alkenyl, cyano-$C_3$-$C_6$-alkenyl, $C_3$-$C_6$-haloalkenyl, $C_3$-$C_6$-alkynyl, cyano-$C_3$-$C_6$-alkynyl, $C_3$-$C_6$-haloalkynyl, $C_1$-$C_4$-alkoxy-$C_1$-$C_4$-alkyl, $C_1$-$C_4$-haloalkoxy-$C_1$-$C_4$-alkyl, $C_3$-$C_4$-alkenyloxy-$C_1$-$C_4$-alkyl, $C_3$-$C_4$-alkynyloxy-$C_1$-$C_4$-alkyl, $C_3$-$C_8$-cycloalkyloxy-$C_1$-$C_4$-alkyl, amino-$C_1$-$C_4$-alkyl, $C_1$-$C_4$-alkylamino-$C_1$-$C_4$-alkyl, di($C_1$-$C_4$-alkyl)amino-$C_1$-$C_4$-alkyl, $C_1$-$C_4$-alkylthio-$C_1$-$C_4$-alkyl, $C_1$-$C_4$-haloalkylthio-$C_1$-$C_4$-alkyl, $C_3$-$C_4$-alkenylthio-$C_1$-$C_4$-alkyl, $C_3$-$C_4$-alkynylthio-$C_1$-$C_4$-alkyl, $C_1$-$C_4$-alkylsulfinyl-$C_1$-$C_4$-alkyl, $C_1$-$C_4$-haloalkylsulfinyl-$C_1$-$C_4$-alkyl, $C_3$-$C_4$-alkenyl-sulfinyl-$C_1$-$C_4$-alkyl, $C_3$-$C_4$-alkynylsulfinyl-$C_1$-$C_4$-alkyl, $C_1$-$C_4$-alkylsulfonyl-$C_1$-$C_4$-alkyl, $C_1$-$C_4$-haloalkyl-sulfonyl-$C_1$-$C_4$-alkyl, $C_3$-$C_4$-alkenylsulfonyl-$C_1$-$C_4$-alkyl, $C_3$-$C_4$-alkynylsulfonyl-$C_1$-$C_4$-alkyl, hydroxycarbonyl-$C_1$-$C_4$-alkyl, ($C_1$-$C_4$-alkoxy)carbonyl-$C_1$-$C_4$-alkyl which may carry a cyano or ($C_1$-$C_4$-alkoxy)carbonyl group, ($C_1$-$C_4$-alkylthio)carbonyl-$C_1$-$C_4$-alkyl, aminocarbonyl-$C_1$-$C_4$-alkyl, $C_1$-$C_4$-alkylamino-carbonyl-$C_1$-$C_4$-alkyl, di($C_1$-$C_4$-alkyl)aminocarbonyl-$C_1$-$C_4$-alkyl, di($C_1$-$C_4$-alkyl)phosphonyl-$C_1$-$C_4$-alkyl, ($C_1$-$C_4$-alkoxy)imino-$C_1$-$C_4$-alkyl, ($C_3$-$C_4$-alkenyloxy)imino-$C_1$-$C_4$-alkyl, $C_3$-$C_8$-cycloalkyl, $C_3$-$C_8$-cycloalkyl-$C_1$-$C_4$-alkyl, phenyl, phenyl-$C_1$-$C_4$-alkyl, 3- to 7-membered heterocyclyl or heterocyclyl-$C_1$-$C_4$-alkyl, where each cycloalkyl and each heterocyclyl ring may contain a carbonyl or thiocarbonyl ring member,

and where each cycloalkyl, phenyl and heterocyclyl ring may be unsubstituted or carry one to four substituents selected in each case from the group consisting of cyano, nitro, amino, hydroxyl, carboxyl, halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfonyl, $C_1$-$C_4$-haloalkylsulfonyl, ($C_1$-$C_4$-alkoxy)carbonyl, ($C_1$-$C_4$-alkyl)carbonyl, ($C_1$-$C_4$-haloalkyl)carbonyl, ($C_1$-$C_4$-alkyl)carbonyloxy, ($C_1$-$C_4$-haloalkyl)carbonyloxy and di($C_1$-$C_4$-alkyl)amino; if X is a chemical bond, oxygen, sulfur, -NH- or -N ($R^7$)-, $R^6$ may also be ($C_1$-$C_4$-alkyl)carbonyl, ($C_1$-$C_4$-haloalkyl)carbonyl, ($C_1$-$C_4$-alkoxy)carbonyl, $C_1$-$C_4$-alkylsulfonyl or $C_1$-$C_4$-haloalkylsulfonyl;
if X is a chemical bond, $R^6$ may furthermore be hydrogen, cyano, mercapto, amino, halogen, -$CH_2$-CH (halogen) -$R^8$, -CH=CH-$R^8$ or -CH=C(halogen)-$R^8$, where $R^8$ is hydroxycarbonyl, ($C_1$-$C_4$-alkoxy)carbonyl, ($C_1$-$C_4$-alkylthio) carbonyl, aminocarbonyl, $C_1$-$C_4$-alkylaminocarbonyl, di($C_1$-$C_4$-alkyl)aminocarbonyl or di($C_1$-$C_4$-alkyl)phosphonyl;
or $R^6$ and $R^7$ together are a 1,3-propylene, tetramethylene, pentamethylene or ethyleneoxyethylene chain which may in each case be unsubstituted or carry one to four $C_1$-$C_4$-alkyl groups or one or two ($C_1$-$C_4$-alkoxy)carbonyl groups,
and the agriculturally useful salts of these compounds I.

**2.** The use of a substituted pyrazol-3-ylbenzazole and agriculturally useful salts thereof, as claimed in claim 1, as herbicides or for the desiccation/defoliation of plants.

**3.** A herbicidal composition comprising a herbicidally active amount of at least one substituted pyrazol-3-ylbenzazole of the formula I or an agriculturally useful salt of I, as claimed in claim 1, and at least one inert liquid and/or solid carrier and, if desired, at least one surfactant.

**4.** A composition for the desiccation and/or defoliation of plants, comprising an amount of at least one substituted pyrazol-3-ylbenzazole of the formula I or an agriculturally useful salt of I, as claimed in claim 1, which acts as a desiccant and/or defoliant, and at least one inert liquid and/or solid carrier and, if desired, at least one surfactant.

**5.** A process for preparing herbicidally active compositions, which comprises mixing a herbicidally active amount of at least one substituted pyrazol-3-ylbenzazole of the formula I or an agriculturally useful salt of I, as claimed in claim 1, with at least one inert liquid and/or solid carrier and, if desired, at least one surfactant.

**6.** A process for preparing compositions which act as desiccants and/or defoliants, which comprises mixing an amount of at least one substituted pyrazol-3-ylbenzazole of the formula I or an agriculturally useful salt of I, as claimed in claim 1, which acts as a desiccant and/or defoliant, with at least one inert liquid and/or solid carrier and, if desired, at least one surfactant.

7. A method for controlling undesirable vegetation, which comprises allowing a herbicidally active amount of at least one substituted pyrazol-3-ylbenzazole of the formula I or of an agriculturally useful salt of I, as claimed in claim 1, to act on plants, their habitat or on seeds.

8. A method for the desiccation and/or defoliation of plants; which comprises allowing an amount of at least one substituted pyrazol-3-ylbenzazole of the formula I or of an agriculturally useful salt of I, as claimed in claim 1, which acts as a desiccant and/or defoliant, to act on plants.

9. A method as claimed in claim 8, wherein cotton is treated.

10. A process for preparing a substituted pyrazol-3-ylbenzazole of the formula I as claimed in claim 1 where Z is -N=C($NH_2$)-S-, which comprises reacting an aminophenylpyrazole of the formula IIIa or IIIb

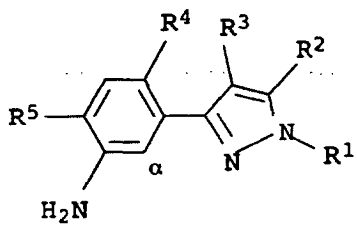

IIIa

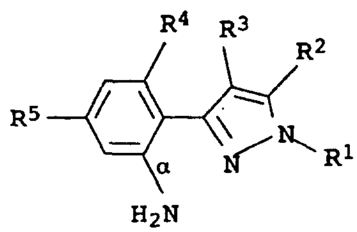

IIIb

either with a halogen and ammonium thiocyanate or an alkali metal thiocyanate or alkaline earth metal thiocyanate, or initially with ammonium thiocyanate or an alkali metal thiocyanate or alkaline earth metal thiocyanate and then with a halogen.

11. A process for preparing a substituted pyrazol-3-ylbenzazole of the formula I where $XR^6$ is halogen or cyano, which comprises diazotizing a substituted pyrazol-3-ylbenzazole I where $XR^6$ is amino and subsequently reacting the resulting diazonium salt with copper(I) halide or copper(I) cyanide.

12. A process for preparing a substituted pyrazol-3-ylbenzazole of the formula I where X is sulfur, which comprises diazotizing a substituted pyrazol-3-ylbenzazole I where $XR^6$ is amino and subsequently reacting the resulting diazonium salt with a dialkyl disulfide of the formula $R^6S\text{-}SR^6$.

13. A process for preparing a substituted pyrazol-3-ylbenzazole of the formula I where $XR^6$ is $-CH_2\text{-}CH(Cl)\text{-}R^8$, $-CH_2\text{-}CH(Br)\text{-}R^8$, $-CH{=}CH\text{-}R^8$, $-CH{=}C(Cl)\text{-}R^8$ or $-CH{=}C(Br)\text{-}R^8$, which comprises diazotizing a substituted pyrazol-3-ylbenzazole I where $XR^6$ is amino and subsequently reacting the resulting diazonium salt with an alkene of the formula $H_2C{=}CH\text{-}R^8$ or an alkyne of the formula $HC{\equiv}C\text{-}R^8$ and copper(I) chloride, copper(I) bromide, copper(II) chloride or copper(II) bromide.

14. A process for preparing a substituted pyrazol-3-ylbenzazole of the formula I where X is oxygen, sulfur, -NH- or -N($R^7$)-or where $XR^6$ is $CH(R^b)R^c$ where $R^b$ and $R^c$ independently of one another are each cyano or ($C_1$-$C_4$-alkoxy)carbonyl, which comprises reacting a substituted pyrazol-3-ylbenzazole I where $XR^6$ is chlorine, bromine, $-SO_2$-alkyl or $-SO_2$-haloalkyl in the presence of a base with a nucleophile $HOR^6$, $HSR^6$, $H_2NR^6$, $HN(R^6)R^7$ or $H_2C(R^b)R^c$.

15. A process for preparing a substituted pyrazol-3-ylbenzazole of the formula I where $XR^6$ is $C_1$-$C_6$-alkyl, which comprises reacting a substituted pyrazol-3-ylbenzazole I where $XR^6$ is halogen, if appropriate in the presence of a catalyst, with a ($C_1$-$C_6$-alkyl)-Grignard reagent.

16. A process for preparing a substituted pyrazol-3-ylbenzazole of the formula I as claimed in claim 1 where Z is -N=C($R^6$)-O-, which comprises diazatizing an aminophenylpyrazole of the formula IIIa or IIIb

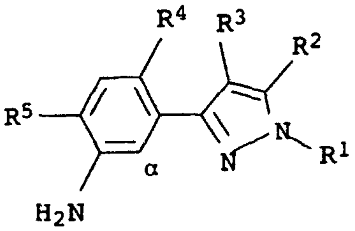

IIIa

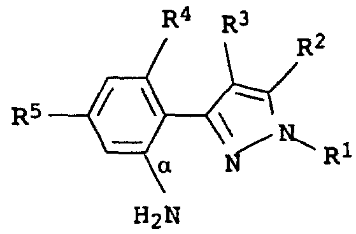

IIIb

,

reacting the resulting diazonium salt with an alkali metal azide to give an azidophenylpyrazole of the formula Va or Vb

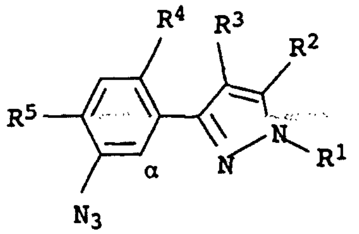

Va

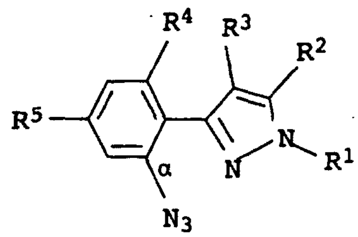

Vb

and finally reacting this with a carboxylic acid of formula $R^6$-COOH.

## Revendications

**1.** Pyrazol-3-ylbenzazoles substitués de formule I

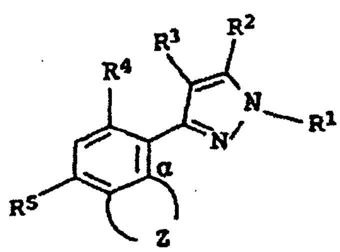

I,

dans laquelle les variables ont les significations suivantes :

$R^1$ représente un hydrogène, un alkyle en $C_1$ à $C_4$ ou un halogénalkyle en $C_1$ à $C_4$;
$R^2$ représente un alkyle en $C_1$ à $C_4$, un halogénalkyle en $C_1$ à $C_4$, un alcoxy en $C_1$ à $C_4$, un halogénalcoxy en $C_1$ à $C_4$, un alkylthio en $C_1$ à $C_4$, un halogénalkylthio en $C_1$ à $C_4$, un (alkyle en $C_1$ à $C_4$)sulfinyle, un (halogénalkyle en $C_1$ à $C_4$)sulfinyle, un (alkyle en $C_1$ à $C_4$)sulfonyle ou un (halogénalkyle en $C_1$ à $C_4$)sulfonyle;
$R^3$ représente un hydrogène, un halogène, un cyano, un nitro, un alkyle en $C_1$ à $C_4$ ou un halogénalkyle en $C_1$ à $C_4$;
$R^4$ représente un hydrogène ou un halogène ;
$R^5$ représente un halogène, un cyano ou un trifluorométhyle ;
Z représente un groupe -N=C($XR^6$)-O- ou -N=C($XR^6$)-S-, qui peut être lié à $\alpha$ par l'intermédiaire de l'azote, de l'oxygène ou du soufre ;
X représente une liaison chimique, un oxygène, un soufre, -S(O)-, $-SO_2-$,-NH- ou -N($R^7$)- ;

$R^6$, $R^7$ représentent, indépendamment l'un de l'autre alkyle en $C_1$ à $C_6$, cyano(alkyle en $C_1$ à $C_4$), hydroxy (alkyle en $C_1$ à $C_4$), alcényle en $C_3$ à $C_6$, cyano(alcényle en $C_3$ à $C_6$), halogénalcényle en $C_3$ à $C_6$, alcynyle en $C_3$ à $C_6$, cyano(alcynyle en $C_3$ à $C_6$), halogénalcynyle en $C_3$ à $C_6$, (alcoxy en $C_1$ à $C_4$)-(alkyle en $C_1$ à $C_4$), (halogénalcoxy en $C_1$ à $C_4$)-(alkyle en $C_1$ à $C_4$), (alcényloxy en $C_3$ à $C_4$)-(alkyle en $C_1$ à $C_4$), (alcynyloxy en $C_3$ à $C_4$)-(alkyle en $C_1$ à $C_4$), (cycloalkyloxy en $C_3$ à $C_8$)-(alkyle en $C_1$ à $C_4$), amino-(alkyle en $C_1$ à $C_4$), (alkylamino en $C_1$ à $C_4$)-(alkyle en $C_1$ à $C_4$), di-(alkyle en $C_1$ à $C_4$)-amino-(alkyle en $C_1$ à $C_4$), (alkylthio en $C_1$ à $C_4$)-(alkyle en $C_1$ à $C_4$), (halogénalkylthio en $C_1$ à $C_4$)-(alkyle en $C_1$ à $C_4$), (alcénylthio en $C_3$ à $C_4$)-(alkyle en $C_1$ à $C_4$), (alcynylthio en $C_3$ à $C_4$)-(alkyle en $C_1$ à $C_4$), (alkyle en Ci à $C_4$)-sulfinyl-(alkyle en $C_1$ à $C_4$), (halogénalkyle en $C_1$ à $C_4$)-sulfinyl-(alkyle en $C_1$ à $C_4$), (alcényle en $C_3$ à $C_4$)-sulfinyl-(alkyle en $C_1$ à $C_4$), (alcynyle en $C_3$ à $C_4$)-sulfinyl-(alkyle en $C_1$ à $C_4$), (alkyle en $C_1$ à $C_4$)-sulfonyl-(alkyle en $C_1$ à $C_4$), (halogénalkyle en $C_1$ à $C_4$)-sulfonyl-(alkyle en $C_1$ à $C_4$), (alcényle en $C_3$ à $C_4$)-sulfonyl-(alkyle en $C_1$ à $C_4$), (alcynyle en $C_3$ à $C_4$)-sulfonyl-(alkyle en $C_1$ à $C_4$), hydroxycarbonyl-(alkyle en $C_1$ à $C_4$), (alcoxy en $C_1$ à $C_4$)-carbonyl-(alkyle en $C_1$ à $C_4$), qui peut porter un groupe cyano ou (alcoxy en $C_1$ à $C_4$)carbonyle, (alkylthio en $C_1$ à $C_4$)carbonyl-(alkyle en $C_1$ à $C_4$), aminocarbonyl-(alkyle en $C_1$ à $C_4$), (alkyle en $C_1$ à $C_4$)-aminocarbonyl-(alkyle en $C_1$ à $C_4$), di (alkyle en $C_1$ à $C_4$)-aminocarbonyl-(alkyle en $C_1$ à $C_4$), di(alkyle en $C_1$ à $C_4$)-phosphonyl-(alkyle en $C_1$ à $C_4$), (alcoxy en $C_1$ à $C_4$)-imino-(alkyle en $C_1$ à $C_4$), (alcényloxy en $C_3$ à $C_4$)-imino-(alkyle en $C_1$ à $C_4$), cycloalkyle en $C_3$ à $C_8$, (cycloalkyle en $C_3$ à $C_8$)-(alkyle en $C_1$ à $C_4$), phényle, phényl-(alkyle en $C_1$ à $C_4$), hétérocyclyle à 3 à 7 chaînons ou hétérocyclyl-(alkyle en $C_1$ à $C_4$), où chaque noyau cycloalkyle et chaque noyau hétérocyclyle peut contenir un chaînon carbonyle ou thiocarbonyle, et où chaque noyau cycloalkyle, phényle et hétérocyclyle peut être non substitué ou porter de un à quatre substituants, à chaque fois choisis dans le groupe constitué par cyano, nitro, amino, hydroxy, carboxy, halogène, alkyle en $C_1$ à $C_4$, halogénalkyle en $C_1$ à $C_4$, alcoxy en $C_1$ à $C_4$, halogénalcoxy en $C_1$ à $C_4$, alkylthio en $C_1$ à $C_4$, halogénalkylthio en $C_1$ à $C_4$, (alkyle en $C_1$ à $C_4$) sulfonyle, (halogénalkyle en $C_1$ à $C_4$)sulfonyle, (alcoxy en $C_1$ à $C_4$)carbonyle, (alkyle en $C_1$ à $C_4$)carbonyle, (halogénalkyle en $C_1$ à $C_4$)carbonyle, (alkyle en $C_1$ à $C_4$)carbonyloxy, (halogénalkyle en $C_1$ à $C_4$)carbonyloxy et di(alkyle en $C_1$ à $C_4$)amino ; dans la mesure où X est une liaison chimique, un oxygène, un soufre, -NH-ou -N($R^7$)-, $R^6$ peut également représenter un (alkyle en $C_1$ à $C_4$)carbonyle, (halogénalkyle en $C_1$ à $C_4$)carbonyle, un (alcoxy en $C_1$ à $C_4$)carbonyle, un (alkyle en $C_1$ à $C_4$)sulfonyle ou un (halogénalkyle en $C_1$ à $C_4$)sulfonyle ; dans la mesure où X est une liaison chimique, $R^6$ peut en outre représenter un hydrogène, un cyano, un mercapto, un amino, un halogène, -$CH_2$-CH(halogène)-$R^8$, -CH=CH-$R^8$ ou -CH=C(halogène)-$R^8$, où $R^8$ représente un hydroxycarbonyle, (alcoxy en $C_1$ à $C_4$)carbonyle, (alkylthio en $C_1$ à $C_4$)carbonyle, aminocarbonyle, (alkyle en $C_1$ à $C_4$)aminocarbonyle, di(alkyle en $C_1$ à $C_4$)aminocarbonyle ou di(alkyle en $C_1$ à $C_4$)phosphonyle ; ou $R^6$ et $R^7$ représentent ensemble une chaîne 1,3-propylène, tétraméthylène, pentaméthylène ou éthylèneoxyéthylène, qui est à chaque fois non substituée ou peut porter de un à quatre groupes alkyle en $C_1$ à $C_4$ ou un ou deux groupes (alcoxy en $C_1$ à $C_4$)carbonyle,

ainsi que les sels utilisables en agriculture de ces composés I.

**2.** Utilisation de pyrazol-3-ylbenzazoles substitués et de leurs sels utilisables en agriculture, selon la revendication 1, comme herbicides ou pour la dessiccation/défoliation des plantes.

**3.** Agent herbicide contenant une quantité à action herbicide d'au moins un pyrazol-3-ylbenzazole substitué de formule I ou d'un sel utilisable en agriculture de I, selon la revendication 1, et au moins un support liquide et/ou solide inerte ainsi que si on le souhaite au moins un matériau tensioactif.

**4.** Agent de dessiccation et/ou de défoliation des plantes, contenant une quantité à action dessicative et/ou défoliante d'au moins un pyrazol-3-ylbenzazole substitué de formule I ou d'un sel utilisable en agriculture de I, selon la revendication 1, et au moins un support liquide et/ou solide inerte ainsi que si on le souhaite au moins un matériau tensioactif.

**5.** Procédé de fabrication d'agents à action herbicide, **caractérisé en ce qu'**on mélange une quantité à action herbicide d'au moins un pyrazol-3-ylbenzazole substitué de formule I ou d'un sel utilisable en agriculture de 1, selon la revendication 1, avec au moins un support liquide et/ou solide inerte ainsi que si on le souhaite au moins un matériau tensioactif.

**6.** Procédé de fabrication d'agents à action de dessiccation et/ou défoliants, **caractérisé en ce qu'**on mélange une quantité à action dessicative/défoliante d'au moins un pyrazol-3-ylbenzazole substitué de formule I ou d'un sel utilisable en agriculture de I, selon la revendication 1, avec au moins un support liquide et/ou solide inerte, ainsi que si on le souhaite au moins un matériau tensioactif.

**7.** Procédé de lutte contre la croissance végétale indésirable, **caractérisé en ce qu'**on fait agir une quantité à action herbicide d'au moins un pyrazol-3-ylbenzazole substitué de formule I ou d'un sel utilisable en agriculture de I, selon la revendication 1, sur les plantes, leur habitat ou sur les semences.

**8.** Procédé de dessiccation et/ou de défoliation de plantes, **caractérisé en ce qu'**on fait agir sur les plantes une quantité à action de dessiccation et/ou défoliante d'au moins un pyrazol-3-ylbenzazole substitué de formule I ou d'un sel utilisable en agriculture de I, selon la revendication 1.

**9.** Procédé selon la revendication 8, **caractérisé en ce qu'**on traite le coton.

**10.** Procédé de préparation de pyrazol-3-ylbenzazoles substitués de formule I selon la revendication 1, dans laquelle Z représente -N=C(NH2)-S-, **caractérisé en ce qu'**on fait réagir un aminophénylpyrazole de formule IIIa ou

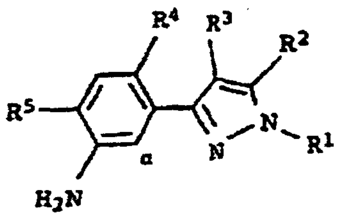

IIIa

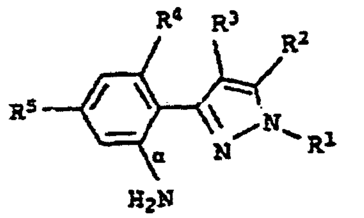

IIIb

IIIb

soit avec un halogène et du thiocyanate d'ammonium, soit avec un thiocyanate de métal alcalin ou alcalino-terreux, soit encore tout d'abord avec du thiocyanate d'ammonium ou un thiocyanate de métal alcalin ou alcalino-terreux, puis avec un halogène.

**11.** Procédé de préparation de pyrazol-3-ylbenzazoles substitués de formule I, dans laquelle $XR^6$ représente un halogène ou un cyano, **caractérisé en ce qu'**on diazote un pyrazol-3-ylbenzazole substitué I, dans lequel $XR^6$ représente un amino, puis **en ce qu'**on fait réagir le sel de diazonium obtenu avec un halogénure de cuivre (I) ou le cyanure de cuivre (I).

**12.** Procédé de préparation de pyrazol-3-ylbenzazoles substitués de formule I, dans laquelle X représente le soufre, **caractérisé en ce qu'**on diazote un pyrazol-3-ylbenzazole substitué I, dans lequel $XR^6$ représente un amino, puis **en ce qu'**on fait réagir le sel de diazonium obtenu avec un disulfure de dialkyle de formule $R^6S-SR^6$.

**13.** Procédé de préparation de pyrazol-3-ylbenzazoles substitués de formule I, dans laquelle $XR^6$ représente $-CH_2-CH(Cl)-R^8$, $-CH_2-CH(Br)-R^8$, $-CH=CH-R^8$, $-CH=C(Cl)-R^8$ ou $-CH=C(Br)-R^8$, **caractérisé en ce qu'**on diazote un pyrazol-3-ylbenzazole substitué I, dans lequel $XR^6$ représente un amino, puis **en ce qu'**on fait réagir le sel de diazonium obtenu avec un alcène de formule $H_2C=CH-R^8$ ou un alcyne de formule $HC\equiv C-R^8$ et le chlorure de cuivre (I), le bromure de cuivre (I), le chlorure de cuivre (II) ou le bromure de cuivre (II).

**14.** Procédé de préparation de pyrazol-3-ylbenzazoles substitués de formule I, dans lesquels X représente un oxygène, un soufre, -NH- ou $-N(R^7)-$, ou dans lesquels $XR^6$ représente $CH(R^b)R^c$, où $R^b$ et $R^c$ représentent indépendamment l'un de l'autre un cyano ou un (alcoxy en $C_1$ à $C_4$)carbonyle, **caractérisé en ce qu'**on fait réagir un pyrazol-3-ylbenzazole substitué I, dans lequel $XR^6$ représente un chlore, un brome, un $-SO_2$-alkyle ou un $-SO_2$-halogénoalkyle, en présence d'une base avec un agent nucléophile $HOR^6$, $HSR^6$, $H_2NR^6$, $HN(R^6)R^7$ ou $H_2C(R^b)R^c$.

**15.** Procédé de préparation de pyrazol-3-ylbenzazoles substitués de formule I, dans lesquels $XR^6$ représente un alkyle en $C_1$ à $C_6$, **caractérisé en ce qu'**on fait réagir un pyrazol-3-ylbenzazole substitué I, dans lequel $XR^6$ représente un halogène, si on le souhaite en présence d'un catalyseur, avec un composé de alkyle en $C_1$ à $C_6$-.

**16.** Procédé de préparation de pyrazol-3-ylbenzazoles substitués de formule I selon la revendication 1, dans laquelle Z représente $-N=C(R^6)-O-$**caractérisé en ce qu'**on diazote un aminophénylpyrazole de formule IIIa ou IIIb

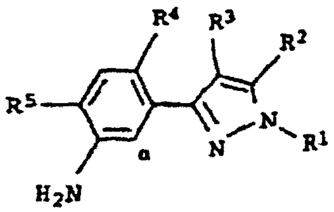

IIIa

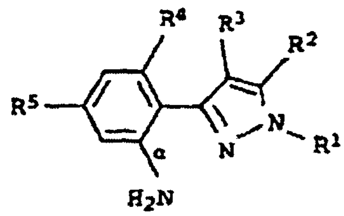

IIIb

**en ce qu'**on fait réagir le sel de diazonium formé avec un azide de métal alcalin pour donner un azidophénylpyrazole de formule Va ou Vb

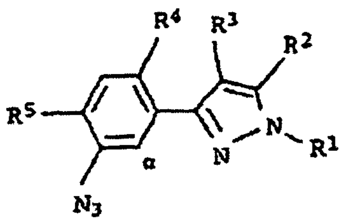

Va

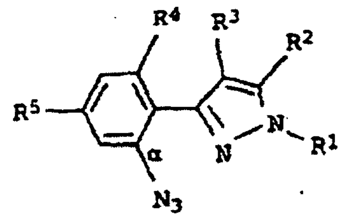

Vb

et enfin **en ce qu'**on fait réagir celui-ci avec un acide carboxylique de formule $R^6$-COOH.